(19) Europäisches Patentamt
European Patent Office
Office européen des brevets

(11) EP 1 600 511 A1

(12) EUROPEAN PATENT APPLICATION
published in accordance with Art. 158(3) EPC

(43) Date of publication:
**30.11.2005 Bulletin 2005/48**

(21) Application number: **04706870.5**

(22) Date of filing: **30.01.2004**

(51) Int Cl.[7]: **C12N 15/86**, C12N 15/861, C12N 5/10, A61K 48/00, A61P 9/10

(86) International application number:
**PCT/JP2004/000957**

(87) International publication number:
**WO 2004/074494 (02.09.2004 Gazette 2004/36)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **19.02.2003 JP 2003040806**

(71) Applicant: **Dnavec Research Inc.**
**Tsukuba-shi, Ibaraki 305-0856 (JP)**

(72) Inventors:
- **HAMADA, Hirofumi**
  **Sapporo-shi, Hokkaido 064-0959 (JP)**
- **ITO, Yoshinori**
  **Sapporo-shi, Hokkaido 064-0921 (JP)**
- **TAKAHASHI, Kazuhiro**
  **Sapporo-shi, Hokkaido 064-0804 (JP)**
- **MORIKAWA, Masayuki**
  **Sapporo-shi, Hokkaido 063-0001 (JP)**

(74) Representative: **Vossius & Partner**
**Siebertstrasse 4**
**81675 München (DE)**

# (54) METHOD OF TREATING ISCHEMIC DISEASE

(57) The present invention provides methods for treating ischemic diseases, which comprise the step of administering angiopoietin-1 (Ang1) or an Ang1-encoding vector. The present invention also provides ischemic disease treatment kits which comprise Ang1. Ang1-expressing vectors were prepared, and each was administered alone intramyocardially to rats in the acute phase of myocardial infarction to express Ang1 in the local cardiac muscle. The results indicate that marked effects have been obtained, such as decrease in post-infarction mortality rate, increase in blood vessel number in myocardium, reduction of myocardial infarct size, and improvement of cardiac function. Administration of the required VEGF was not necessary for the angiogenic activity of Ang1. Furthermore, when an Anglviral expression vector was administered alone to an animal model of severe limb ischemia, in which ischemia had been induced by arterial ligation, a remarkable limb salvage effect was obtained. The Ang1 gene therapy is excellent as a safe and effective therapeutic method for ischemic diseases such as ischemic heart diseases and limb ischemia.



Printed by Jouve, 75001 PARIS (FR)

## Description

### Technical Field

The present invention relates to methods of treating ischemic diseases with the use of angiopoietin-1 (Ang1) or vectors encoding Ang1. The present invention also relates to ischemic disease treatment kits comprising Ang1 or an Ang1-encoding vector.

### Background Art

Ischemia caused by acute injury or arterial occlusion sometimes results in loss of fingers, functional disorders, or serious diseases that lead to death. Due to changes of social environment and the arrival of an aging society, ischemic heart diseases such as acute myocardial infarction and severe angina pectoris, in particular have increased rapidly, and now account for the majority of lifestyle-related diseases. Surgical revascularization procedures such as percutaneous transluminal coronary angioplasty (PTCA) and coronary artery bypass graft (CABG) are used mainly to treat acute myocardial infarction. The use of such conventional therapeutic methods in combination with genetic engineering techniques to enhance revascularization enables active improvement of cardiac function and reduction of the period confined to bed.

Primarily, the United States has been conducting clinical trials for therapeutic angiogenesis using vascular endothelial growth factor (VEGF) genes and proteins to treat coronary artery ischemia (Losordo, D. W., *et al.* (1998) Circulation. 98: 2800-2804; Rosengart, T. K., *et al.* (1999) Circulation. 100: 468-474; Lathi, K. G., *et al.* (2001) Anesth Analg. 92: 19-25; Symes, J. F., *et al.* (1999) Ann Thorac Surg. 68: 830-836; discussion 836-837) and severe limb ischemia (Baumgartner, I., *et al.* (1998) Circulation. 97: 1114-1123; Isner, J. M., *et al.* (1998) J Vasc Surg. 28: 964-973; discussion 973-965; Baumgartner, I., *et al.* (2000) Ann Intern Med. 132: 880-884) due to its rong activity of stimulating vascular endothelial proliferation. As of now, application of the VEGF gene therapy for ischemic heart diseases is limited to only severe angina pectoris, and does not cover acute myocardial infarction. It has been found that in acute ischemia such as myocardial infarction, VEGF production is enhanced in local cardiac muscle, peripheral blood leukocytes, mononuclear cells, and macrophages shortly after infarction, resulting in an exceedingly high level of circulating VEGF (Xu, X., *et al.* (2001) J Thorac Cardiovasc Surg. 121: 735-742; Li, J., *et al.* (1996) Am J Physiol. 270: H1803-1811; Ladoux, A. and C. Frelin. (1993) Biochem Biophys Res Commun. 195: 1005-1010; Seko Y, *et al.* Clin Sci 92, 453-454, 1997; Banai S, *et al.* Cardiovasc Res. 28,1176-1179,1994; Berse B, *et al.* Mol Biol Cell, 3, 211-220, 1992; Taichman NS, J leukoc Biol, 62, 397-400, 1997). While the physiological significance of enhanced VEGF production is not fully understood, VEGF is assumed to contribute to the rapid recovery from ischemia by protecting and repairing blood vessels in ischemic sites (Banai S, *et al.* Cardiovasc Res. 28,1176-1179,1994). However, excessive administration of VEGF increases fragile blood vessels and premature blood vessels (Thurston, G., *et al.* (1999) Science. 286: 2511-2514), and induces hemangioma formation at the administration site (Schwarz, E. R., *et al.* (2000) J Am Coll Cardiol. 35: 1323-1330). Furthermore, Matsuno *et al.* have recently reported that high-level VEGF in myocardial infarction may aggravate pulmonary edema and such, and increase the mortality rate in acute myocardial infarction (Matsuno H *et al.* Blood 100, 2487, 2002).

### Disclosure of the Invention

The present invention provides methods for treating ischemic diseases using Ang1 or Ang1-encoding vectors. The present invention also provides ischemic disease treatment kits comprising Ang1 or an Ang1-encoding vector.

VEGF165, having a strong angiogenesis-inducing activity, was expressed by an adenoviral vector in local cardiac muscle in the acute phase of myocardial infarction. The angiogenesis-inducing activity was then confirmed in infarcted hearts of the surviving rats. However, an increased mortality rate in the acute phase, i.e., four to five days after the infarction, was confirmed. Autopsies of the dead rats revealed marked pleural effusion (4 ml to 5 ml) (data not shown). In view that VEGF enhances capillary permeability, the effect of VEGF 165 administration on the vascular permeability in lungs after myocardial infarction was studied. As a result, the vascular permeability was markedly increased (data not shown). Matsuno *et al.* have reported induction of high-level VEGF in $\alpha$1-antiplasmin knockout mice after myocardial infarction, and a consequential increase in the mortality rate of pulmonary edema. Also in the above-described experiment performed by the present inventors, along with the high VEGF level after myocardial infarction, it can be inferred that VEGF165 over-expression enhanced the vascular permeability in lungs and induced pulmonary edema, thus increasing the mortality rate. The present inventors focused on angiopoietin-1 (Ang1) to develop safer and more effective methods of gene therapy for myocardial infarction.

Ang1, a ligand for the Tie-2 receptor and an important angiogenesis factor, acts synergistically with VEGF and is involved in angiogenesis, and vascular maturation and stabilization (Davis, S., *et al.* (1996) Cell. 87: 1161-1169;

Sato, T. N., *et al.* (1995) Nature. 376: 70-74). Co-administration of Ang1 and VEGF has been reported to enhance revascularization in animal models of ischemia (Jones, M. K., *et al.* (2001) Gastroenterology. 121: 1040-1047; Chae, J. K., *et al.* (2000) Arterioscler Thromb Vasc Biol. 20: 2573-2578). The present inventors have also reported that in an obstructive arteriosclerosis model, gene therapy using a combination of the Ang1 and VEGF genes enhances the angiogenesis activity of VEGF while reducing such adverse effects as edema resulted from the increased vascular permeability by VEGF (Ito Y, *et al.,* Molecular Therapy, 5(5), S162, 2002; WO02/100441). In the present invention, the present inventors examined the therapeutic effect of administering Ang1 alone into ischemic hearts. It is generally believed that Ang1 alone does not stimulate vascular endothelial proliferation, and the truth is in Ang1 transgenic mice, the vascular diameters are increased but not the vascular densities (Thurston, G, J. Anat. 200: 575-580 (2002)). However, the present inventors conceived that angiogenesis effect can be obtained through the sole administration of Ang1, in view of the extremely high level of endogenous VEGF in acute ischemia such as myocardial infarction. Specifically, the inventors conceived that a strategy using Ang1 in the acute phase of myocardial infarction to enhance angiogenesis, in synergy with the actively produced VEGF in the body, and to promote revascularization while reducing the toxicity accompanied by the enhanced VEGF production would be possible. Ang1 antagonistically suppresses the enhancement of vascular permeability and blood coagulation induced by inflammatory cytokines, such as VEGF, IL-1, and TNF, which are involved in the aggravation of myocardial infarction (Thurston, G. (2002) J Anat. 200: 575-580; Thurston, G, *et al.* (2000) Nat Med. 6: 460-463; Thurston, G., *et al.* (1999) Science. 286: 2511-2514). The present inventors predicted that the Ang1 administration could also prevent the elevation of vascular permeability and the acceleration of blood coagulation induced by inflammatory cytokines, which have been actively produced in the acute phase of myocardial infarction.

Thus, the present inventors prepared an adenoviral vector expressing the Ang1 gene, and administered the vector intramyocardially to a rat myocardial infarction model. The inventors studied the angiogenesis effect, effect of reducing the infarct size, improvement of cardiac function, and decrease in mortality rate.

The arterial ligation-induced myocardial infarction markedly decreased the vascular density in the infarcted site and its surrounding area. Furthermore, after the myocardial infarction, a decrease in vascular density in the septal myocardium distant from the gene administration site was also revealed. The reason remains unknown, but it can be assumed to reflect the post-myocardial infarction heart failure. Model rats were administered intramyocardially with the adenoviral vector in the heart at the surrounding area of the site to be infarcted. The expression level of the administered gene was examined five days after the surgical operation, and was shown to be comparable in the infarcted hearts (approximately 80%) and the normal hearts to which the vector had also been administered. It was thereby demonstrated that sufficiently high levels of the gene expression could be attained when it is injected into the peri-infarct area. Interestingly, in the Ang1 gene-administered group, the vascular density was increased not only in the infarcted site and its surrounding area, but also in the septal region apparently. This suggests that Ang1 not only enhances angiogenesis in the administration site, but is secreted into blood and also induces angiogenesis in distal myocardium. Furthermore, the number of blood vessels with 10-μm or greater diameter was clearly increased in the Ang1 gene-administered group. In addition, blood vessels with pericytes, which are indicative of the more functional blood vessels, were significantly increased. This supports the idea that Ang1-induced blood vessels are more functional.

Furthermore, the present inventors found that minus-strand RNA viral vectors are highly effective in Ang1 gene therapy for ischemic diseases. A study, where the gene was introduced into myocardial cells, demonstrated that the efficiency of gene introduction into myocardial cells was significantly higher with a minus-strand RNA viral vector than with an adenoviral vector. The minus-strand RNA viral vector carrying the Ang1 gene also exhibited an outstanding therapeutic effect on myocardial infarction and limb ischemia. Among vectors used for treating cardiovascular diseases, particularly cardiac diseases, adenoviral vectors are most commonly used because they ensures efficient gene transfer and high-level gene expression in nondividing cells including myocardial cells. However, it has been pointed out that the adenoviral vector may induce inflammation due to its high immunogenicity and produce adverse effects as a result of its exceedingly high affinity for the liver. Thus, there is a demand for safer and more efficient alternatives to the gene introduction technique that uses adenoviral vectors. The adeno-associated viral vector (AAV), the lentiviral vector, and others have been previously tested, and were found to exhibit long-term gene expression in the heart. However, there is a possibility that these retroviral vectors and DNA viral vectors interact with the chromosome in the host cell nucleus and become integrated into the host chromosome. In contrast, the minus-strand RNA viral vector can strongly express the gene that it carries in the cytoplasm without being integrated into the host cell chromosome, and thus conferring no risk of chromosome damage. The use of minus-strand RNA viral vectors may allow a more effective and safer Ang1 gene therapy for ischemic diseases.

Ang1 was found to increase vascular density in infarcted hearts. When Ang1 is used clinically, it is most important to assess whether the increase in the vascular density indeed contributes to reduction of infarcted region and improvement of cardiac functions. The infarcted region was measured four weeks after myocardial infarction. It was then found that the infarcted region was reduced and the infarcted wall was thickened in the Ang1 gene-administered group. The cardiac functions, particularly fractional shortening (FS) of left ventricular short-axis diameter, left ventricular area at systole (LVAs), and left ventricular ejection fraction (EF), were found to be improved. It has been reported previously that the hepatocyte growth factor (HGF), hypoxia inducible factor-1 $\alpha$ (HIF-1 $\alpha$), and VEGF induce angiogenesis and reduce the infarcted region in a rat model of myocardial infarction, which had been prepared by ligating the left anterior descending branch. However, there are very few reports on improving cardiac functions after serious myocardial infarction by administration of an angiogenesis factor alone. It has been reported that cardiac functions are improved effectively only when such an angiogenesis factor is used in combination with cell therapy by fetal cardiac muscle, ES cells, myoblasts, or such, which complements the absolute mass of cardiac muscle (Yau, T. M., Circulation 104: I218-I222 (2001); Suzuki, K., Circulation 104: I207-212 (2001); Orlic, D., Proc. Natl. Acad. Sci. USA 98: 10344-10349 (2001)). The present invention demonstrated for the first time that administration of Ang1 alone can improve cardiac functions of an infarcted heart. The administration of Ang1 in the acute phase of myocardial infarction produces marked effects, such as decreasing the post-infarction mortality rate, increasing the number of blood vessels in cardiac muscle, reducing the infarct size, and improving cardiac functions. Thus, Ang1 gene therapy can be a new effective therapy for acute myocardial infarction.

The present inventors also performed gene therapy where Ang1 gene alone was administered into an animal model of severe limb ischemia, using an adenoviral vector and a minus-strand RNA viral vector which highly express Ang1. Naked DNA was expressed with an exceedingly high efficiency in cardiac muscle. In contrast, the expression level of the introduced gene by the naked DNA vector was lower in skeletal muscles (Example 8). Therefore, it appeared that direct administration of the Ang1 plasmid to ischemic limbs produced a less-than-sufficient effect on limb salvage (WO02/100441). However, it has been made clear that by using a viral vector with higher expression efficiency in the skeletal muscles than the naked DNA, the administration of Ang1 gene alone exerted a marked effect on limb salvage (Examples 7, 13, and 14). A noteworthy finding was that the effect on limb salvage as a result of the Ang1 gene administration was also observed prior to the initiation of blood perfusion in tissues due to arteriogenesis. Therefore, it can be conceived that the Ang1 gene therapy produced not only a therapeutic effect by inducing angiogenesis, but also an unexpected effect in protecting ischemic tissues beginning at an early stage prior to the induction of angiogenesis, as a result of antiapoptotic activity or such. Thus, it can be expected that administration of Ang1 gene alone using an Ang1-encoding viral vector produces a therapeutic effect, which would have been impossible with a plasmid vector, not only in ischemic heart diseases but also in general ischemic diseases including extremity ischemia, injuries associated with impaired circulation, and traumatic injury such as amputation, and fractures. Conventional therapy used in combination with VEGF is risky in that excess VEGF enhances vascular permeability and then aggravates pulmonary edema or such. However, when an Ang1-encoding viral vector is administered alone, ischemia can be treated effectively while such adverse effects are avoided.

Specifically, the present invention relates to methods for treating ischemic diseases using Ang1 or an Ang1-encoding vector, and ischemic disease treatment kits comprising Ang1 or an Ang1-encoding vector, and more specifically relates to the invention described in each claim. The present invention also relates to inventions comprising a desired combination of one or more (or all) of the inventions described in the respective claims, in particular, to inventions comprising a desired combination of one or more (or all) of the inventions described in claims (dependent claims) which cite identical independent claims (claims each relating to an invention which is not encompassed in the inventions described in any other claims). The invention described in each independent claim comprises inventions comprising an arbitrary combination of its dependent claims. Specifically, the present invention provides:

[1] a method for treating ischemic heart diseases, which comprises the step of administering angiopoietin-1 or a vector encoding angiopoietin-1;
[2] the method for treating ischemic heart diseases according to [1], which comprises the step of administering angiopoietin-1 or a vector encoding angiopoietin-1, and in which a vascular endothelial growth factor is not administered;
[3] the method according to [1] or [2], wherein angiopoietin-1 or the vector encoding angiopoietin-1 is a viral vector encoding angiopoietin-1;
[4] the method according to [3], wherein the viral vector is an adenoviral vector;
[5] the method according to [3], wherein the viral vector is a minus-strand RNA viral vector;
[6] the method according to [1] or [2], wherein angiopoietin-1 or the vector encoding angiopoietin-1 is a naked DNA;
[7] the method according to any one of [1] to [6], wherein angiopoietin-1 or the vector encoding angiopoietin-1 is a vector that drives angiopoietin-1 expression using CA promoter or a promoter having a transcriptional activity equivalent to or higher than that of said CA promoter;
[8] the method according to any one of [1] to [7], wherein the administration of angiopoietin-1 or the vector encoding angiopoietin-1 is an injection into cardiac muscle;
[9] a method for treating ischemic diseases, which comprises the step of administering a viral vector encoding angiopoietin-1;

[10] the method for treating ischemic diseases according to [9], which comprises the step of administering a viral vector encoding angiopoietin-1, and wherein a vascular endothelial growth factor is not administered;
[11] the method according to [9] or [10], wherein the viral vector is an adenoviral vector;
[12] the method according to [9] or [10], wherein the viral vector is a minus-strand RNA viral vector;
[13] the method according to any one of [9] to [12], wherein the vector administration is an injection into an ischemic site;
[14] a genetically modified mesenchymal cell comprising a foreign gene encoding angiopoietin-1;
[15] the mesenchymal cell according to [14], into which an adenoviral vector encoding angiopoietin-1 has been introduced;
[16] the mesenchymal cell according to [14], into which a minus-strand RNA viral vector encoding angiopoietin-1 has been introduced;
[17] a therapeutic composition for ischemia, which comprises the mesenchymal cell according to any one of [14] to [16] and a pharmaceutically acceptable carrier;
[18] a method for producing a genetically modified mesenchymal cell, wherein the method comprises the step of contacting the mesenchymal cell with a minus-strand RNA viral vector carrying a gene; and,
[19] the method according to [18], wherein the gene encodes angiopoietin-1.

The present invention relates to methods for treating ischemic heart diseases, which comprises the step of administering Ang1 or an Ang1-encoding vector. Ang1 alone does not have the activity to stimulate vascular endothelial proliferation. It was unclear as to whether administration of Ang1 gene alone had produced any therapeutic effect on ischemic heart diseases. However, in the present invention, it was demonstrated that an administration of Ang1 alone produced a marked therapeutic effect in myocardial infarction. It has been known that VEGF increases in the sera of patients with acute myocardial infarction two to three days after infarction, and the local VEGF expression level in the heart of a myocardial infarction model also increases one to three days after myocardial infarction, and the high-level expression continues for one week or longer. In addition, the local and serum levels of VEGF were confirmed to increase in a rat myocardial infarction model produced by the present inventors (data not shown). Accordingly, the therapeutic effect brought upon the administration of Ang1 alone may be a combined effect with endogenous VEGF. Excess VEGF enhances lung vascular permeability and causes pulmonary edema, thereby increasing mortality rate. Administration of Ang1 alone without VEGF allows endogenous VEGF and Ang1 expressed from the introduced gene to act synergistically to produce a strong angiogenesis effect, while eliminating the possible adverse effects produced by VEGF administration. In particular, the present invention provides methods for treating ischemic heart diseases, comprising the step of administering Ang1 or an Ang1-encoding vector without administration of vascular endothelial growth factor (VEGF) or its gene. The vascular density was clearly increased in the infarcted site and its surrounding area when Ang1 is administered alone; the vascular density-increasing effect was comparable to that produced by introducing the same amount of VEGF 165 gene alone via an adenoviral vector. According to the present invention, ischemic tissues can be protected in a safer and more effective manner by administering Ang-1 its gene without administration of VEGF. The methods of the present invention for treating ischemic diseases and ischemic heart diseases are useful methods for protection of ischemic tissues, regeneration of rejected tissues, and revascularization in rejected tissues.
Herein, "angiopoietin-1 (Ang1)" refers to a ligand that binds to the Tie-2 receptor, and through the receptor activates signal transduction and enhances angiogenesis. Tie-2 is a tyrosine kinase receptor and is expressed in endothelial cell lines (Ac. No. NM_000459, protein ID. Q02763, NP_000450)(Ziegler, S. F. *et al.,* Oncogene 8 (3), 663-670 (1993); Boon, L. M. *et al.,* Hum. Mol. Genet. 3 (9), 1583-1587 (1994); Dumont, D. J. *et al.,* Genomics 23 (2), 512-513 (1994); Gallione CJ *et al.,* J. Med. Genet. 32 (3), 197-199 (1995); Vikkula M *et al.,* Cell 87 (7), 1181-1190 (1996); Witzenbichler, B. *et al.,* J. Biol. Chem. 273 (29), 18514-18521 (1998); Asahara, T. *et al.,* Circ. Res. 83 (3), 233-240 (1998); Calvert, J. T. *et al.,* Hum. Mol. Genet. 8 (7), 1279-1289 (1999)). Tie-2 has been isolated not only from human but also from non-human mammals including cow and mouse (Sato, T. N. *et al.,* Proc. Natl. Acad. Sci. U.S.A. 90 (20), 9355-9358 (1993); Iwama, A. *et al.,* Biochem. Biophys. Res. Commun. 195 (1), 301-309 (1993)). The nucleotide sequence of a wild-type human Tie-2-encoding DNA and its amino acid sequence are shown in SEQ ID NOs: 1 and 2, respectively. Ligands for the human Tie-2 shown in SEQ ID NO: 2 and the above-described mammalian homologues which enhance angiogenesis can be used preferably in the present invention. The Ang1 of the present invention comprises not only the naturally-occurring protein, but also modified proteins and partial peptides thereof that have the function of a Tie-2 ligand similarly to naturally-occurring Ang1. Furthermore, it can be a fragment of an anti-Tie-2 antibody which binds to the extracellular domain of Tie-2, or a non-peptide compound which functions as a Tie-2 ligand.
Mammalian Ang1 proteins have been isolated from various mammalian species, including human, mouse, rat, pig, and cow (Davis, S. *et al.,* Cell 87 (7), 1161-1169 (1996); Valenzuela, D. M. *et al.,* Proc. Natl. Acad. Sci. U.S.A. 96 (5), 1904-1909 (1999); Suri, C. *et al.,* Cell 87 (7), 1171-1180 (1996); Valenzuela, D. M. *et al.,* Proc. Natl. Acad. Sci. U.S.A. 96 (5), 1904-1909 (1999); Kim, I., *et al.,* Cardiovasc. Res. 49 (4), 872-881 (2001); Mandriota, S. J. and Pepper, M. S., Circ. Res. 83 (8), 852-859 (1998); Goede, V. *et al.,* Lab. Invest. 78 (11), 1385-1394 (1998))(GenBank Ac. No: U83508, UNM_009640, AF233227, NM_053546; protein_ID: AAB50557, NP_033770, 008538, AAK14992, NP_445998, O18920). The nucleotide sequence of a wild-type human Ang1-encoding DNA and its amino acid sequence are shown in SEQ ID NOs: 3 and 4, respectively. Human Ang1 shown in SEQ ID NO: 4 and the mammalian homologues described above can be used preferably.

The Ang1 of the present invention also comprises: a protein comprising an amino acid sequence with one or more amino acid substitutions, deletions, and/or additions in the human or other mammalian Ang1amino acid sequence; a protein comprising an amino acid sequence which has 70% or higher, preferably 75% or higher, more preferably 80% or higher, more preferably 85% or higher, still more preferably 90% or higher, yet more preferably 95% or higher identity to the human or other mammalian Ang1 amino acid sequence; and a protein encoded by a nucleic acid hybridizing under stringent conditions to a nucleic acid comprising the entire coding region or a portion of the human or other mammalian Ang1 gene, wherein the protein binds to a mammalian Tie-2 receptor and activates signal transduction via the receptor, thereby enhancing angiogenesis. Such proteins may comprise polymorphic and splicing variants of Ang1.

The number of amino acids changed by amino acid substitution, deletion, and/or addition is typically 15 residues or less, preferably 11 residues or less, more preferably 9 residues or less, more preferably 7 residues or less, still more preferably 5 residues or less. Particularly when amino acids have been substituted conservatively, proteins tend to retain their original activities. Conservative substitutions include amino acid substitutions within each group of: basic amino acids (for example, lysine, arginine, and histidine); acidic amino acids (for example, aspartic acid and glutamic acid); non-charged polar amino acids (for example, glycine, asparagine, glutamine, serine, threonine, tyrosine, and cysteine); non-polar amino acids (for example, alanine, valine, leucine, isoleucine, proline, phenylalanine, methionine, and tryptophan), β-branched amino acids (for example, threonine, valine, and isoleucine), and aromatic amino acids (for example, tyrosine, phenylalanine, tryptophan, and histidine). Amino acid sequence identities can be determined, for example, using the BLASTP program (Altschul, S. F. *et al.,* 1990, J. Mol. Biol. 215: 403-410). Specifically, the blastp program can be used for this purpose. For example, when the search is carried out using BLAST in the website of National Center for Biotechnology Information (NCBI), the filters comprising low complexity are all switched off and default parameters are used (Altschul, S. F. *et al.* (1993) Nature Genet. 3:266-272; Madden, T. L. *et al.* (1996) Meth. Enzymol. 266:131-141; Altschul, S. F. *et al.* (1997) Nucleic Acids Res. 25:3389-3402; Zhang, J. & Madden, T. L. (1997) Genome Res. 7:649-656). For example, the sequence identity can be determined by preparing an alignment of two sequences using the blast2sequences program for comparison of two sequences (Tatiana A *et al.* (1999) FEMS Microbiol Lett. 174:247-250). Gaps are treated similarly to mismatches. For example, identities to the entire amino acid sequence of mammalian wild-type Ang1 protein are computed. Alternatively, identities can be determined by hybridization, in which a probe is prepared either from a nucleic acid comprising the Ang1 protein coding sequence derived from human or other animals, or from a target nucleic acid of hybridization. Hybridization of the probe to other nucleic acids is then tested. Stringent hybridization conditions comprise hybridizing in a solution comprising 5x SSC, 7% (W/V) SDS, 100 μg/ml denatured salmon sperm DNA, and 5x Denhardt's solution (1x Denhardt's solution comprises 0.2% polyvinyl pyrrolidone, 0.2% bovine serum albumin, and 0.2% Ficoll) at 48°C, preferably at 50°C, more preferably at 52°C, followed by 2 hours of washing while shaking at the same temperature used in the hybridization, more preferably at 60°C, still more preferably at 65°C, most preferably at 68°C in 2x SSC, preferably in 1x SSC, more preferably in 0.5x SSC, and more preferably in 0.1x SSC.

An "Ang1-encoding vector" refers to a vector comprising an Ang1 protein-encoding nucleic acid. The phrase "protein-encoding" means that a nucleic acid contains an ORF encoding an amino acid sequence of the protein in a sense or antisense strand (in a certain viral vector or such), so that the nucleic acid can express the protein under appropriate conditions. The nucleic acid may be a single- or double-stranded nucleic acid. Furthermore, the nucleic acid may be DNA or RNA. The vector includes plasmid vectors, other naked DNAs, and viral vectors.

"Naked DNA" refers to a DNA that does not bind to reagents for introducing nucleic acids into cells, such as viral envelope, liposome, and cationic lipids (Wolff *et al.,* 1990, Science 247, 1465-1468). Naked DNA can be used after being dissolved in a physiologically acceptable solution, for example, sterilized water, physiological saline, or buffer. The injection of naked DNA such as plasmid is the safest and simplest gene transfer method, and is used as the major procedure in previously approved clinical protocols of gene therapy for cardiovascular diseases (Lee, Y. *et al.,* Biochem. Biophys. Res. Commun. 2000; 272: 230-235). However, the relatively low expression of the introduced gene and the low efficiency of introduction into myocardial cells impair the therapeutic benefits of this approach (Lin, H. *et al.,* Circulation 1990; 82: 2217-2221; Kass-eisler, A. *et al.,* Proc Natl Acad Sci USA 1993; 90: 11498-11502). For example, cytomegalovirus (CMV) promoter is one of the most potent transcriptional regulatory sequences available, and vectors comprising the CMV promoter have been commonly used in gene therapy (Foecking, M. K, and Hofstetter H. Gene 1986; 45: 101-105). However, some reports on injection of the plasmids into skeletal muscles suggested that the expression level or period of the introduced gene was often insufficient even when the strong CMV promoter was used.

Surprisingly, however, the present inventors found that when a naked plasmid is introduced into the cardiac muscle by direct injection, the expression level in the cardiac muscle is approximately an order of magnitude greater than that in the skeletal muscle. The expression level of the gene introduced into the heart using 20 µg of a plasmid vector comprising the CA promoter, whose transcriptional activity is particularly strong, was comparable to that achieved by using 6.0x $10^9$ optical units (OPU) of an adenoviral vector. Thus, the gene therapy for ischemic diseases according to the present invention can be performed using a plasmid comprising the CA promoter or a promoter with transcriptional activity comparable to or higher than that of the CA promoter. The CA promoter is a chimeric promoter comprising the CMV immediately early enhancer and the chicken β-actin promoter (Niwa, H. *et al.* (1991) Gene. 108: 193-199). Safer gene therapy using a naked DNA can be achieved by using the CA promoter or a promoter with transcriptional activity comparable to or higher than that of the CA promoter.

The CMV immediately early enhancer to be used may be an immediately early gene enhancer derived from a desired CMV strain. The enhancer includes the nucleotide sequence of positions 1 to 367 of SEQ ID NO: 5. The chicken β-actin promoter to be used includes a DNA fragment which comprises the transcription initiation site derived from the chicken β-actin genomic DNA and has a promoter activity. Since the first intron of the chicken β-actin gene has transcription-enhancing activity, it is preferred that a genomic DNA fragment comprising at least a portion of this intron is used. Specifically, such a chicken β-actin promoter includes the nucleotide sequence from positions 368 to 1615 of SEQ ID NO: 5. A sequence from another gene, for example, the intron-acceptor sequence of rabbit β-globin, may be appropriately used as the intron acceptor sequence. A preferred CA promoter of the present invention is a DNA, in which the chicken β-actin promoter comprising a portion of the intron is ligated to the downstream of the CMV immediately early enhancer sequence, and then a desired intron-acceptor sequence is placed downstream thereof. An example of such DNA is shown in SEQ ID NO: 5. The Ang1 protein-encoding sequence may be attached to the last ATG of the above-described sequence which serves as the initiation codon. However, sequence polymorphisms in the CMV enhancer and the chicken β-actin gene may exist among isolated strains or isolated individuals. It is not necessary to use the same regions shown in SEQ ID NO: 5 as the CMV immediately early enhancer and the chicken β-actin promoter. Those skilled in the art can construct different variant types. Every variant having a transcriptional activity equivalent to or higher than that of the promoter shown in SEQ ID NO: 5 can be used preferably in the present invention.

If SV40ori is comprised in the vector, the SV40ori sequence is preferably deleted. The SV40 large T antigen is involved in some types of human cancers, and there is a risk that the SV40ori-comprising vector might be amplified in patients with SV40-associated cancer (Martini, F. *et al.,* Cancer 2002; 94: 1037-1048; Malkin, D. Lancet 2002; 359: 812-813). The present inventors verified that the deletion of SV40ori from the vector had no effect on the expression level of the introduced gene in the heart as well as in the skeletal muscle (Example 8). This result suggests that the SV40ori-free vector, which expresses Ang1 under the control of the CA promoter, is one of the safest and the most useful vectors in clinical applications of cardiac muscle gene therapy. In particular, the pCA1 vector in which SV40ori has been deleted is considered suitable for cardiac muscle gene therapy.

In addition, a DNA can be appropriately administered in combination with a transfection reagent. For example, the transfection efficiency can be improved by combining the DNA with liposomes or desired cationic lipids.

Another preferred vector of the present invention to be used in the treatment of ischemic diseases is a viral vector. Ang1 can be expressed at sufficiently high levels not only in the cardiac muscle, but also in other tissues such as the skeletal muscle by using a viral vector. The viral vector includes, but is not limited to, an adenoviral vector, an adeno-associated viral vector, a retroviral vector, a lentiviral vector, a herpes simplex virus vector, and a vaccinia virus vector. A preferred viral vector is an adenoviral vector. The adenoviral vector can introduce a gene into myocardial cells at high efficiencies and express the introduced gene at high levels. As shown in the Examples, the Ang1-expressing adenoviral vector produces significant therapeutic effects on ischemic hearts and limbs. Thus, the adenoviral vector can be suitably used in the present invention. In the present invention, conventional adenoviral vectors can be appropriately used. In such vectors, genes of the wild-type virus may be modified, for example, to improve the expression level of a foreign gene or to attenuate their antigenicity. Adenoviral vectors can be prepared, for example, using the COS-TPC method developed by Saito *et al.* (Miyake, S., Proc. Natl. Acad. Sci. USA 93: 1320-1324 (1996)).

When Ang1 is incorporated into a vector, the sequence around the initiation codon of Ang1 is preferably made into a Kozak's consensus sequence [for example, CC(G/A)CCATG] to increase the efficiency of the Ang1 gene expression (Kozak, M., Nucleic Acids Res 9(20), 5233 (1981); Kozak, M., Cell 44, 283 (1986); Kozak, M. Nucleic Acids Res.15:8125 (1987); Kozak, M., J. Mol. Biol. 196, 947 (1987); Kozak, M., J. Cell Biol. 108, 229 (1989); Kozak, M., Nucl. Acids Res. 18, 2828 (1990)).

Another viral vector preferably used in the present invention is a minus-strand RNA viral vector. As shown in the Examples, the minus-strand RNA viral vector could achieve higher expressions of an introduced gene with a lower titer than those of the adenovirus. The Ang1-encoding minus-strand RNA viral vector is one of the most preferably used vectors in the present invention. A "minus-strand RNA virus" refers to a virus comprising a minus-strand (an antisense strand complementary to a viral protein-encoding sense strand) RNA as the genome. The minus-strand RNA is also called a "negative strand RNA". In particular, the minus-strand RNA virus to be used in the present invention includes single-stranded minus-strand RNA viruses (also called "non-segmented minus-strand RNA viruses"). A "single-stranded negative strand RNA virus" refers to a virus comprising a single-stranded negative strand (i.e., minus strand) RNA as the genome. Such viruses include: paramyxovirus *(Paramyxoviridae* such as the genus *Paramyxovirus,* the genus *Morbillivirus,* the genus *Rubulavirus,* and the genus *Pneumovirus);* rhabdovirus *(Rhabdoviridae* such as the genus Vesiculovirus, the genus *Lyssavirus,* and the genus *Ephemerovirus);* filovirus *(Filoviridae);* orthomyxovirus *(Orthomyxoviridae* such as Influenza viruses A, B, and C, and Thogoto-like virus); bunyavirus *(Bunyaviridae* such as the genus *Bunyavirus,* the genus *Hantavirus,* the genus *Nairovirus,* and the genus *Phlebovirus);* and arenavirus *(Arenaviridae).* The minus-strand RNA viral vector to be used in the present invention may be a vector having transmissibility, or a deficient vector having no transmissibility. The phrase "having transmissibility" means that after a viral vector infects host cells, the virus was replicated and infectious virus particles are produced in the cells.

Specifically, the minus-strand RNA virus that can be used preferably in the present invention includes Sendai virus, Newcastle disease virus, mumps virus, measles virus, respiratory syncytial virus (RS virus), rinderpest virus, distemper virus, simian parainfluenza virus (SV5), human parainfluenza viruses type 1, type 2, and type 3, which belong to *Paramyxoviridae;* influenza virus which belongs to *Orthomyxoviridae;* and vesicular stomatitis virus and rabies virus which belong to *Rhabdoviridae.*

The virus that can be used in the present invention further includes Sendai virus (SeV), human parainfluenza virus-1 (HPIV-1), human parainfluenza virus-3 (HPIV-3), phocine distemper virus (PDV), canine distemper virus (CDV), dolphin morbillivirus (DMV), peste-des-petits-ruminants virus (PDPR), measles virus (MV), rinderpest virus (RPV), Hendra virus (Hendra), Nipah virus (Nipah), human parainfluenza virus-2 (HPIV-2), simian parainfluenza virus 5 (SV5), human parainfluenza virus-4a (HPIV-4a), human parainfluenza virus-4b (HPIV-4b), mumps virus (Mumps), and Newcastle disease virus (NDV). More preferably, the virus includes that selected from the group consisting of Sendai virus (SeV), human parainfluenza virus-1 (HPIV-1), human parainfluenza virus-3 (HPIV-3), phocine distemper virus (PDV), canine distemper virus (CDV), dolphin morbillivirus (DMV), peste-des-petits-ruminants virus (PDPR), measles virus (MV), rinderpest virus (RPV), Hendra virus (Hendra), and Nipah virus (Nipah).

More preferably, the virus belongs to the subfamily Paramyxoviridae (including the genus Respirovirus, the genus Rubulavirus, and the genus Morbillivirus) or is a derivative thereof. Still more preferably, the virus belongs to the genus Respirovirus (also called the genus Paramyxovirus) or a derivative thereof. Such derivatives include viruses genetically modified or chemically modified without impairing the virus's ability to introduce genes. Examples of viruses of the genus *Respirovirus* to which the present invention can be applied include human parainfluenza virus type 1 (HPIV-1), human parainfluenza virus type 3 (HPIV-3), bovine parainfluenza virus type 3 (BPIV-3), Sendai virus (also called murine parainfluenza virus type 1), and simian parainfluenza virus type 10 (SPIV-10). The paramyxovirus of the present invention is most preferably the Sendai virus. These viruses may be derived from natural strains, wild-type strains, mutant strains, laboratory-passaged strains, artificially constructed strains, etc.

Reconstitution of the recombinant minus-strand RNA viral vector can be achieved by using known methods (WO 97/16539; WO 97/16538; WO 00/70055; WO 00/70070; WO 03/025570; Durbin, A. P. *et al.,* Virology 235, 323-332, 1997; Whelan, S. P. *et al.,* Proc. Natl. Acad. Sci. USA 92, 8388-8392, 1995; Schnell. M. J. *et al.,* EMBO J. 13, 4195-4203, 1994; Radecke, F. *et al.,* EMBO J. 14, 5773-5784, 1995; Lawson, N. D. *et al.,* Proc. Natl. Acad. Sci. USA 92, 4477-4481, 1995; Garcin, D. *et al.,* EMBO J. 14, 6087-6094, 1995; Kato, A. *et al.,* Genes Cells 1, 569-579, 1996; Baron, M. D. and Barrett, T., J. Virol. 71, 1265-1271, 1997; Bridgen, A. and Elliott, R. M., Proc. Natl. Acad. Sci. USA 93, 15400-15404, 1996; Hasan, M. K. *et al.,* J. Gen. Virol. 78: 2813-2820, 1997; Kato, A. *et al.,* 1997, EMBO J. 16: 578-587; Yu, D. *et al.,* 1997, Genes Cells 2: 457-466). The minus-strand RNA virus, including parainfluenza, vesicular stomatitis virus, rabies virus, measles virus, rinderpest virus, and Sendai virus, can be reconstituted from its DNA using such methods. The viruses of the present invention can be reconstituted according to these methods. As for the DNA that constitutes the viral genome, when genes encoding the envelope-constituting proteins, such as the F gene, HN gene, and/or M gene, have been deleted from the viral genome, infectious viral particles are not formed automatically. However, infectious viral particles can be formed when the deleted genes and/or envelope proteins from another virus (for example, the gene encoding vesicular stomatitis virus (VSV) G protein (VSV-G) (J. Virology 39: 519-528 (1981)) are separately introduced into the host cells and expressed in the cells (Hirata, T. *et al.,* 2002, J. Virol. Methods, 104:125-133; Inoue, M. *et al.,* 2003, J. Virol. 77:6419-6429).

Transcription/replication of the minus-strand RNA virus takes place only in the host cell cytoplasm and since the virus has no DNA phase, the virus is not integrated into the chromosome (Lamb, R.A. and Kolakofsky, D., *Paramyxoviridae:* The viruses and their replication. In: Fields BN, Knipe DM, Howley PM, (eds). Fields Virology, 3rd Edition, Vol. 2. Lippincott - Raven Publishers: Philadelphia, 1996, pp. 1177-1204). Therefore, the vector does not cause safety problems, such as canceration and immortalization due to chromosomal abnormalities. This characteristic of minus-strand RNA viruses largely contributes to its safety when used as a vector. According to the results of heterologous gene expression, for example, even after many generations of continuous passages of a Sendai virus (SeV), almost no nucleotide mutations were found; thus the genome stability was high and the inserted heterologous genes could be expressed stably for a long period (Yu, D. *et al.,* Genes Cells 2, 457-466 (1997)). In addition, the virus's packaging flexibility and the flexible gene size to be introduced are advantageous since the virus has no capsid proteins. Furthermore, the Sendai virus is pathogenic and causes pneumonia in rodents, but is not pathogenic in human. This is further supported by previous reports that the wild-type Sendai virus produces no serious adverse effects in non-human primates when introduced nasally (Hurwitz, J.L. *et al.,* Vaccine 15: 533-540, 1997; Bitzer, M. *et al.,* J. Gene Med. 5: 543-553, 2003). Thus, the minus-strand RNA viral vector is highly useful as a therapeutic vector in gene therapy for human ischemic diseases.

The collected viral vector can be purified to substantial homogeneity. The purification can be achieved by using conventional purification/separation methods such as filtration, centrifugation, adsorption, and column purification, or any combination thereof. The phrase "substantially pure" means that the viral components constitute a large share of the solution that contains the viral vector. For example, a viral vector composition can be verified to be substantially pure if the proteins contained as the viral vector components constitute 10%(W/W) or more, preferably 20% or more, more preferably 50% or more, preferably 70% or more, more preferably 80% or more, still more preferably 90% or more of the total proteins (excluding the proteins that have been added as carriers or stabilizers) in a solution. For example, when the paramyxovirus vector is used, specific purification methods include, but are not limited to, the method using cellulose sulfate ester or crosslinked polysaccharide sulfate ester (Japanese Patent Application Kokoku Publication No. (JP-B) S62-30752 (examined, approved Japanese patent application published for opposition); JP-B S62-33879; and JP-B S62-30753), and the method based on the adsorption by fucose sulfate-containing polysaccharide and/or degradation products thereof (WO 97/32010).

Herein, "ischemic diseases" refers to functional abnormality, or tissue degeneration or necrosis, caused by the reduction or disruption of blood supply to tissues, and specifically comprises ischemic heart diseases such as myocardial infarction and angina pectoris, and extremity ischemia, injuries associated with impaired circulation, traumatic injuries such as amputation, and fractures. Specifically, the ischemic diseases of the present invention include not only ischemic diseases but also ischemic states as a result of injury or damage. Administration of Ang1 or an Ang1-encoding vector suppresses necrosis of tissues surrounding the ischemic site and improves their functions through induction of angiogenesis, and other effects such as anti-apoptotic effect and anti-inflammatory effect. Preferably, VEGF is not included in the Ang1 administration of the present invention. Significant therapeutic effects can be produced by administering Ang1 alone without administering VEGF. The phrase "without administering VEGF" specifically means that VEGF or a vector encoding VEGF is not administered within at least 12 hours, preferably within 24 hours, more preferably within 14 days before or after the administration of Ang1 or an Ang1-encoding vector. However, as long as not a significant activity of VEGF is detectable after the administration of a small or trace amount of VEGF or a VEGF-encoding vector, VEGF is considered not to be administered. Vascular Endothelial Growth Factor (VEGF) is a growth factor specific to vascular endothelial cells, currently classified into VEGF A, B, C, D, and E, and was reported as the Vascular Permeability Factor (VPF) in 1989 (Shibuya M., "VEGF receptor and signal transduction" SAISHIN IGAKU 56:1728-1734, 2001). VEGF A is further divided into six subtypes. Among them, soluble VEGF 121 and 165, in particular, have a strong vascular proliferation ability and are currently used in clinic. VEGF of the present invention includes particularly VEGF165 and VEGF121, and preferably various VEGF members comprising VEGF165 and VEGF121. In particular, the therapeutic methods of the present invention are highly effective for ischemic diseases which are accompanied by an elevation of the endogenous VEGF level. The expression "endogenous VEGF level is elevated" means that the endogenous VEGF level in blood or in local tissues is higher than in healthy individuals. The endogenous VEGF level is elevated in the above-described myocardial infarction, angina pectoris, acute extremity ischemia, injuries associated with impaired circulation, amputation, fractures, or such.

Herein, "ischemic heart diseases" refers to functional abnormality of the heart, or cardiac muscle degeneration or necrosis caused by the reduction or disruption of blood supply to cardiac muscle, and includes specifically angina pectoris, myocardial infarction, and some types of cardiomyopathy. Angiogenesis is enhanced and cardiac functions are improved by administering Ang1 or an Ang1-encoding vector into ischemic hearts. The methods of the present invention are highly effective for ischemic heart diseases which are accompanied by elevation of the endogenous VEGF level, and are appropriately used to treat, for example, angina pectoris, myocardial infarction, and ischemic cardiomyopathy (Xu, X., *et al.* (2001) J Thorac Cardiovasc Surg. 121: 735-742; Banai, S., *et al.* (1994) Cardiovasc Res. 28: 1176-1179; Sellke, F.W., *et al.* (1996) Am J Physiol. 271: H713-720), etc. The ischemic heart disease to which the methods of the present invention are most effective is myocardial infarction.

"Angina pectoris" refers to a clinical syndrome with the main symptom as chest discomfort caused by transient ischemia, namely lack of oxygen, in the cardiac muscle (Ogawa H., "pharmacotherapy for angina pectoris", supplementary volume, Journal of Clinical and Experimental Medicine (Igaku no Ayumi); circulatory diseases: 352-355,1996, Eds. Yazaki Y. *et al.* Ishiyaku Publisher Inc.). "Acute myocardial infarction" is an ischemic heart disease, where necrosis of the cardiac muscle is caused by obstruction of blood flow in the coronary arteries (Abu M., and Takano T., Acute myocardial infarction: The latest therapy for circulatory diseases 2002-2003 II, coronary artery diseases: 37-42, 2002, eds., Shinoyama, S. and Yazaki Y., Nankodo Co. Ltd.).

When treating an ischemic disease according to the present invention, it is preferable that neither VEGF nor any other angiogenesis factors or angiogenesis factor-encoding vectors are administered. An "angiogenesis factor" refers to a factor that is directly or indirectly involved in the development, migration, proliferation, or maturation of cells which participate in vascularization. Specifically, vascular endothelial growth factors (VEGFs), fibroblast growth factors (FGFs), epithelial growth factor (EGF), hepatocyte growth factor (HGF), placenta-derived growth factor (PDGF), monocyte chemoattractant protein-1 (MCP-1), thymidine phosphorylase (TP), angiopoietin, ephrin(Eph), matrix metalloproteinase (MMP), and tissue inhibitor of metalloproteinase (TIMP) (Kuwano, M. *et al.,* Angiogenesis Int. Med. 40: 565-572 (2001); Freedman, S. B. *et al.,* Ann. Intern. Med. 136:54-71 (2002)) are included. The phrase "not administered" means a dosage that would lead to a significantly detectable effect of such an angiogenesis factor or a dosage higher than that is not administered to an individual.

Ang1 or an Ang1-encoding vector is administered systemically or locally to ischemic tissues. Ang1 does not produce marked adverse effects even when administered at high doses. Therefore, ischemia can be treated by administering Ang1 systemically. Ang1 or an Ang1-encoding vector may be introduced directly or *via* carriers. The carrier should be physiologically acceptable, and includes organic substances such as biopolymer and inorganic substances such as hydroxyapatite; specifically, collagen matrix, polylactic acid polymer or copolymer, polyethylene glycol polymer or copolymer, and chemical derivatives thereof. Furthermore, the carrier may be mixed compositions of the physiologically acceptable materials described above. The vector to be used is not limited as long as it is a physiologically acceptable vector, and desired vectors including viral vectors and non-viral vectors can be used. The vector can be administered in the form of a vector-treated cell derived from the patient him/herself. For example, the vector or cells into which the vector has been introduced can be administered via intramuscular injection (to cardiac muscle or skeletal muscle) or intravenous injection *(in vivo* and *ex vivo* administration). Cells which have been administered systemically (via intramuscular or intravenous injection) can transfer to lesion sites and enhance the survival of ischemic tissues. For example, it has been recently reported that mesenchymal stem cells (MSCs) not only differentiate into bone cells, cartilage cells, adipocytes, and such, but also retain the ability of differentiating into skeletal muscles, cardiac muscles, and neurons. Thus, these stem cells are being studied intensively as a cell source for regenerative medicine. Excellent therapeutic effects are expected from introducing Ang-1 gene into MSCs according to the present invention and using the resulting cells for ischemia treatment. MSCs can be prepared, for example, by the method described in Tsuda, H. *et al.* Mol Ther 7(3): 354-65 (2003). For local administration to the heart, Ang1 or an Ang1-encoding vector can be injected into the cardiac muscle. Alternatively, cells into which an Ang1-encoding vector has been introduced may be transplanted into the cardiac muscle *(ex vivo* administration). The injection can be achieved using manufactured products such as standard medical injectors, and external and indwelling continuous infusers.

In the case of a virus, the dosage can be administered, for example, at one or more sites (for example, two to ten sites) in the surviving muscle (skeletal muscle, cardiac muscle, or such) surrounding the ischemic site. In the case of an adenovirus, the dosage preferably ranges from, for example, $10^{10}$ to $10^{13}$ pfu/body, and more preferably $10^{11}$ to $10^{13}$ pfu/body. The dosage of a minus-strand RNA virus preferably ranges from, for example, 2x $10^5$ to 5x $10^{11}$ CIU. A naked DNA can be administered at one or more sites (for example, two to ten sites) in the surviving muscle surrounding the ischemic site. The injection dosage per site preferably ranges from, for example, 10 μg to 10 mg, and more preferably 100 μg to 1 mg. When performing an *ex vivo* administration of cells into which a vector has been introduced, the vector is introduced into the target cells (for example, in a test tube or dish) *ex vivo,* for example, at a multiplicity of infection (MOI) of 1 to 500. In the present invention, minus-strand RNA viral vectors have been found to introduce foreign genes into mesenchymal cells with exceedingly high efficiency. Accordingly, when mesenchymal cells are used in an *ex vivo* administration, it is preferable to use a minus-strand RNA viral vector to introduce genes into the mesenchymal cells. When Ang-1 gene-introduced cells are used, for example, $10^5$ to $10^9$ cells, and preferably $10^6$ to $10^8$ cells can be transplanted to ischemic tissues. When a protein preparation is used, it may be administered at one or more sites (for example, two to ten sites) in the surviving muscle surrounding the ischemic site. The dosage preferably ranges from, for example, 1 μg/kg to 10 mg/kg, and more preferably 10 μg/kg to 1 mg/kg. Alternatively, the vector or the protein preparation may be administered, for example, several times (one to ten times) to the artery that leads to the ischemic tissue (for example, the coronary artery of an ischemic heart). In such cases, when a protein preparation is used, the dosage per site preferably ranges from, for example, 1 μg/kg to 10 mg/kg, and more preferably 10 μg/kg to 1 mg/kg. A vector or protein preparation may be administered intravenously several times (one to ten times) or it may be administered continuously. In such cases, when a protein preparation is used, the total dosage preferably ranges from, for example, 1 μg/kg to 10 mg/kg, and more preferably 10 μg/kg to 1 mg/kg. When a vector is used, it may be administered at the same dosage as described above for the intramuscular injection. See, Freedman SB *et al.* Ann Intern Med 136:54-71 (2002), for dosage.

However, the vector dosage may vary depending on patient's weight, age, sex, and symptoms; dosage form of the composition to be administered; method of administering the vector; and so on. Those skilled in the art can appropriately adjust the dosage. The frequency of administration may range from one to several times within clinically acceptable limits of adverse effects. There may be one or more site of administration. The per kg dosage for non-human animals may be the same as that for human, or can be alternatively converted from the above-described dosage, for example, based on the volume ratio (for example, average value) between the ischemic organs (such as heart) of the subject animal and human. Animals subjected to the treatments of the present invention include human and other desired mammals, specifically, human, monkey, mouse, rat, rabbit, sheep, cow, and dog.

The therapeutic methods of the present invention can be conducted singly or in combination with other standard or advanced methods. For example, the methods of the present invention for treating ischemic heart diseases can be used preferably in combination with surgical revascularization, such as percutaneous transluminal coronary angioplasty (PTCA) or coronary artery bypass graft (CABG). Combined use with the therapeutic methods of the present invention can actively improve cardiac functions and reduce the period confined to bed. Treatments using Ang1 of the present invention are also expected to be more effective when used in combination with therapeutic methods that enhance remodeling in the infarcted region, for example, regeneration of infarcted myocardium. While the Ang1 gene therapy increases the infarct thickness, it has a relatively weak effect on improving the diastolic parameters such as LVAd and Edd, whose improvements are seen at the same time that a deficiency of the absolute mass of cardiac muscle is improved through combined use of cell therapy or such. The improvement by Ang1 of systolic volume, ejection fraction, and so on, is assumed to prevent the hypofunction of peri-infarct muscle by increasing the vascular density in the surviving cardiac muscle such as peri-infarct muscle, and then to also improve functions of the cardiac muscle by enhancing the compensatory hypertrophy of the surviving cardiac muscle. Therefore, it is preferable to combine its use with a cell therapy which compensates for the deficiency of the absolute mass of cardiac muscle, when considering transplantation of fetal cardiac muscle, ES cells, myoblasts, mesenchymal cells, or such, or induction of cell migration to infarcted sites. It is also useful to enhance the therapeutic effect on ischemic tissues by *ex vivo* introduction of Ang-1 gene into these cells.

The present invention also provides therapeutic agents comprising Ang1 or an Ang1-encoding vector for ischemic heart diseases. The present invention also provides uses of Ang1 or an Ang1-encoding vector in administering to ischemic hearts for the treatment of ischemic heart diseases. Furthermore, the present invention also provides uses of Ang1 or an Ang1-encoding vector in producing therapeutic agents for ischemic heart diseases, which are used to administer Ang1 or an Ang1-encoding vector to ischemic hearts. In particular, the present invention provides therapeutic agents comprising Ang1 or an Ang1-encoding vector for ischemic heart diseases, to administer Ang1 or an Ang1-encoding vector to ischemic hearts without the administration of VEGF or a VEGF vector. In addition, the present invention also provides uses of Ang1 or an Ang1-encoding vector for the treatment of ischemic heart diseases, in which Ang1 or an Ang1-encoding vector is administered to ischemic hearts without the administration of VEGF or a VEGF vector. Furthermore, the present invention also provides uses of Ang1 or an Ang1-encoding vector in producing therapeutic agents for ischemic heart diseases, which are used to administer Ang1 or an Ang1-encoding vector to ischemic hearts without the administration of VEGF or a VEGF vector. Regarding the therapeutic agents and uses described above, it is preferable that neither VEGF nor any other angiogenesis factors or angiogenesis factor-encoding vectors are administered. Furthermore, Ang1 or an Ang1-encoding vector is preferably formulated for local administration to ischemic hearts. For example, such a formulation is preferably administered by injection into the cardiac muscle. The Ang1-encoding vector is preferably a viral vector or a naked DNA that encodes Ang1. The viral vector is not particularly limited, but adenoviral vectors and minus-strand RNA viral vectors are particularly preferable. The naked DNA includes plasmids, which may be circular or linear. Preferably, the plasmid does not contain SV40ori. The vector promoter which drives the transcription of Ang1 preferably has strong transcriptional activity. For example, a CA promoter can be used suitably.

The present invention also relates to kits for treating ischemic heart diseases, which comprise: (a) Ang1 or an Ang1-encoding vector, and (b) a recording medium containing a description of instruction that VEGF or a VEGF vector should not be administered when Ang1 or an Ang1-encoding vector is administered, or a link to the description. The kits are to be used for treating at least one of ischemic heart diseases including myocardial infarction and angina pectoris. The kits of the present invention are preferably used to treat angina pectoris and/or acute myocardial infarction. The kits comprise the Ang1 or the Ang1-encoding vector described above. The Ang1-encoding vector is preferably a naked DNA or a viral vector that encodes Ang1. The viral vector is not particularly limited, but adenoviral vectors and minus-strand RNA viral vectors, in particular, are preferred. The Ang1 or Ang1-encoding vector in the kits may be a composition that comprises in addition to Ang1, a desired pharmaceutically acceptable carrier and/or additive. For example, the composition may comprise sterilized water, physiological saline, a standard buffer (such as phosphoric acid, citric acid, and other organic acids), a stabilizer, salt, an antioxidant (such as ascorbic acid), a detergent, a suspending agent, an isotonizing agent, or a preservative. For local administration, the Ang1 or Ang1-encoding vector is preferably combined with an organic substance such as biopolymer, an inorganic substance such as hydroxyapatite, specifically collagen matrix, polymer or copolymer of polylactic acid, polymer or copolymer of polyethylene glycol, and derivatives thereof. In a preferred embodiment, the Ang1 or Ang1-encoding vector is prepared in a dosage form suitable for injection. For this purpose, the Ang1 or Ang1-encoding vector is preferably dissolved in a pharmaceutically acceptable aqueous solution, or is preferably a soluble freeze-dry formulation or such. The kits of the present invention may further comprise a desired pharmaceutically acceptable carrier that can be used to dissolve or dilute the Ang1 or Ang1-encoding vector. Such a carrier includes, for example, distilled water and physiological saline.

The present invention also provides therapeutic agents for ischemic diseases, comprising an Ang1-encoding viral vector. The present invention also provides uses of an Ang1-encoding viral vector for the treatment of ischemic diseases. In addition, the present invention provides uses of an Ang1-encoding viral vector for the production of therapeutic agents for ischemic diseases, wherein the therapeutic agents comprises the Ang1-encoding viral vector. In particular, the present invention provides therapeutic agents for ischemic diseases, comprising an Ang1-encoding viral vector and which are used to administer the Ang1 viral vector to an individual with ischemia without the administration of VEGF or a VEGF vector. In addition, the present invention provides uses of an Ang1-encoding viral vector for the treatment of ischemic diseases, in administering the Ang1 viral vector to an individual with ischemia without the administration of VEGF or a VEGF vector. Furthermore, the present invention provides uses of the Ang1 viral vector in producing therapeutic agents for ischemic diseases, which are used to administer the Ang1-encoding viral vector to an individual with ischemia without the administration of VEGF or a VEGF vector. In the therapeutic agents and uses described above, neither VEGF nor any other angiogenesis factors or vectors encoding these factors are preferably administered. Furthermore, the Ang1-encoding viral vector is preferably formulated for local administration to ischemic tissues. The preferable viral vectors that can be used are adenoviral vectors and minus-strand RNA viral vectors.

The present invention also relates to kits for treating ischemic diseases, which comprise (a) an Ang1-encoding viral vector, and (b) a recording medium containing a description of instruction that VEGF or a VEGF vector should not be administered when the Ang1-encoding viral vector is administered, or a link to the description. The kits are kits for treating at least one of ischemic heart diseases such as myocardial infarction and angina pectoris, and ischemic diseases such as extremity ischemia, injuries associated with impaired circulation, traumatic injuries including amputation, and fractures. The kits comprise the Ang1-encoding viral vector described above. The viral vector is not particularly limited, but adenoviral vectors and minus-strand RNA viral vectors are particularly preferred. The Ang1-encoding viral vector in the kits may be a composition that comprises in addition to the vector, a desired pharmaceutically acceptable carrier and/or additive. For example, the composition may comprise sterilized water, physiological saline, a standard buffer (such as phosphoric acid, citric acid, and other organic acids), a stabilizer, salt, an antioxidant (such as ascorbic acid), a detergent, a suspending agent, an isotonizing agent, or a preservative. For local administration, the vector is preferably combined with an organic substance such as biopolymer, an inorganic substance such as hydroxyapatite, and specifically collagen matrix, polylactic acid polymer or copolymer, polyethylene glycol polymer or copolymer, and derivatives thereof. In a preferred embodiment, the Ang1-encoding viral vector is prepared in a dosage form suitable for injection. For this purpose, the Ang1-encoding viral vector is preferably dissolved in pharmaceutically acceptable aqueous solution, or is preferably a soluble freeze-dry formulation or such. The kits of the present invention may further comprise a desired pharmaceutically acceptable carrier that can be used to dissolve or dilute the Ang1-encoding viral vector. Such carriers include, for example, distilled water and physiological saline.

The kits of the present invention comprise a recording medium containing a description of instruction that VEGF or a VEGF vector should not be administered when Ang1 or an Ang1-encoding vector is administered, or a link to the description. "Instruction that VEGF or a VEGF vector should not be administered" refers to, for example, a description of instruction or recommendation that the administration of VEGF or a VEGF should be contraindicated or avoided. Specifically, the description instructs that VEGF or a vector encoding VEGF should not be administered within at least 12 hours before or after administering Ang1 or an Ang1-encoding vector. Preferably, the description instructs that VEGF or a vector encoding VEGF should not be administered within 24 hours, more preferably within 14 days before and after the administration of Ang1 or an Ang1-encoding vector. VEGF includes VEGF165 and VEGF 121, in particular, and preferably various members of VEGF including VEGF 165 and VEGF 121. The kits of the present invention preferably include a description of a therapeutically effective amount of Ang1 or an Ang1-encoding vector to be administered to affected individuals, or a link to the description. The recording medium includes desired recording media, for example, print media such as paper and plastic, and computer-readable recording media such as flexible disc (FD), compact disc (CD), digital video disc (DVD), and semiconductor memory. A typical example of the recording medium is an instruction attached to the kits. "Link" means that the description, which instructs that VEGF121 should not be administered when Ang1 or an Ang1-encoding vector is administered, is linked via a mark or the like in the kits so that the mark provides a shortcut to the description, rather than being directly enclosed in the kits. For example, the instruction may give directions or suggestions to refer to an attached sheet or URL when the description is contained in the attached sheet or URL.

Introduction of genes into mesenchymal cells using the minus-strand RNA viral vector is described below. The mesenchymal cells of the present invention refer to preferably bone marrow cells (the mononuclear cell fraction component of bone marrow cells), cord blood cells or peripheral blood cells, mesenchymal stem cells, cells derived from these cells or such. The mesenchymal cells of the present invention also include, for example, cells associated with mesenchyme, and mesodermal stem cells. Even if a cell that is described herein as a "mesenchymal cell" is classified into a non-mesenchymal cell in future, the cell can be suitably used in the present invention.

The bone marrow contains two stem cell types: hematopoietic stem cell and "mesenchymal stem cell (MSC)".

Herein, "stem cell" typically refers to an undifferentiated cell that has both the abilities of self-reproduction and differentiation into cells with particular functions in the physiological process of proliferation and differentiation of cells constituting the living body. "Hematopoietic stem cell" refers to a stem cell that differentiates into an erythrocyte, leukocyte, or platelet. A mesenchymal stem cell may differentiate into neuron, cardiovascular system, internal organ, bone, cartilage, fat, or muscle.

In the present invention, mesenchymal stem cells are mainly used; however hematopoietic stem cells and other stem cells (precursor cells) in the body may also be used. The mesenchymal stem cells can be obtained by separation from bone marrow cells collected from the bone marrow. Like the mesenchymal stem cells, bone marrow cells containing the mesenchymal stem cells, although less effective, can also be used in the therapy.

Furthermore, it is also possible that mesenchymal stem cell-like cells can be prepared from the peripheral blood. Thus, cells that have functions equivalent to those of the mesenchymal stem cells can be prepared by culturing cells in the peripheral blood and used in the present invention.

Herein, "mesodermal stem cell" refers to a cell that constitutes tissues which are classified as mesoderm embryologically including a blood cell. Furthermore, "mesodermal stem cell" refers to a cell that can make (divide or proliferate into) copies of cells with the same ability as itself, and which can differentiate into all types of cells that constitute mesodermal tissues. The mesodermal stem cell comprises, but is not limited to, those cells with markers such as SH2(+), SH3(+), SH4(+), CD29(+), CD44(+), CD14(-), CD34(-), and CD45(-). Furthermore, the so-called "mesenchyme-associated stem cells" are also included in the mesodermal stem cells of the present invention.

The above mesenchyme-associated cells refer to mesenchymal stem cells, mesenchymal cells, precursor cells of mesenchymal cells, and cells derived from mesenchymal cells.

"Mesenchymal stem cells" refer to stem cells that can be obtained, for example, from the bone marrow, peripheral blood, skin, hair root, muscle tissue, uterine endometrium, blood, cord blood, and primary cultures of various tissues. Cells having functions equivalent to the mesenchymal stem cells, which can be obtained by culturing cells in the peripheral blood, are also included in the mesenchymal stem cells of the present invention.

The preferred mesenchymal cells of the present invention include bone marrow cells and bone marrow stem cells (mesenchymal stem cells). In addition, examples of the preferred cells of the present invention are cord blood cells, peripheral blood cells, and fetal hepatocytes.

In a preferred embodiment of the present invention, bone marrow cells, cord blood cells, peripheral blood cells, and fetal hepatocytes include cell fractions separated from the bone marrow, cord blood, peripheral blood, or fetal liver, and cell fractions that can differentiate into cells of the cardiovascular system or myocardial cells.

In another embodiment, the cell fraction contains mesodermal liver cells with SH2(+), SH3(+), SH4(+), CD29 (+), CD44(+), CD14(-), CD34(-), and CD45(-).

In addition to the above, the cell fractions of the present invention include a cell fraction that contains interstitial cells comprising the following cell markers: Lin(-), Sca-1(+), CD10(+), CD11D(+), CD44(+), CD45(+), CD71(+), CD90 (+), CD105(+), CDw123(+), CD127(+), CD164(+), fibronectin(+), ALPH(+), and collagenase-1(+); and a cell fraction containing cells with the AC 13 3 (+).

The bone marrow cells, cord blood cells, or peripheral blood cells (cell fractions) of the present invention are typically derived from vertebrates. The cells are preferably derived from mammals (for example, mouse, rat, rabbit, pig, dog, monkey, and human), but are not limited thereto.

In the present invention, mesenchymal cells into which the Ang1 gene had been introduced were found to produce a stronger therapeutic effect on ischemia as compared with non-genetically modified mesenchymal cells. Thus, useful cells for treating ischemia can be prepared by introducing an exogenous Ang1 gene into mesenchymal cells. Angiogenesis and revascularization in ischemic tissues can be enhanced by administering these cells to the ischemic tissues. Ang1 gene can be introduced into mesenchymal cells using the plasmid vector described above, other naked DNAs, or viral vectors. The vector promoter is preferably a high efficiency promoter so that Ang1 is expressed at high levels in tissues to which the vector has been administered. The CA promoter described above is used preferably. In other preferred embodiments, Ang1 gene is introduced into mesenchymal cells using a viral vector. A particularly preferred viral vector includes an adenoviral vector and a minus-strand RNA viral vector. The minus-strand RNA viral vector is most preferably used. Ang1 gene can be expressed at exceedingly high levels in the mesenchymal cells by using a minus-strand RNA viral vector.

To introduce a gene into mesenchymal cells using a viral vector, mesenchymal cells are contacted with the viral vector carrying the gene to be introduced. The vector can be contacted with mesenchymal cells *in vivo* or *in vitro,* for example, in a desired physiological aqueous solution such as culture solution, physiological saline, blood, plasma, serum, and body fluids. In case of an *in vitro* introduction, the multiplicity of infection (MOI; the number of infecting virus particles per cell) is preferably adjusted within the range of 1 to 500, more preferably 1 to 300, even more preferably 1 to 200, still more preferably 1 to 100, and yet still more preferably 1 to 70. For example, the infection can be conducted by combining the viral vector with a cell fraction containing mesenchymal cells. When a minus-strand RNA viral vector is used, it is sufficient to contact the mesenchymal cells with the only vector in a brief period for infection. The contact time can bel minute or longer, preferably 3 minutes or longer, 5 minutes or longer, 10 minutes or longer, or 20 minutes or longer, for example, about 1 to 60 minutes, and more specifically about 5 to 30 minutes. Needless to say, the contact may be continued for a longer time, for example, 24 hours, or several days or longer. The contact can be achieved *in vivo* or *ex vivo*. For example, in *ex vivo* gene introduction that involves contacting a viral vector *ex vivo* with mesenchymal cells removed from the body and returning the cells to the body, gene introduction methods which utilize a minus-strand RNA viral vector and thereby require a short contact between the vector and mesenchymal cells are preferably used. Mesenchymal cells into which a gene associated with angiogenesis has been introduced by the methods of the present invention are useful in gene therapy for cardiac ischemia, extremity ischemia, and so on.

The mesenchymal cells can be prepared, for example, according to the method described in Tsuda, H. *et al.* Mol Ther 7(3): 354-65 (2003). As for the culturing of human mesenchymal cells, see the description in Kobune M, *et al.,* Hamada H, Telomerized human multipotent mesenchymal cells can differentiate into hematopoietic and cobblestone area-supporting cells Exp. Hematol Exp Hematol. 31(8):715-22, 2003. The prepared cells may be used for therapy immediately, or after being cultured *in vitro* to about 10 to 40 population doublings (PD).

More specifically, a cell fraction containing mesenchymal cells can be prepared by, for example, fractionation of bone marrow cells or cord blood cells collected from vertebrate by performing density gradient centrifugation at 2,000 rpm for long enough to separate the cells based on their specific gravity in the solution, and then collecting the cell fractions with a certain specific density in the range of 1.07 g/ml to 1.1 g/ml. Herein, the phrase "long enough to separate the cells based on their specific gravity" refers to a sufficient period of time which allows the cells to settle in a position that corresponds to their own specific gravity in the solution used for the density gradient centrifugation. The period of time typically ranges from about 10 minutes to 30 minutes. The specific gravity of the cell fraction to be collected preferably ranges from 1.07 g/ml to 1.08 g/ml (for example, 1.077 g/ml). Solutions to be used for the density gradient centrifugation include, but are not limited to, Ficoll solution and the Percoll solution. Alternatively, cord blood cells collected from vertebrate by the same procedure described above may be used as a cell fraction.

A specific example involves first mixing a solution (2 ml L-15 + 3 ml Ficoll) with a bone marrow fluid (5 μl to 10 μl) collected from vertebrate, and centrifuging the resulting mixture at 2,000 rpm for 15 minutes to separate a mononuclear cell fraction (about 1 ml). The cells were then washed by mixing the mononuclear cell fraction with a culture solution (2 ml of DMEM), and centrifuging the mixture at 2,000 rpm for 15 minutes for the second time. The supernatant is then discarded, and the precipitated cells are collected. In addition to the thighbone, the cell fractions of the present invention have sources including the sternum, and the iliac bone which constitutes the pelvis. The cell fractions can be obtained not only from these bones but also from other large bones. The cell fractions can be also collected from bone marrow fluids or cord blood stored in bone-marrow banks. When cord blood cells are used, the cell fraction can be collected from cord blood stored in bone-marrow banks.

In another embodiment of the present invention, the cell fraction is a mononuclear cell fraction isolated and purified from bone marrow cells, cord blood cells, or peripheral blood cells, comprising mesodermal stem cells (mesenchymal stem cells) that can differentiate into cells of the cardiovascular system. Cell fractions containing mesodermal stem cells can be obtained by selecting cells with cell surface markers, such as the above SH2, from the above-mentioned cell fractions, for example, from bone marrow cells, cord blood cells, or peripheral blood cells.

A cell fraction containing mesodermal stem cells (mesenchymal stem cell) that can differentiate into cells of the cardiovascular system can be prepared by fractionation of bone marrow cells and cord blood cells collected from vertebrate, by performing density gradient centrifugation at 900 g for long enough to separate the cells based on their specific gravity in the solution, followed by collecting cell fractions with a particular specific density in the range of 1.07 g/ml to 1.1 g/ml. Herein, the phrase "long enough to separate the cells based on their specific gravity" refers to a sufficient period of time that allows the cells to settle in a position corresponding to their own specific gravity in the solution used for density gradient centrifugation. The time typically ranges from about 10 minutes to about 30 minutes. The specific gravity of the cell fraction to be collected can vary depending on the type of animal species (for example, human, rat, and mouse) from which the cells are derived. Solutions for the density gradient centrifugation include Ficoll solution and Percoll solution, but are not limited thereto.

A specific example involves first mixing the bone marrow fluid (25 ml) or cord blood collected from vertebrate with an equal volume of PBS, centrifuging the resulting mixture at 900g for 10 minutes, and mixing the precipitated cells with PBS (the cell density is about 4x $10^7$ cells /ml) to remove the blood component. A 5-ml aliquot of the cells is then mixed with a Percoll solution (1.073 g/ml), followed by centrifugation of the mixture at 900 g for 30 minutes to separate the mononuclear cell fraction. The mononuclear cell fraction is mixed with a culture medium (DMEM containing 10% FBS and 1% antibiotic-antimycotic solution) for cell washing. The mixture is centrifuged at 2,000 rpm for 15 minutes and the supernatant is discarded. The precipitated cells are collected and cultured (at 37°C with 5% $CO_2$ in air).

In another embodiment of the present invention, the cell fraction is a mononuclear cell fraction separated from bone marrow cells or cord blood cells, containing interstitial cells that can differentiate into cells of the cardiovascular system. The interstitial cells are, for example, cells with the markers: Lin(-), Sca-1(+), CD10(+), CD11D(+), CD44(+), CD45(+), CD71(+), CD90(+), CD105(+), CDW123(+), CD127(+), CD164(+), fibronectin(+), ALPH(+), and collagenase-1(+). The cell fraction containing interstitial cells can be obtained, for example, by selecting cells with cell surface markers, such as Lin, from the above-described cell fractions obtained from bone marrow cells or cord blood cells by centrifugation.

Alternatively, the cell fraction can be prepared by fractionation of bone marrow cells or cord blood cells collected from vertebrate, by performing density gradient centrifugation at 800 g for long enough to separate the cells based on their specific gravity in the solution, followed by collecting cell fractions with a particular specific density in the range of 1.07 g/ml to 1.1 g/ml. Herein, the phrase "long enough to separate the cells based on their specific gravity" refers to a sufficient period of time that allows the cells to settle in a position corresponding to their own specific gravity in the solution used in the density gradient centrifugation. The period of time typically ranges from about 10 minutes to about 30 minutes. The specific gravity of the cell fraction to be collected preferably ranges from 1.07 g/ml to 1.08 g/ml (for example, 1.077 g/ml). Solutions used in the density gradient centrifugation include, but are not limited to, Ficoll solution and Percoll solution.

A specific example involves first mixing a bone marrow fluid or cord blood collected from vertebrate with an equal volume of the solution (PBS containing 2% BSA, 0.6% sodium citrate, and 1% penicillin-streptomycin), and mixing a 5-ml aliquot of the cell sample with a Ficoll/Paque solution (1.077 g/ml). The resulting mixture is then centrifuged at 800 g for 20 minutes to separate a mononuclear cell fraction. The mononuclear cell fraction is then mixed with a culture solution (Alfa MEM containing 12.5% FBS, 12.5% horse serum, 0.2% i-inositol, 20 mM folic acid, 0.1 mM 2-mercaptoethanol, 2 mM L-glutamine, 1 μM hydrocortisone, and 1% antibiotic-antimycotic solution). The resulting mixture is then centrifuged at 2,000 rpm for 15 minutes, and the supernatant is discarded. The precipitated cells are collected and cultured (at 37°C with 5% $CO_2$ in air).

In another embodiment of the present invention, the cell fraction is a mononuclear cell fraction separated from bone marrow cells, cord blood cells, peripheral blood cells, or fetal hepatocytes, and that contains cells which comprise the AC133(+) marker and can differentiate into cells of the cardiovascular system. The cell fraction can be obtained, for example, by selecting cells with the above cell surface marker AC133(+) from the above-described cell fractions, which are obtained from bone marrow cells, cord blood cells, or peripheral blood cells by centrifugation.

In another embodiment, the cell fraction can be prepared by fractionation of fetal hepatocytes collected from vertebrate, by performing density gradient centrifugation at 2,000 rpm for long enough to separate the cells based on their specific gravity in the solution, followed by collecting cell fractions with a specific density in the range of 1.07 g/ml to 1.1g/ml and then collecting cells with the marker AC133(+) from the cell fractions. Herein, the phrase "long enough to separate the cells based on their specific gravity" refers to a sufficient period of time that allows the cells to settle in a position corresponding to their own specific gravity in the solution used for the density gradient centrifugation. The period of time typically ranges from about 10 minutes to about 30 minutes. Solutions used in the density gradient centrifugation include, but are not limited to, Ficoll solution and Percoll solution.

A specific example involves first washing liver tissues collected from vertebrate in an L-15 solution, followed by enzyme treatment (in L-15 solution comprising 0.01% DNaseI, 0.25% trypsin, and 0.1% collagenase at 37°C for 30 minutes). The tissues are separated into single cells by pipetting. These single hepatocytes are centrifuged by the same procedure used to prepare the mononuclear cell fraction from the thigh bone. The obtained cells are washed, and then AC133(+) cells are collected from the washed cells using an anti-AC133 antibody. Thus, cells that can differentiate into cells of the cardiovascular system can be prepared from fetal hepatocytes. The collection of AC133(+) cells using an antibody can be achieved by using magnetic beads or a cell sorter (FACS and so on).

The cells comprised in the aforementioned cell fraction that can differentiate into cells of the cardiovascular system include, but are not limited thereto, mesodermal stem cells (mesenchymal stem cells), interstitial cells, and AC133-positive cells in the cell fractions described above.

The present invention also relates to methods for producing genetically modified oral squamous cells, comprising the step of contacting oral squamous cells with a minus-strand RNA viral vector. Furthermore, the present invention also relates to methods for producing genetically modified macrophages, comprising the step of contacting macrophages with a minus-strand RNA viral vector that carryies a gene. The present invention also relates to methods for producing genetically modified dendritic cells, comprising the step of contacting dendritic cells with a minus-strand RNA viral vector that carryies a gene. It was found that a minus-strand RNA viral vector can introduce genes into oral squamous cells, macrophages, and dendritic cells with an exceedingly higher efficiency, compared with an adenoviral vector which is generally expected to express high levels of an introduced gene. Thus, a minus-strand RNA viral vector is highly useful to introduce genes into oral squamous cells (including oral squamous carcinoma cells), macrophages, and dendritic cells. Specifically, the present invention relates to (i) methods for producing genetically modified oral squamous cells, comprising the step of contacting oral squamous cells with a minus-strand RNA viral vector carrying a gene; (ii) methods for producing genetically modified macrophages, comprising the step of contacting dendritic cells with a minus-strand RNA viral vector carrying a gene, and (iii) methods for producing genetically modified macrophages, comprising the step of contacting dendritic cells with a minus-strand RNA viral vector carrying a gene. Furthermore, the present invention also relates to genetically modified cells produced by the methods described above. Such a genetic modification of cells is useful for regulating the immune system in gene therapy for oral squamous cell carcinoma and gene therapy for cancers and immune diseases.

Brief Description of the Drawings

Fig. 1 is a graph showing the expression of LacZ gene, whose introduction was mediated by an adenovirus, in normal or infarcted rat hearts. An adenoviral vector (1x $10^9$ to 1x $10^{10}$ opu) carrying E. *coli* β-galactosidase gene was injected into the anterior cardiac wall of normal or infarcted rat hearts. The rats were sacrificed on 5 day after the gene introduction. The excised hearts were homogenized with a tissue lysis buffer. The β-galactosidase activity of the heart homogenates was assayed. Open bars indicate sham-operated hearts (normal) and gray bars indicate infarcted hearts.

Fig.2 presents photographs showing the distribution of LacZ-positive areas in the sham-operated rat hearts or the infarcted hearts. (A) An overall view of the X-gal-stained hearts. Top panel: normal (sham-operated) hearts in which AxCAZ3 (1x $10^{10}$ opu) was injected into the cardiac muscle; Middle panel: infarcted hearts into which physiological saline was injected; Bottom panel: infarcted hearts in which AxCAZ3 (1x $10^{10}$ opu) was injected into the cardiac muscle. The left-hand side images in each panel are views from the right ventricular side; central images in each panel are views from the abdominal side (frontal view); right-hand side images in each panel are views from the left ventricular side. Solid arrows (yellow) indicate the ligation sites in the left coronary artery (LAD), and broken arrows (white) indicate the injection sites. The regions marked by arrowheads (red) indicate the infarcted regions in cardiac muscles. (B) Cross sectional view of the X-gal-stained hearts with myocardial infarction. The infarcted hearts were cut horizontally at a midpoint position between the ligation site and the cardiac apex, and at a one-quarter position from the bottom. The pale gray areas are the infarcted regions; the dark gray areas are the X-gal-positive cardiac muscle. LV: the left ventricle; RV: the right ventricle.

Fig. 3 presents photographs showing the adenovirus-mediated expression of Ang1 in sham-operated hearts and infarcted hearts. Expression of human Ang1-specific mRNA in the normal and infarcted hearts was examined by PCR five days after the gene introduction. (A) Human Ang1-specific expression. Lane 1: a 100-base pair DNA ladder as a length marker; lane 2: positive control (HeLa cells infected with AxCAhAng1); lane 3: normal hearts; lane 4: hearts injected with AxCAZ3; lane 5: normal hearts injected with AxCAhAng1; lane 6: infarcted hearts injected with AxCAhAng1. (B) The expression of rat GAPDH as an internal control in corresponding cells and tissues. The most intense band in the length marker corresponds to 500 base pairs.

Fig. 4 presents graphs and a photograph showing the capillary density in various regions of infarcted hearts. The CD34-positive capillary density was determined for each region (A: infarcted wall; B: septal wall; and C: boundary region adjacent to the infarcted region). The number of capillaries stained with an anti-CD34 monoclonal antibody was counted in a blind manner. The capillary density is indicated in number/$mm^2$ unit. *:$p<0.01$.

Fig. 5 presents photographs showing the histological findings of sham-operated hearts and infarcted hearts. The hearts were excised four weeks after myocardial infarction and then subjected to Masson's trichrome staining (A-D), and immunostaining with an anti-CD34 monoclonal antibody (E-H) and an anti-α-SMA monoclonal antibody (I-L). The sham-operated hearts (A, E, and I); physiological saline controls (B, F, and J); adenovirus controls (C, G, and K); Ang1-treated hearts (D, H, and L) are shown. Bar represents 50 μm.

Fig. 6 presents photographs of the long axis views at end-systole and end-diastole from echocardiography. The cardiac functions were evaluated four weeks after myocardial infarction by echocardiography. The photographs show the long axis views obtained by two-dimensional echocardiography. Top panels: end-systole; Bottom panels: diastole. Areas between the two arrowheads correspond to the infarcted anterior walls. The areas marked with broken lines correspond to the left ventricular lumen.

Fig. 7 presents photographs showing the necrosis-suppressing effect by an Ang1-expressing adenoviral vector, which had been administered alone to a mouse model of acute lower limb ischemia.

Fig. 8 presents graphs showing the expression of LacZ in rat skeletal muscles (A) and hearts (B) as a result of the injection of naked DNA. An indicated amount of plasmid (20 μg) was injected into the femoral muscle of the lower limb or cardiac apex (n=4). β-gal activity was assayed using Galacto-light plus kit four days after the plasmid injection and is shown as ng activity of LacZ in muscle or heart. Bar represents the standard error.

Fig. 9 is a graph showing a comparison of the LacZ expression levels by the injection of naked DNA and the injection of an adenoviral vector into rat hearts. Either 20 μg of pCAZ2 or AxCAZ3 in various amounts was injected into cardiac muscles. Bar represent the standard error (n=4).

Fig. 10 presents graphs showing effects of the gene introduced into myocardial cells using an adenoviral (Ad) vector or a Sendai virus (SeV) vector. An adenoviral vector (AxCAZ3) or a SeV vector (SeVAng1), both encoding LacZ, was introduced into rat myocardial cells at varying MOIs and time of infection. The expression level of β-galactosidase was determined.

Fig. 11 presents graphs showing the gene introduction into cardiac muscle using an adenoviral vector or a Sendai viral vector via *in vivo* administration. The expression levels of the reporter gene were determined three days after the LacZ-expressing AdV or SeV was administered to the cardiac muscle.

Fig. 12 is a graph showing the organ distribution of gene expression by intravenous or intramyocardial administration of the Ad or SeV vector. SeVLacZ and AxCAZ3 were administered via the penile vein of normal rats at 1x $10^8$ CIU and 1x $10^{10}$ opu, respectively. The organs were excised 72 hours later and the expression levels of LacZ in the organs were determined. The expression levels of LacZ in the respective organs were also determined after intramyocardial administration of the SeV vector.

Fig. 13 presents graphs showing the therapeutic effect on myocardial infarction resulted from introduction of the Ang1 gene into infarcted rat hearts using a SeV vector. 5x $10^7$ CIU of SeVAng1 was injected evenly at two sites in the anterior wall of the left ventricle surrounding the LAD perfusion area. The size and thickness of infarcts determined after four weeks are shown.

Fig. 14 presents graphs showing the therapeutic effect on myocardial infarction resulted from introduction of Ang1 gene into infarcted rat hearts using a SeV vector. 5x $10^7$ CIU of SeVAng1 was injected evenly at two sites in the anterior wall of the left ventricle surrounding the LAD perfusion area. Vascular densities of the cardiac muscle determined after four weeks are shown.

Fig. 15 is a graph showing the therapeutic effect of introducing Ang1 gene with a SeV vector in a rat model of lower limb ischemia. 5x $10^7$ CIU of SeVAng1 was administered to rats treated with femoral artery ligation at two sites on the rectus femoris muscle. The blood flows were analyzed by laser Doppler blood-flow imaging for two weeks following ischemia. The ratios between the blood flow in ischemic lower limbs and that in normal lower limbs (tissue blood flow ratio: ischemic limb blood flow/ normal limb blood flow) are shown.

Fig. 16 is a graph showing gene introduction into mesenchymal cells (MSC) using an adenoviral (Ad) vector or a Sendai virus (SeV) vector. An adenoviral vector (AxCAZ3) or a SeV vector (SeVAng1), both encoding LacZ, was introduced into rat MSCs at various MOIs. The β-galactosidase expression was assayed.

Fig. 17 presents photographs showing X-gal-stained MSCs, into which the LacZ gene had been introduced using an adenoviral (Ad) vector or a Sendai virus (SeV) vector.

Fig. 18 is a graph showing the therapeutic effect using Angl-introduced MSCs towards the treatment of limb ischemia.

Fig. 19 presents graphs and photographs showing a comparison between the gene introduction into a cell line mediated by a SeV vector and by an adenoviral vector.

Fig. 20 presents graphs showing the gene introduction into an adenovirus-resistant human oral squamous carcinoma cell line using a SeV vector.

Fig. 21 is a graph showing the gene introduction into human macrophages and dendritic cells using a SeV vector.

## Best Mode for Carrying Out the Invention

Herein below, the present invention will be specifically described using Examples, however, it is not to be construed as being limited thereto. All publications cited herein are incorporated as a part of the present specification.

### [Example 1] Adenoviral VEGF and Ang1 expression vectors

Human VEGF gene was obtained by PCR cloning of cDNA derived from a human glioma cell line U251. The nucleotide sequence of the obtained VEGF gene was confirmed by BigDye Terminator method (Perkin-Elmer). Human Ang1 gene was PCR cloned from cDNA derived from human bone marrow cells, and the nucleotide sequence was confirmed by the same procedure described above. Comparison of the determined nucleotide sequence of the Ang1 gene with that registered under the accession number U83508 in GenBank suggested that they are identical, except that the nucleotide A at position 933 had been replaced with G. Despite of the nucleotide substitution, the amino acid sequence of Ang1 protein is identical to that of U83508 in GenBank. The cloned VEGF and Ang1 cDNAs were individually inserted between the restriction sites EcoRI and BglII of a pCAcc vector (WO 02/100441; Ito., Y., *et al.* (2002) Mol Ther. 5: S162) derived from pCAGGS (Niwa, H. *et al.* (1991) Gene. 108: 193-199). Thus, the respective VEGF and Ang1 expression vectors, pCAhVEGF and pCAhAng1, were prepared. Adenoviruses expressing either VEGF or Ang1 were prepared by the COS-TPC method developed by Saito *et al.* (Miyake, S., Proc. Natl. Acad. Sci. USA 93: 1320-1324 (1996)). The plasmids of pCAhVEGF and pCAhAng1 were digested with a restriction enzyme ClaI. The resulting gene expression units, each comprising a VEGF or Ang1 cDNA and a CA promoter, were inserted into the ClaI restriction site of the cosmid pAxcw (Nakamura, T. *et al.* (2002) Hum Gene Ther. 13: 613-626) comprising a portion of the adenovirus type 5 gene, to produce pAxCAhVEGF/Ang1. A DNA-terminal protein complex (TPC) comprising pAxCAhVEGF/Ang1 and full-length adenovirus type 5 was digested with a restriction enzyme EcoT22I, and then the product was introduced into 293 cells by a calcium phosphate coprecipitation method. Plaques which contain the modified adenovirus were then harvested (Graham, F. L. and A. J. van der Eb. (1973) Virology. 52: 456-467). The adenovirus from each plaque was confirmed based on its restriction enzyme digestion pattern. Furthermore, it was confirmed by PCR that the viruses were not contaminated with the wild-type virus. Thus, the respective adenoviral vectors AxCAhVEGF and AxCAhAng1 for expressing VEGF and Ang1 were prepared. The adenoviruses to be used for generating a rat model of myocardial infarction were purified by ultracentrifugation in a CsCl discontinuous density gradient and dialyzed against 10% glycerol/PBS (Kanegae, Y., *et al.* (1995) Nucleic Acids Res. 23: 3816-3821). The concentrations (optical density units/ml, opu/ml) of the purified adenoviral vectors were measured by the $A_{260}$ in the presence of 0.1% SDS and determined by using the following formula (Nyberg-Hoffman, C. *et al.* (1997) Nat Med. 3: 808-811):

$$opu = A_{260} \times (1.1 \times 10^{12})$$

The virus titers (plaque forming units: pfu) were determined by the limiting dilution analysis using 293 cells (Miyake, S., *et al.* (1996) Proc Natl Acad Sci U S A. 93: 1320-1324). AxCAZ3 expressing the E. *coli* β-galactosidase gene (Nakamura, T. *et al.* (2002) Hum Gene Ther. 13: 613-626) was used as a control adenovirus. This vector is the same as AxCAhAng1 except for the inserted cDNA. The opu/pfu ratios of the viral vectors: AxCAhAng1, AxCAhVEGF, and AxCAZ3, used in the present invention were 13.3, 28.0, and 80.0, respectively.

[Example 2] Adenoviral vector-mediated gene expression in infarcted hearts

Expression levels of foreign genes have been reported to be very low in infarcted hearts compared with normal hearts (Leor, J. *et al.* (1996) J Mol Cell Cardiol. 28: 2057-2067). Thus, prior to the start of the therapeutic experiment, it was examined whether genes introduced with an adenovirus were sufficiently expressed in rat models of myocardial infarction.

<u>Preparation of a myocardial infarction rat model</u>

A rat model of myocardial infarction was prepared according to the method of Pfeffer *et al.* (Pfeffer, M.A. *et al.* Cir. Res. 44: 503-512, 1979). Lewis rats (eight-week old, male, about 300 g body weight) were anesthetized by inhalation of diethyl ether and intraperitoneal injection of 70 mg/kg ketamine and 6 to 7 mg/kg xylazine, and then the rats were intubated. The rats were anesthetized by inhalation of 0.5% to 2.0% halothane under the conditions of: 200 to 250 ml minute ventilation, 3 ml tidal volume, 60 to 80 cycles/min respiratory rate, and 1 l/min of $O_2$; and left thoracotomy was then performed. The left anterior descending (LAD) branch was identified and then ligated at the height of the left atrial appendage using a 6-0 nonabsorbable suture (nylon suture). After ligation, the lungs were expanded by positive end-expiratory pressure. After the intercostal incision was closed carefully so as not to damage the lungs, the muscle layer and the skin were closed with a continuous suture. For the sham-operated control group, the rats were treated in the same surgical procedure except that the coronary artery was not ligated. After ligation of the left anterior descending branch, $5 \times 10^9$ opu/50 µl (total amount: $1 \times 10^{10}$ opu) of the adenoviral vector was introduced intramyocardially using a 30G needle on the right and left peripheries of the area estimated to be the perfusion area of the left anterior descending branch.

<u>Examination of intramyocardial LacZ gene expression</u>

Expression of E. *coli* β-galactosidase gene, which resulted from the administration of the adenoviral vector, were confirmed in the rat cardiac muscles by X-gal staining (Nakamura, Y., *et al.* (1994) Cancer Res. 54: 5757-5760). Five days after intramyocardial administration of $1 \times 10^{10}$ OPU/100 µl of AxCAZ3, organ fixation was performed by perfusing the whole body with 2% paraformaldehyde under deep anesthesia. The fixed hearts were excised, and then stained with X-gal (Sigma Chemical Co. St. Louis, MO) by 16 hours of immersion in an X-gal solution (PBS (pH 7.2) containing 2 mM $MgCl_2$, 4 mM potassium ferricyanide, and 1 mg/ml X-gal) at 30°C. Furthermore, the expression levels of β-galactosidase in the hearts were examined quantitatively by assaying the β-galactosidase enzymatic activity (Shaper NL *et al.* J. Biol. Chem. 269(40), 25165-25171, 1994) using the Galacto-Light Plus Kit (Tropix Inc. Bedford, MA) and a standard β-galactosidase sample (Roche). The rats were sacrificed five days after intramyocardial administration of $1 \times 10^9$ to $1 \times 10^{10}$ opu of AxCAZ3. The excised hearts were homogenized in a lysis buffer (100 mM potassium phosphate (pH 7.8), 0.2% Triton X-100, 1 mM dithiothreitol, 0.2 mM phenylmethylsulfonyl fluoride, and 5 µg /ml leupeptin). The homogenates were centrifuged at 12,500 xg for 10 min, and then the endogenous β-galactosidase in the supernatants was inactivated by one hour incubation at 48°C (Young DC, Anal. Biochemi. 215, 24-30, 1993).

The supernatants were incubated with Galacto-Light Plus at room temperature for one hour. The enzymatic activities in the supernatants were determined by chemical luminescence using Mini-Lumat LB9506 (Berthold Technologies GmbH & Co. KG, Wildbad, Germany). The results obtained (in relative light units) were converted into β-galactosidase activities (pg/ml) using a standard curve prepared with the standard sample of recombinant β-galactosidase (Roche Diagnostics, Mannheim, Germany).

The adenoviral vector was administered intramyocardially by the procedure described below. After the left anterior descending branches were ligated, 5x $10^9$ opu/50 μl (total amount of vector used was 1x $10^{10}$ opu) of the adenoviral vector was administered intramyocardially using a 30G needle at two sites: the right and left peripheries of an area estimated to be the perfusion area for the left anterior descending branch. The adenoviral vector was divided and administered intramyocardially at two sites in each of the normal hearts and infarcted hearts. The expression levels were determined five days after the administration. As shown in Fig. 1, when a 5x $10^9$ opu or higher dose of the adenoviral vector was administered, the gene expression in both the normal and infarcted hearts was clearly recognized, and the expression level in the infarcted hearts was almost equivalent to that in the normal hearts. The introduced gene was expressed in a dose-dependent manner and the expression level increased with an increasing dosage of adenovirus. As shown in Fig. 2 (A), the gene expression distribution in the infarcted hearts was a broad region in the center of the anterolateral wall, which was the site of gene introduction. However, as clearly seen in the X-gal stained cross sectional view (Fig.2 (B)), the gene expression was not recognized in the cardiac muscles of the infarcted region, the septal area, and the right ventricular cardiac muscle.

[Example 3] Post-myocardial infarction survival rate after introduction of the VEGF and Ang1 genes

Since the gene expression from 1x $10^{10}$ opu of adenovirus was clearly recognized in the infarcted cardiac muscle, the angiogenesis factor gene was used to treat the rat myocardial infarction model. The therapeutic effect of VEGF gene on myocardial infarction, previously shown to be effective for chronic myocardial ischemia, was examined at the same time. The survival rates in the untreated post-myocardial infarction group, adenovirus-administered control group, AxCAhVEGF-administered group, and AxCAhAng1-administered group were calculated four weeks after myocardial infarction. Rats that had died within 24 hours of the model preparation were eliminated in this calculation.

Ang1 expression in the hearts, into which the vector had been introduced, was also examined by RT-PCR (Fig. 3). The hearts were excised five days after the adenoviral vector-mediated gene introduction (1x $10^{10}$ opu/heart). Total RNAs were extracted from the left ventricular cardiac muscle using RNeasy Kit (Qiagen KK, Tokyo, Japan). To avoid DNA contamination in the total RNAs of cardiac muscle, the samples were treated with DNaseI using RNase-free DNase Set (Qiagen) according to the attached instruction. The first cDNA strands were synthesized from the total RNAs by a random priming method using a random primer mixture (Invitrogen, Carlsbad, CA) and Superscript™ II (Invitrogen). The human Ang1-specific mRNA transcribed from the adenoviral vector was detected using a forward primer that is human Ang1-specific and a reverse primer for the rabbit β-globulin located at the terminator site of the Ang1 expression unit. The internal control rat glyceraldehyde 3-phosphate dehydrogenase (GAPDH) was also detected by RT-PCR. The human Ang1 forward primer, rabbit β-globulin reverse primer, and GAPDH primer are shown below.

Human Ang1 primer:

Forward primer: 5'-CAGAGGCAGTACATGCTAAGAATTGAGTTA-3' (SEQ ID NO: 6)

Rabbit β-globulin primer:

Reverse primer: 5'-AGATGCTCAAGGGGCTTCATGATG-3' (SEQ ID NO: 7)

Rabbit GAPDH primer:

Forward primer: 5'-TATTGGGCGCCTGGTCACCA-3' (SEQ ID NO: 8)

Reverse primer: 5'-CCACCTTCTTGATGTCATCA-3' (SEQ ID NO: 9)

Thirty cycles of PCR were performed, and human Ang1 mRNA and GAPDH mRNA were detected. The resulting PCR products were separated on a 2% agarose gel. Total RNA was extracted from HeLa cells that had been infected with AxCAhAng1 at 100 opu/cell, and used as a positive control for the human Ang1 mRNA.
The 407-bp product corresponding to rat GAPDH gene (internal control) was found in all the cardiac muscle RNA samples (Fig. 3). A 453-bp PCR band specific to human Ang1 was found in the rat heart samples administered with AxCAhAng1 and in the HeLa cell samples, into which the gene had been introduced using AxCAhAng1. In contrast, the human Ang1-specific band was detectable neither in the normal hearts nor in the AxCAZ3-administered hearts.
The mortality rate in the myocardial infarction model was approximately 25%, excluding rats that had died within 24 hours of the model preparation. In the control group where adenovirus AxCAZ3 had been administered, the mortality rate was 20% and thus the administered adenovirus had little influence on the mortality rate. First, the therapeutic effect of VEGF gene on myocardial infarction was examined; the gene was previously reported to be effective for chronic myocardial ischemia. Rather, the mortality rate in the VEGF gene-administered group was found to have risen to approximately 40% four weeks after myocardial infarction. In contrast, in the group into which 1x $10^{10}$ opu of AxCAhAng1 had been administered, the mortality rate was decreased to 8% (Table 1).
These findings indicate that Ang1 is more effective for acute myocardial infarction than VEGF.

Table 1

| Survival rate of rats affected with experimental myocardial infarction | | |
|---|---|---|
| | | Survival rate (%) |
| Sham operation | | 75.9 (18/25) |
| AxCAZ3 | 1 x $10^{10}$ opu | 81.3 (13/16) |
| AxCAhVEGF | 1 x $10^{10}$ opu | 60.0 (6/10) |
| AxCAhAngl | 1 x $10^{10}$ opu | 92.0 (23/25) |

[Example 4] Angiogenesis induced by Ang1 and VEGF genes after myocardial infarction

VEGF gene has a strong angiogenesis-inducing activity. Ang1 potentiates angiogenesis through its cooperation with VEGF. To directly demonstrate the effect of the introduced Ang1 gene, the vascular density in the infarcted hearts into which the gene had been introduced was determined. Four weeks after the creation of myocardial infarction, the vascular density in the cardiac muscle was evaluated by immunostaining of vascular endothelial cells with an anti-CD34 monoclonal antibody. The hearts were fixed with formalin and embedded in paraffin, and then sliced into 10-μm sections. The sections were stained with an anti-CD34 monoclonal antibody (MoAb) (NU-4A1, Nichirei, Tokyo Japan) as the primary antibody, and then with a biotinylated anti-mouse IgG secondary antibody and avidin-horseradish peroxidase (DAB paraffin IHC staining module, Ventana Medical System Inc, Tucson, AZ). The specificity of the primary antibody was verified using a mouse IgG, which has an identical subtype as the antibody. The number of blood vessels in the sections of the interventricular septum, peripheral region of the infarcted site, and surviving cardiac muscles in the myocardial infarct were determined in a blind manner under a microscope with 200 times magnification. Five randomly selected fields were scored for each of the 40 sections per heart. The stained blood vessels in the infarcted region, boundary region, and septal wall were counted, and the results are represented as average values of blood vessel number per unit area (mm2). To confirm the presence of mature blood vessels, the sections were also stained with an anti-α-SMA MoAb (clone 1A4, Dako Japan, Tokyo, Japan) by the same procedure used for the anti-CD34 MoAb immunostaining. The α-SMA-positive blood vessels were counted in a similar manner as for the capillary density described above.
The vascular densities in the infarcted site and the peri-infarct myocardium of the infarcted hearts were found to have decreased as compared with the normal hearts (Fig. 4). When VEGF or Ang1 was administered using an adenovirus, the vascular density was significantly increased in the infarcted site and the peri-infarct myocardium. In particular, the vascular density in the peri-infarct region, which is close to the site of gene administration, was increased to a level higher than in normal heart muscles (the vascular density in the peri-infarct myocardium: 644±96 /$mm^2$ in the Ang1-treated group, 350±79 /$mm^2$ in the physiological saline-treated group ($p<0.01$ vs the Ang1-treated group), 332±127 /$mm^2$ in the AxCAZ3-treated group ($p<0.01$ vs the Ang1-treated group), or 402±121 /$mm^2$ in the sham- operated group). Hemangioma was not found in the Ang1-treated group, either macroscopically or microscopically. Interestingly, the physiological saline-treated group and AxCAZ3-treated group showed a reduction in the number of blood vessels in the interventricular septum distant from the site of gene administration four weeks after myocardial

infarction (341±60 /mm$^2$ and 367±113 /mm$^2$, respectively). The decrease in the number of septal blood vessels was suppressed by the administration of Ang1 gene (461±100 /mm$^2$) or VEGF gene (483±46 /mm$^2$ in the sham-operated group). Fig. 5 shows immunostaining of vascular endothelia with the anti-CD34 MoAb. Micro vessels with 10 μm or less, as well as blood vessels with 10 μm or more, were found in the Ang1 gene-administered group (for every sample, many α-SMA-positive blood vessels were found in the left ventricular region of the infarcted hearts treated with Ang1; 38.9±7.35 /mm$^2$ in the septal region, 38.9±4.81 /mm$^2$ in the boundary region, and 112±26.1 /mm$^2$ in the infarcted region). In every group except for the Ang1-treated group, there was no significant alteration in the density of α-SMA-positive blood vessels greater than 10 μm (19 to 22 /mm$^2$). In addition, it was found that administration of Ang1 alone increased the vascular density to the same extent as the administration of the VEGF gene (Fig. 4).

[Example 5] Reduction of myocardial infarct size by Ang1 gene

The effect of the Ang1 gene on infarcts was confirmed using the myocardial infarction model. The myocardial infarct size was measured by the procedure described below. The infarct size was measured four weeks after the myocardial infarction by the methods of Edelberg *et al.* (Edelberg JM *et al.* Circulation 105, 608-613, 2001) and Roberts *et al.* (Roberts CS *et al,* Am. J. Cardiol. 51, 872-876, 1983). The rats were sacrificed and the infarcted hearts were excised four weeks after the model preparation. The hearts were immersed in cold physiological saline to remove blood from the ventricles, and then fixed in 4% formaldehyde for 48 hours. The hearts were embedded in paraffin and sliced into 10 μm sections. The sections were prepared by slicing in the short axis direction at an intermediate position between the cardiac apex and the ligation site of the left anterior descending branch. The infarcted site was stained with Hematoxylin-Eosin staining and Masson's trichrome staining. The section images were taken with a digital camera and then using NIH image, the following parameters were determined in a blind manner:

Total left ventricle (LV) area (mm$^2$), infarction area (mm$^2$), septal wall thickness (mm), infarction wall thickness (mm), epicardial and endocardial circumference of LV (mm), and epicardial and endocardial infarction length (mm). From these results, evaluation was performed using the following formulas:

% infarction size = infarcted region / total LV area x 100

% Ant/septal wall thickness = anterior wall (infarct) thickness / septal wall thickness x 100

Viable LV area = (total LV myocardial area) - (infarcted myocardial area);

%endocardial infarct length = endocardial infarct length / endocardial circumference of LV x 100;

%epicardial infarct length = epicardial infarct length / epicardial circumference of LV x 100;

As shown in Fig. 5, clear signs of cardiac failure are observed in the infarcted cardiac muscles, including thinning of the myocardial walls in the infarct and throughout the surviving left ventricular myocardium, and tendency of left ventricular lumen enlargement. As shown in Table 2, when compared with those of the control group, the infarct region was reduced significantly (% infarction size) and the mass of surviving myocardium increased significantly (%viable LV area) in the Ang1 gene-administered group. Thus, it was clearly shown that Ang1 has an effect on surviving myocardium, as well as an effect of reducing the size of myocardial infarct. The % infarct thickness parameter, which reflects the thickness of an infarcted wall, was also found to have significantly increased in the Ang1- administered group.

Table 2

| Anatomical alterations in the left ventricles of rats following myocardial infarction with and without Ang1 gene therapy | | | | | |
|---|---|---|---|---|---|
| | % Infarct size | % Ant/septal wall thickness | Surviving LV area ($mm^2$) | % endocardial infarct length | % epicardial infarct length |
| Sham operation (*n*=5) | - | 123±29.0 | 37.4±9.36 | - | - |
| Physiological saline (*n*=14) | 31.6±8.66 | 34.0±7.05 | 19.8±4.91 | 44.0±10.8 | 34.4±8.86 |
| AxCAZ3 (*n*=8) | 34.9±6.69 | 31.3±4.64 | 19.2±4.68 | 52.5±3.99 | 43.7±7.52 |
| AxCAhAng1 (*n*=20) | 21.1±5.38*‡ | 56.5±9.62*‡ | 27.0±5.20*‡ | 40.2±13.2‡ | 30.2±8.82‡ |

*, $p < 0.01$ vs Physiological saline;
‡, $p < 0.05$ vs AxCAZ3

(Table legend) Each value is represented as mean ± SD. LV indicates the left ventricle. Rats were sacrificed four weeks after myocardial infarction. All parameters were obtained from a cross section of an intermediate position between the cardiac apex and the ligation site of the coronary artery. The statistical analysis was carried out using the ANOVA of Bonferroni/Dunn test.

[Example 6] Improvement of cardiac functions by Ang1 gene following myocardial infarction

Ang1 was found to have angiogenesis activity and effect of reducing myocardial infarct size in infarcted hearts. It was then examined whether these effects indeed contributed to the improvement of cardiac functions. The cardiac functions were evaluated using the M-mode method by echocardiography and the area-length method in the B-mode long axis view.

Specifically, cardiac functions were measured using echocardiogram (LOGIQ500, GE Yokokawa Medical System, Tokyo, Japan) four weeks after LAD ligation. The measurements were carried out under anesthesia by an intramuscular injection of ketamine hydrochloride (50 mg/kg) and xylazine (2.5 mg/kg). The position for the M-mode measurement was determined using a 10 MHz probe based on the long axis view. The left ventricular end-diastolic diameter (Edd) and left ventricular end-systolic diameter (Esd) were measured by the M-mode method, and then the fractional shortening (FS) of left ventricular short-axis diameter was calculated.

$$FS (\%) = (Edd-Eds)/Edd \times 100$$

Left ventricular area at diastole (LVAd), left ventricular area at systole (LVAs), left ventricular long-axis length at diastole (LVLd), and left ventricular long-axis length at systole (LVLs) were determined from the long axis view of the left ventricle obtained by the B-mode method. Left ventricular ejection fraction (EF) was calculated according to the following formula (the area-length method) (Sjaastad, I. *et al.* (2000) J. Appl. Physiol. 89: 1445-1454):

$$EF (\%) = [(0.85x\ LVAd^2/LVLd) - (0.85x\ LVAs^2/LVLs)]/(0.85x\ LVAd^2/LVLd) \times 100$$

The long axis views obtained by echocardiography in the rat model of myocardial infarction are shown in Fig. 6. Signs of cardiac failure such as thinning of the anterior wall of the left ventricle (i.e., the infarcted site), increase of echo brightness, and enlargement of the left ventricular lumen were apparently found in the infarcted hearts, as similar as those observed in the histological images.

Various parameters determined by echocardiography are shown in Table 3. Marked increases of Edd, Esd, LVAd, and LVAs were found in both physiological saline- and adenovirus (AxCAZ3)-treated control groups after myocardial infarction. FS and EF were also decreased to 40 to 50% of the normal heart level. According to echocardiographic parameters, these groups were confirmed to be in a state of cardiac failure four weeks after myocardial infarction. Meanwhile, in the Ang1 gene-administered group, no significant improvement of Edd, Esd, and LVAd was recognized. However, FS and LVAs were increased compared with the control groups. EF also improved to 55%.

Table 3

| Cardiac function evaluation of the infarcted hearts after Ang1 gene therapy by echocardiography | | | | | | | |
|---|---|---|---|---|---|---|---|
| | M-mode evaluation | | | Two-dimensional evaluation | | | |
| | Edd (mm) | Esd (mm) | FS (%) | LVAd ($mm^2$) | LVAs ($mm^2$) | FAC (%) | EF (%) |
| Sham operation | 5.50±0.20 | 2.50±0.177 | 54.6±1.99 | 55.2±9.57 | 24.6±5.50 | 55.5±4.82 | 72.8±5.86 |
| Physiological saline | 7.43±1.25 | 5.86±0.0851 | 20.8±4.63 | 75.3±9.03 | 57.8±1.89 | 22.6±6.99 | 36.0±9.46 |
| AxCAZ3 | 7.54±0.544 | 5.93±0.693 | 21.6±4.50 | 73.7±4.04 | 54.3±6.66 | 26.4±5.54 | 40.5±7.62 |
| AxCAhAng1 | 7.13±0.985 | 4.69±1.31 | 34.7±11.1*‡ | 71.6±3.46 | 45.0±5.12*‡ | 34.8±5.47*‡ | 55.0±2.16*‡ |

*, $p < 0.05$ **vs**. physiological saline ;

‡ , **$p < 0.05$ vs. AxCAZ3**

(Table legend) Cardiac functions of the hearts treated with 1x $10^{10}$ opu of AxCAZ3 or AxCAhAng1 were measured four weeks after infarction by echocardiography using the M-mode and area-length methods. Each value is represented as mean ± SD. Edd indicates the left ventricular end-diastolic diameter; Esd, left ventricular end-systolic diameter; FS, fractional shortening of left ventricular short-axis diameter; LVAd, left ventricular area at diastole; LVAs, left ventricular area at systole; FAC, fractional area change of left ventricular lumen; EF, ejection fraction of left ventricle. The statistical analysis was carried out using the ANOVA of Bonferroni/Dunn test.

[Example 7] Necrosis-suppressing effect of administering the Ang1 gene alone in a mouse model of acute lower limb ischemia

The production of tissue VEGF is enhanced in acute lower limb ischemia as well as in myocardial ischemia. Thus, a mouse model of acute lower limb ischemia was prepared, and anti-ischemic therapy was conducted by administering the Ang1 adenoviral expression vector alone. The lower limb ischemia model was prepared using C3H/HeN mice (male, 20 to 25 g) according to the method of Couffinhal *et al.* (Couffinhal T *et al.* (1998) Am J Pathol 152 (6): 1667-79). The mice were anesthetized systemically with intramuscular injection of Ketalar (50 mg/kg) and xylazine (20 mg/kg). After the mice were shaven on both lower limbs, the left inguinal region was opened and the left femoral artery and all its branches were exposed. The origin of the femoral artery was ligated using a 7-0 nylon suture. Likewise, the artery was also ligated right before branching off to the popliteal artery and the saphenous artery. In addition, all other branches were ligated, and then the left femoral artery was excised. The operation was completed by closure suturing of the surgical wound.

The Ang1-expressing adenovirus, AxCAhAng1, was prepared by the procedure described above (opu/pfu ratio was 13.3). Immediately after lower limb ischemia was induced, AxCAhAng1 (1x $10^{10}$ opu/head) was administered intramuscularly to the left femoral adductor and the left gastrocnemial muscle. 25 µl (2.5x $10^9$ opu) of AxCAhAng1 was injected at two sites each (i.e., four sites were injected in total) using a 1.0 ml injector with a 29G needle. A control group consisted of five mice with induced ischemia. On day 3, day 9, or day 10 after the model preparation, ischemia in the mice was evaluated by macroscopic observation of necrotized regions, loss of fingers, loss of lower limbs, and ulcer formation.

In the control group, three days after the model preparation, necrotized regions (3/5, i.e., three in five cases) were confirmed; loss of fingers (2/5), loss of lower limbs (0/5), and ulcer formation (1/5) were detected. On day 9, necrotized regions (3/5) were confirmed; loss of fingers (3/5), loss of lower limbs (0/5), ulcer formation (1/5), and progression of ischemia were detected. Meanwhile, in the Ang1 group, necrotized regions (0/5) were confirmed; loss of fingers (0/5), loss of lower limbs (0/5), and ulcer formation (2/5) were detected three days after the model preparation. On day 9, necrotized regions (3/5) were confirmed; loss of fingers (1/5), loss of lower limbs (0/5), and ulcer formation (2/5), and progression of ischemia were suppressed. The results are shown in Fig. 7.

Administration of the Ang1-expressing adenovirus alone was found to markedly suppress the loss of fingers. The diseased limbs were examined on the third day after the ischemia, and the ischemia-mediated alterations were apparently reduced in the Ang1-administered limbs as compared with the control limbs. There are many steps to angiogenesis, such as sprouting and branching. In particular, arteriogenesis is involved in the formation of functional blood vessels that enhance tissue perfusion, and is observed mainly 10 days after ischemia, namely, in the late phase of angiogenesis. Accordingly, the early effects of Ang1 observed in this experiment cannot be attributed to angiogenesis, and may have resulted from a mechanism other than angiogenesis. It is known that Ang1 activates PI3 kinase via Tie-2, which in turn activates Akt having anti-apoptotic activity. Via Tie-2, Ang1 also enhances the production of NO, which has an effect of suppressing vascular endothelial apoptosis. It is possible these activities of Ang1 suppress the apoptosis of vascular endothelial cells and thus prevent the development of ischemia.

The enhanced production of VEGF caused by acute ischemia is presumed to aggravate tissue edema and further impair tissue perfusion. In fact, it is reported that the over-expression of VEGF enhances necrosis and loss of lower limbs in the present model. In this Example, it is presumed that the administration of Ang1 in the acute phase of ischemia suppressed the development of edema leading to impaired perfusion. In the Ang1-administered group, there was only a single case of severe necrosis with loss of fingers even on day 9.

As described above, in the acute lower limb ischemia model, the administration of Ang1 was confirmed to suppress the development of necrosis and to salvage limbs from post-necrotic limb loss.

[Example 8] Naked plasmid-mediated introduction of the Ang1 gene into skeletal and cardiac muscles

The direct injection of naked plasmids into tissues is the safest and simplest gene transfer method. Cytomegalovirus (CMV) promoter-based plasmids are mostly used in the previously approved clinical protocols of gene therapy for cardiovascular diseases. One of the major disadvantages of naked plasmid injection is that the expression level of the introduced gene is low. CA promoter (a chicken β-actin promoter comprising the cytomegalovirus enhancer) is one of the strongest transcriptional regulatory modules *in vitro* and *in vivo.* However, the level of gene expression driven by CA promoter varies depending on the cell type or organ type. In fact, when a CA promoter-based vector is used to inject a naked plasmid, it is unclear whether the introduced gene is expressed at an appropriate level in the cardiac tissue. Thus, CA promoter-based naked plasmids were prepared to examine the expression level of a gene introduced into the cardiac muscle by direct injection.

*Escherichia coli* β-galactosidase (LacZ) gene was excised from pIND/lacZ (Invitrogen). The LacZ gene was inserted into each of the following vectors: pcDNA3 (Invitrogen), pCAGGS comprising the CA promoter (Niwa, M. *et al.,* Gene 1991; 108: 193-199), and pCA1 prepared by deleting the replication origin of simian virus (SV40ori) from pCAGGS. The constructed plasmids were named pcDNA3LacZ, pCAZ2, and pCA1LacZ, respectively. The plasmid pCAZ2 used in the present invention was that the one disclosed by Yoshida *et al.* (Hum. Gene Ther. 9:2503-2515, 1998). The plasmid pCA1 was prepared by digesting pCAGGS with BamHI and HindIII to remove a SV40ori-comprising fragment (522 bp). The 5' end of the digested plasmid was then filled in with T4 DNA polymerase. The plasmid was ligated using T4 DNA ligase to prepare the expression vector pCA1. All plasmids were purified using the Endofree Maxi Kit (Qiagen GmbH, Hilden, Germany).

Twenty μg of naked plasmid in 0.1 ml of 0.9% physiological saline was injected into the skeletal muscles or hearts of Lewis rats (male, 8-week old, 250 to 300 g weight; Sankyo Labo Service (Tokyo, Japan)) using a 1 ml syringe with a 27G injection needle. AxCAZ3 adenoviral particles ($10^{10}$, 5x $10^9$, and $10^9$ OPU) in 0.1 ml of 0.9% physiological saline were also injected into the hearts. For the injection into skeletal muscles, the hind leg was incised by 2 cm long to facilitate injection into the femoral muscles (Wolff, J.A. *et al.,* Science 1990; 247: 1465-1468). For the injection into the heart, the left chest was opened and the naked plasmids or adenovirus particles were injected into the cardiac apex (Lin, H. *et al.,* Circulation 1990; 82: 2217-2221). After the injection, the incision wounds were sutured with silk sutures.

β-gal activities in the skeletal muscles and hearts were analyzed by a previously reported procedure (Shaper, N.L. *et al.,* J Boil Chem 1994; 269: 25165-25171). Specifically, tissues (0.8 g to 1.0 g) were homogenized in 4 ml of tissue lysis buffer (100 mM potassium phosphate, 0.2% Triton X-100, 2 mM leupeptin, 1 mM phenylmethylsulfonyl fluoride, and 0.5 mM dithiothreitol, pH 7.8) for 1 min. The homogenized tissues were then centrifuged at 12,000 xg for 10 min. The supernatants were collected and heated at 48°C for one hour to inactivate the endogenous β-galactosidase activity. β-gal activities in the supernatants were assayed using Galacto-Light™ Plus Kit (Tropix, Bedford, MA) according to the manufacturer's protocol. Chemiluminescent signals were detected using a MicroLumat LB96 luminometer (Wallac, Gaitherburg, MD). The data obtained in relative light units (RLU) were converted into ng activity of LacZ using a recombinant β-galactosidase standard (Roche Diagnostics, Manheim, Germany). In the histochemical detection of LacZ, first, 10 μm cryosections of cardiac tissues were stained with an X-gal solution at 37°C for 24 hours (Nabel, E. G. *et al.,* Science 1989; 244: 1342-1344). The sections were then counterstained with eosin. The data are represented as mean ± S.E. Statistical analysis was carried out by the Scheffe test. When p value is lower than 0.05, the data are assumed to be significant.

The naked plasmid dosage (20 μg) used in this Example is equivalent to that (g/kg) used in the clinical gene therapy trials for limb ischemia (Losordo, D.W. *et al.,* Circulation 1998; 98: 2800-2804) and heart diseases (Baumgartner, I. *et al.,* Circulation 1998; 97: 1114-1123). A total of 4 mg naked DNA was used in the former and 200 μg to 2,000 μg of naked DNA was used in the latter. The reporter gene expression from the CA promoter- and CMV promoter-based vectors was examined in the rat skeletal muscles and hearts in the present study. Both tissues are well known sites for injection of naked plasmids in clinical cardiovascular gene therapy. Five days after the plasmid injection into hind leg femoral muscles (n= 4) and hearts (n= 4), the levels of LacZ expression mediated by CMV promoter-based vectors (pcDNA3LacZ and pCMVβ) and CA promoter-based vectors (pCAZ2 and pCA1LacZ) in the skeletal muscle were 1.6±0.4 ng, 10.2±2.0 ng, 37.2±6.9 ng, and 27.2±6.8 ng, respectively (Fig. 8A). In the skeletal muscle, the CA promoter-based vectors expressed the reporter gene at a higher level than the CMV promoter-based vectors. Likewise, the expression levels of the introduced gene in the heart were higher when mediated by the CA promoter-based vectors (pCAZ2, 510.8±69.8 ng; pCA1LacZ, 509.9±66.7 ng) compared with the expression mediated by the CMV promoter-based vectors (pcDNA3LacZ, 46.2±13.2 ng; pCMVβ, 108.8±37.8 ng). For all plasmids, the expression level of the introduced gene was found to be approximately an order of magnitude higher in the heart than in the skeletal muscle (Fig. 8B). The expression levels of the introduced gene were also examined with the pCA1LacZ vector, from which the SV40ori sequence had been removed to improve safety. As shown in Fig. 8, there was no significant difference in the expression level of LacZ between pCA1LacZ and pCAZ2 in each of the skeletal muscle and heart.

LacZ expression levels in the heart were compared between the CA promoter-based plasmid vector and the adenoviral vector. The adenoviral vector AxCAZ3 was injected into the cardiac apex at various doses ($10^{10}$, 5x $10^9$, and $10^9$ OPU) (n= 4). After five days, the LacZ expression level in the heart, into which AxCAZ3 had been injected, was compared with that in the cardiac tissues into which 20 μg of pCAZ2 had been injected. The result showed that the average expression level of the introduced gene in the heart, mediated by 20 μg of pCAZ2, was found to be comparable to that mediated by 6.0x $10^9$ OPU of AxCAZ3 (Fig. 9).

pcDNA3LacZ (20μg), pCAZ2 (20μg), or 5x $10^9$ OPU of AxCAZ3 was injected, followed by X-gal staining. LacZ-positive muscle cells were found in all the samples from the tested groups. There were almost no LacZ-positive cells in areas surrounding the injection site of the heart samples into which pcDNA3LacZ had been injected. In contrast, when pCAZ2 was used, LacZ-positive myocardial cells which have high expression levels of the gene were found sporadically in the areas surrounding the injection site. The expression level and pattern of the introduced gene in cardiac tissues, into which 5x $10^9$ OPU of AxCAZ3 had been injected, were similar to those in the tissues where pCAZ2 had been injected. As demonstrated above, the direct administration of the plasmids results in exceedingly efficient expression of the introduced genes in the cardiac muscle, and achieves a high-level expression almost equivalent to that with the adenoviral vector, especially when CA promoter is used.

[Example 9] Effect on gene introduction into myocardial cells by a minus-strand RNA viral vector expressing human Ang1 gene

A transmissible Sendai viral vector (SeVAng1), into which human Ang1 cDNA had been inserted, was produced by the conventional method (Kato, A. *et al.,* 1996, Genes Cells 1: 569-579; Yu, D. *et al.,* 1997, Genes Cells 2: 457-466). SeV containing no foreign gene (SeVNull) and an E. *coli* β-galactosidase gene (LacZ) SeV expression vector (SeVLacZ) were used as control viral vectors. A LacZ-encoding adenoviral vector AxCAZ3 was also used for comparison. The SeV vectors were injected into the allantoic cavities of 10-day-old embryonated chicken eggs, amplified, and then collected. The viral titers were determined by hemagglutinin assay using chicken erythrocytes. The viruses were stored at -80°C until use. The adenoviral vector encoding LacZ (AxCAZ3) was amplified in 293 cells derived from human fetal kidney and purified by ultracentrifugation in a CsCl discontinuous density gradient. The viruses were dialyzed against PBS containing 10% glycerol, and then stored at -70°C until use. Prior to use, the viral stocks were tested for their adenovirus density and titer, as well as any contamination of replication-competent adenoviruses. The adenovirus titer was determined by LD50 (plaque forming units: pfu) and A260 (optical particle units: opu) measurement using 293 cells.

LacZ gene expressions in the myocardial cells of neonatal rats mediated by the adenoviral and Sendai viral vectors were examined by the procedure described below. Neonatal rat myocardial cells were isolated by the procedure described below. Hearts were excised from neonatal Lewis rats under deep anesthesia and soaked in oxygen-saturated Tyrode's solution (143 mM NaCl, 5.4 mM KCl, 1.8 mM $CaCl_2$, 0.5 mM $MgCl_2$, 0.33 mM $NaH_2PO_4$, 5.5 mM glucose, and 5.0 mM HEPES), and the ventricular muscles were separated. The obtained ventricular muscles were incubated in a $Ca^{2+}$-free Tyrode's solution containing collagenase (5 mg/ml; Wako Pure Chemical Industries) at 37°C for one hour to isolate myocardial cells. The isolated neonatal rat myocardial cells were suspended by pipetting in KB solution (50 mM L-glutamic acid, 40 mM KCl 40, 20 mM taurine, 20 mM $KH_2PO_4$, 3 mM $MgCl_2$, 10 mM glucose, 0.5 mM EGTA, and 10 mM HEPES) for 5 min to prepare cell suspensions. The cells were plated into wells of 24-well plates at a cell density of 5x $10^4$/well in Dulbecco's modified Eagle Medium (DMEM) containing 10% bovine fetal serum (FBS).

Genes were introduced into neonatal rat myocardial cells by the procedure described below. The myocardial cells were plated into wells of 24-well plates at a cell density of 5x $10^4$/well as described above, and infected with the viral vectors the next day. The neonatal rat myocardial cells were infected with SeVLacZ or AxCAZ3 at 37°C for one hour, in viral solutions with different multiplicities of infection (moi: CIU/cell for the SeV vector; and pfu/cell for the Ad vector), which had been diluted with DMEM containing 2% fetal bovine serum (FBS). The cells were washed with phosphate buffered saline (PBS), and then cultured in DMEM supplemented with 10% FBS for 24 hours. The cells were examined for LacZ expression. The LacZ activity was examined quantitatively as the enzymatic activity of β-galactosidase using the β-gal Reporter Gene Assay Kit (Roche) and a β-galactosidase standard sample (Roche).

The result is shown in Fig. 10. Both SeVLacZ and AxCAZ3 used at a high moi (> 30 moi) were found to achieve high-level expressions of the introduced gene in rat myocardial cells. The reporter gene was highly expressed even when a SeV moi as low as 10 or lower was used for infection. The reporter gene expression level was increased in a virus density-dependent manner and nearly reached a plateau at a moi of 30 or higher. Meanwhile, the Ad vector-mediated gene introduction into neonatal rat myocardial cells is dependent on the virus density. Even when the virus was infected at a moi of 100, the expression level did not reach the maximal level. The LacZ expression level in the neonatal rat myocardial cells infected with the Ad vector at a moi of 10 was almost the same as that with 1 moi of the SeV vector. In particular, when infected at a low moi (10 moi or lower), the SeV vector had a higher gene introduction efficiency than the AdV vector. Next, effects of the virus exposure time on the gene introduction into neonatal rat myocardial cells were examined. When the adenoviral vector was used, long periods of time (120 minutes or longer) were required for the maximal expression of the gene introduced into myocardial cells *in vitro.* In contrast, when the SeV vector was used, only a short time (30 minutes or shorter) of exposing the cells to the virus solution was sufficient for obtaining the maximal expression of the introduced gene under the same conditions.

[Example 10] Gene expression mediated by intramyocardial administration of the SeV vector

Efficient SeV vector-mediated gene expression in neonatal rat myocardial cells was confirmed. However, there is no report or information on the expression of genes in the heart into which a Sev vector had been administered intramyocardially *in vivo.* Various concentrations of the LacZ-expressing Ad or SeV vectors were administered intramyocardially to normal rat hearts. The rats were sacrificed three days later to investigate the reporter gene expression in the hearts. The gene expressions in the rat hearts from the vectors were evaluated *in vivo* as described below. Lewis rats were anesthetized by inhalation of diethyl ether and intraperitoneal injection of ketamine hydrochloride (50 mg/kg) and xylazine (2.5 mg/kg), and then intubated. The left chest was opened by thoracotomy, and SeVLacZ or AxCAZ3 was administered intramyocardially into the cardiac apex at various titers using a syringe with a 30G needle. The rats were sacrificed 72 hours after the gene introduction, and then the LacZ expression levels were evaluated.

The results are shown in Fig. 11. As seen in this Figure, the reporter gene expression levels in the heart increased depending on the amount of viral vector. The reporter gene expression levels were similar between 3.3x $10^9$ opu of Ad vector and 1x $10^8$ CIU of SeV vector.

[Example 11] Organ distribution of the gene expression mediated by intravenously or intramyocardially administered SeV vector

If the blood stream is contaminated with the viral particles due to overflow and such after intramyocardial administration, the resulting expression in organs other than the heart may produce adverse effects, and this is a very serious problem from the clinical viewpoint. However, there has been no report on the organ distribution of gene expression after intravenous administration of SeV vectors. Thus, the organ distributions of gene expression after intravenous and intramyocardial administrations were investigated.

SeVLacZ (1x $10^8$ CIU) was administered to normal rats via penile veins, and the rats were sacrificed 72 hours later. The heart, right lung, liver, right kidney, and spleen were excised and assayed for LacZ expression. Likewise, 1x $10^8$ CIU of SeVLacZ or 1x $10^9$ to 1x $10^{10}$ opu of AxCAZ3 was administered intramyocardially to normal rats, and the rats were sacrificed 72 hours later. The heart, right lung, liver, right kidney, and spleen were excised and assayed for LacZ expression.

Prior to assaying for LacZ expression, the excised organs were homogenized in lysis buffer (100 mM potassium phosphate (pH 7.8), 0.2% Triton X-100, 1 mM dithiothreitol, 0.2 mM phenylmethylsulfonyl fluoride, and 5 µg leupeptin). The homogenates were centrifuged at 12,500 xg for 10 minutes. The resulting supernatants were incubated at 48°C for one hour to inactivate the endogenous β-galactosidase activity in them (Young DC, Anal. Biochemi. 215, 24-30, 1993). The supernatants were treated using a β-gal Reporter Gene Assay Kit at room temperature for one hour. The enzymatic activity in the supernatants was determined by measuring the chemiluminescence using Mini-Lumat LB9506 (Berthold) (Shaper NL *et al.* J. Biol. Chemi. 269(40), 25165-25171, 1994). The results obtained (in relative light units) were converted to β-galactosidase activity (pg/ml) based on the standard curve prepared using a β-galactosidase standard sample.

The results are shown in Fig. 12. As commonly known, the Ad vector-introduced foreign gene was mainly expressed in the liver after intravenous administration. The SeV vector, on the other hand, was different from the AdV; when the SeV vector was used, the reporter gene was expressed in the lung, heart, and spleen, but hardly expressed in the liver. The results shown in the Figure were obtained by using extracts from the whole organs ("lung" indicates the right lung and "kidney" indicates the right kidney). The expression level increases in the order of heart < spleen < lung with considerations given to the organ weight. The organ distribution pattern of gene expression after the intramyocardial administration was found to be nearly the same as that after the intravenous administration. Therefore, organs targeted for gene expression in other organs as a result of the vector overflow were revealed to be the lung and spleen.

[Example 12] Myocardial infarction therapy by Ang1 gene introduction using SeV

Myocardial infarction gene therapy using human angiopoietin-1 (Ang1) in a SeV vector was performed as described below. In the adenoviral vector-mediated Ang1 gene therapy described above, the adenoviral vector was used at 1x $10^{10}$ opu (equivalent to 7.5x $10^8$ pfu). When the LacZ gene was introduced into the myocardial cells (*in vitro*) and heart (*in vivo*) using the SeV vector, the gene introduction efficiency was relatively higher in comparison with the Ad vector. Thus, an intramyocardial administration of 5x $10^7$ CIU SeVAng1 was attempted for the treatment of rat myocardial infarction. Lewis rats were treated by ligating the anterior descending branch of the left coronal artery. Immediately after the ligation, 5x $10^7$ CIU of SeVAng1 was injected evenly at two sites in the anterior wall of the left ventricle surrounding the LAD perfusion area. Four weeks later, the number of capillaries in the myocardial infarct was examined histologically.

The rat myocardial infarction model was prepared according to the method of Pfeffer *et al.* (Pfeffer, M. A., *et*

*al.* (1979). Myocardial infarct size and ventricular function in rats. Circ Res. 44: 503-512). Lewis rats (eight-week old, male, and about 300 g weight) were anesthetized by inhalation of diethyl ether and intraperitoneal administration of 70 mg/kg ketamine and 6 to 7 mg/kg xylazine, and then intubated. The rats were then anesthetized by inhalation of 0.5% to 2.0% halothane under the conditions of 200 to 250 ml minute ventilation, 3 ml tidal volume, 60 to 80 cycles/min respiratory rate, and 1 l/min $O_2$. The left chest was then opened by thoracotomy. The left anterior descending branch (LAD) was identified and then ligated at the height of the left atrial appendage using a 6-0 nonabsorbable suture (nylon suture) (LAD ligation). After the intercostal incision was closed carefully so as not to damage the lungs, the muscle layer and the skin was closed with a continuous suture.

The SeV vector was introduced intramyocardially as described below. After the left anterior descending branch was ligated, 5x $10^7$ CIU of SeVAng1 was administered intramyocardially using a 30G needle at two sites: right and left peripheries of the area estimated to be the perfusion area for the left anterior descending branch. In the null group, 5x $10^7$ CIU of SeVNull was injected instead of SeVAng1, and in the negative control group, 0.9% physiological saline was injected.

The infarct size was measured four weeks after myocardial infarction by the methods of Edelberg *et al.* (Edelberg JM *et al.* Circulation 105, 608-613, 2001) and Roberts *et al.* (Roberts CS *et al,* Am. J. Cardiol. 51, 872-876, 1983). The rats were sacrificed and the infarcted hearts were excised four weeks after the model preparation. The hearts were fixed with formaldehyde and embedded in paraffin. The sections were prepared by slicing the tissues in the short axis direction at an intermediate position between the cardiac apex and the ligation site of the left anterior descending branch. The infarcted sites were stained with Hematoxylin-Eosin staining and Masson's trichrome staining. The section images were taken with a digital camera, and then the following parameters were determined in a blind manner using NIH images:

Total left ventricle (LV) area ($mm^2$), infarction area ($mm^2$), septal wall thickness (mm), infarction wall thickness (mm), epicardial and endocardial circumference of LV (mm), and epicardial and endocardial infarction length (mm). The size was then evaluated based on the results using the following formula.

%infarction size = infarcted region / total LV area x 100

%infarction thickness = anterior wall (infarction) thickness / septal wall thickness x 100

Viable LV area = (total LV myocardial area) - (infarction myocardial area);

Furthermore, vascular density in the cardiac muscle was evaluated by immunostaining of vascular endothelial cells with an anti-CD34 monoclonal antibody four weeks after the creation of myocardial infarction. The sections were stained with the Anti-CD34 MoAb (NU-4A1, Nichirei, Tokyo Japan) as the primary antibody, and then with a biotinylated anti-mouse IgG secondary antibody and avidin-horseradish peroxidase (DAB paraffin IHC staining module, Ventana Medical System Inc, Tucson, AZ). The number of blood vessels in the sections of the interventricular septum, peripheral region of the infarcted site, and the surviving myocardium in the myocardial infarct were determined in a blind manner under a microscope with 200 times magnification. The results are represented as the number of blood vessels/$mm^2$.

The infarct size and thickness after the SeVAng1 treatment of myocardial infarction are shown in Fig. 13. Administration of the control vector SeVNull to cardiac muscle did not lead to apparent improvements on the myocardial infarct size and thickness. In contrast, the infarct size was reduced and the infarct thickness was significantly increased in the SeVAng1-treated group, similarly to the treatment of myocardial infarction using an Ang1-expressing adenovirus. Evaluation of the blood vessel number also indicated that the number of capillaries in the infarct and the peri-infarct myocardium significantly increased in the SeVAng1-treated group (Fig. 14). Thus, even a relatively low viral titer of SeVAng1 was found to produce a therapeutic effect equivalent to that of the Ad vector in the rat myocardial infarction model.

[Example 13] Therapeutic effect of SeVAng1 in the rat model of lower limb ischemia

Lewis rats (eight-week old, male, and about 300 g weight) were anesthetized by inhalation of diethyl ether and intramuscular injection of 40 mg/kg ketamine and 4 mg/kg xylazine via an upper limb. After shaving both lower limbs, the abdominal region and the left inguinal region were incised, and the right iliac artery, right femoral artery, and their branches were all exposed. After the right iliac artery and its branches were ligated, the left femoral artery was also ligated at its origin, immediately before the bifurcation into the popliteal and saphenous arteries. Furthermore, all other branches of the left femoral artery were identified and ligated, and then the left femoral artery was removed surgically. In the operation, 5x $10^7$ CIU of the SeV vector was administered at two sites on the rectus femoris muscle using a 30G needle. After confirming that there were no hemorrhages, the surgical wound was sutured to complete the operation. In the null group, 5x $10^7$ CIU of SeVNull was injected instead of SeVAng1, and in the negative control group, 0.9% physiological saline was injected.

The blood flow analysis was carried out using Laser-Doppler imaging as described below. The blood flow in the lower limb was measured continuously over two weeks after ischemia (on day 1, day 3, day 7, and day 148 after ischemia) using a Laser Doppler system (Moor LDI, Moor Instruments, Devon, United Kingdom). The rats were anesthetized by inhalation of ether, and then further anesthetized and sedated with ketamine (25 mg/kg) and xylazine (2 mg/kg). The rats were kept at 37°C for 10 minutes and then analyzed for blood flow. The continuous blood flow measurements were carried out at the identical spots in the same rats. The resulting blood flow images were analyzed to estimate the mean blood flow in the feet and gastrocnemius regions of both lower limbs. To reduce the influence of measurement conditions, the blood flow ratio of ischemic side (left lower limb) to normal side (right lower limb) (tissue blood flow ratio: blood flow on the ischemic side/blood flow on the normal side) was then calculated.

Three days after the rat lower limb ischemia model was prepared, severe obstruction of blood flow in the diseased limb was observed: 45% in the physiological saline-treated group compared with the healthy limb, and 48% in the SeV vector control group (SeVNull) compared with the healthy limb. Meanwhile, in the SeVAng1-treated group, the day 3 blood flow in the diseased limb was significantly high: 63%. In both the physiological saline-treated and SeVNull-treated groups, spontaneous recovery of the blood flow in the diseased limb was recognized 7 and 14 days after the ischemia model preparation. This blood flow recovery was enhanced by the administration of SeVAng1 and after 14 days, the flow was improved to 87% of that in the healthy limb (Fig. 15).

[Example 14] Treatment of limb ischemia using the Ang1 gene-introduced mesenchymal cells

Mesenchymal stem cell (MSC) has been reported to differentiate into not only mesenchymal tissues such as bone and adipose tissue, but also myocardial tissues, muscle tissues, and so on. Furthermore, it has also been reported that MSC may secrete various angiogenesis factors and induce angiogenesis. In this Example, the blood flow-improving effect produced by MSC transplantation was compared with that of the gene therapy. In addition, genetically modified MSCs were prepared for anti-ischemia therapy.

Rat cardiac mesenchymal stem cells (MSC) were separated from Lewis rat thighbones according to the previous report (Tsuda, H., T. Wada, *et al.* (2003) Mol Ther 7(3): 354-65). Both ends of the thighbones were cut off, and bone marrows were collected by flushing the bones with 10% FBS-containing Dulbecco's modified Eagle's medium (DMEM) with an injector. The resulting bone marrow suspension was passed through 18, 20, and 22G needles successively to prepare a bone marrow cell suspension. The obtained bone marrow cells were plated at a cell density of 5x $10^7$ nucleated cells/10 cm culture dish and cultured for 4 days in culture medium (DMEM containing 10%FBS, 100 μg/ml streptomycin, 0.25 μg/ml amphotericin, and 2 mM L-glutamine). The culture medium was changed every 3 to 4 days to remove floating cells. The adherent cells were passaged and used as rat MSCs.

The rat model of lower limb ischemia was prepared as described in Example 13, and immediately after the preparation, 5x $10^6$ rat MSCs were administered to the rectus femoris muscle. A Sendai viral vector expressing the angiopoietin-1 (Ang1) gene was used in the group treated by gene therapy. The tissue blood flow (diseased limb/ healthy limb) was measured chronologically using Laser Doppler imaging. Three days after the creation of ischemia, severe ischemia was observed in the control (medium) group, which had a 48.2% blood flow ratio. The blood flow was restored to 60 % after seven days, and then reached a plateau. There was no difference in the blood flows between the MSC-administered group and the control group on days 3 and 7. On day 14, the MSC-administered group had a significantly improved blood flow of 89%. Meanwhile, in the group treated by gene therapy, the blood flow was found to be improved in an early stage and was 63% on day 3. The blood flow reached 87% on day 14.

Genetically modified MSCs that combine both the benefits of gene therapy and cell therapy were prepared. MSC is known to be relatively resistant to physical and chemical gene introduction methods, and to viral vectors such as retroviruses and adenoviruses. Accordingly, gene introduction into the rat mesenchymal stem cells was carried out using a Sendai viral vector, which demonstrates high efficiency gene introduction in various primary cell cultures, and then the efficiency was compared with that achieved by an adenoviral vector.

Gene introduction into rat MSCs was carried out as described below. The rat MSCs were plated in 24-well plates at a cell density of 2.5x $10^4$ cells/well, and then the cells were infected with a viral vector (SeVLacZ or AxCAZ3) the next day. The neonatal rat myocardial cells were infected with SeVLacZ or AxCAZ3 at 37°C for one hour, in viral solutions with different multiplicities of infection (moi: CIU/cell for the SeV vector; and pfu/cell for the Ad vector), which had been diluted with DMEM containing 2% fetal bovine serum (FBS). The cells were then washed with phosphate buffered saline (PBS), and cultured in 10% FBS-containing DMEM for 24 hours. The cells were analyzed for LacZ expression. The LacZ activity was assayed using the β-gal Reporter Gene Assay Kit (Roche).

The results are shown in Fig. 16. The reporter gene was expressed at high levels even when the infection was performed with SeV at a low moi (3 moi or lower). The expression levels increased depending on the virus concentration, and nearly reached a plateau when moi was 30 or higher. In contrast, while the gene introduction into rat MSCs using the Ad vector was dependent on the virus concentration, the expression levels were low at any viral concentrations tested for comparison, and at a moi of 30 or lower the expression levels were one hundredth or less of that achieved with the SeV vector. MSCs, into which LacZ gene was introduced using the SeV vector or the Ad vector at a moi of 100, were stained with X-gal. As a result, the number of positive cells was relatively small when the Ad vector was used, but almost all cells were LacZ-positive with the use of the SeV vector (Fig. 17).

The rat model of severe limb ischemia was treated by anti-ischemia therapy using MSCs into which the Ang1 gene had been introduced. Mesenchymal stem cells (MSCs) were isolated from Lewis rats (eight-week old, male) and cultured according to the previous report (Tsuda, H., T. Wada, *et al.* (2003) Mol Ther 7(3): 354-65). The obtained MSCs were infected with SeVAng1 at a moi of 2 at 37°C for one hour to prepare genetically modified MSCs. 24 hours after gene introduction, the genetically modified MSCs (5x $10^6$ cells) were administered to the rat model of lower limb ischemia (the model was prepared as described in Example 13). The genetically modified MSCs were injected immediately after ischemia. The blood flow in the limbs was examined by analyzing laser Doppler images. The data were represented as %blood flow (blood flow on the ischemic side/blood flow on the normal side x 100). In comparison with the control, the transplantation of the Ang1 gene-introduced MSCs resulted in a significant improvement of the blood flow in the ischemic limbs three days after the treatment (Fig. 18). After seven days, the blood flow was improved even more favorably than when SeVAng1 was directly administered (Fig. 18).

[Example 15] Gene introduction using a minus-strand RNA viral vector

The efficiencies of a minus-strand RNA viral vector-mediated gene introduction into the mammalian cells described below were compared with that achieved by an adenoviral vector.

(1) Cultured cell line

The SeV vector (SeV-LacZ) or adenoviral vector (AxCAZ3) that expresses the LacZ gene was used to infected HeLa cells at various virus densities for one hour. Twenty four hours after the vector infection, LacZ activity was determined using β-galactosidase Reporter Assay Kit or by X-gal staining. When the SeV vector was used at a low MOI of 10 or lower (in particular, a MOI of 0.3 to 3), the SeV vector was found to express the introduced gene at considerably higher levels than that with the adenoviral vector (Fig. 19A). The cells into which the gene had been introduced were examined by staining with X-gal. It was found that when the SeV vector was used, the proportion of the cells into which the gene was introduced was considerably higher than that with the adenoviral vector, and the expression levels of the introduced gene in individual cells were significantly higher than those with the adenoviral vector (Fig. 19B).

(2) Human oral squamous cell carcinoma

To examine the gene introducing effect of the vector, a SeV vector was used in the gene introduction into the human oral squamous carcinoma cell lines HSC3 (JCRB Cell Bank: JCRB0623, Rikimaru, K. *et al.,* In Vitro Cell Dev. Biol., 26: 849-856, 1990) and OSC19 (JCRB Cell Bank: JCRB0198, Yokoi, T. *et al.,* Tumor Res., 23: 43-57, 1988; Yokoi, T. *et al.,* Tumor res., 24: 57-77, 1989; Kawahara, E. *et al.,* Jpn. J. Cancer Res., 84: 409-418, 1993; Kawahara, E. *et al.,* Jpn J. Cancer Res, 84: 409-418, 1993; Kawashiri, S. *et al.,* Eur. J. Cancer B Oral Oncol., 31B: 216-221, 1995), which are resistant to gene introduction by the adenoviral vector (AxCAZ3). After infection with SeV-LacZ or AxCAZ3 at various MOIs for one hour, LacZ activity was determined using a β-galactosidase Reporter Assay Kit. The efficiencies of SeV-LacZ-mediated gene introduction into OSC19 and HSC3 were greater than those with AxCAZ3, at all MOIs tested (Fig. 20). Even in HSC3 which shows resistance to wild-type adenovirus and adenovirus targeting integrin (adenovirus with RGD-modified fiber, Dehari H, Ito Y *et al.* Cancer Gene Therapy 10: 75-85, 2003), and into which gene introduction was extremely difficult, high efficiency gene introduction with the Sev vector was confirmed. Thus, the SeV vector is very suitable for the introduction of genes into oral squamous carcinoma cells.

(3) Human macrophages and dendritic cells

The efficiencies of gene introduction into human macrophages and dendritic cells were compared between the SeV and adenoviral vectors. The SeV and adenoviral vectors expressing LacZ were each infected at a MOI of 1 for one hour. Twenty four hours after the vector infection, LacZ activity was assayed using a β-galactosidase Reporter Assay Kit. When the SeV vector was used, the expression levels were 1000 times or higher than those with the adenoviral vector (Fig. 21). Thus, the SeV vector is very suitable for the introduction of genes into macrophages and dendritic cells.

Industrial Applicability

The present invention provides novel agents and methods of gene therapy for ischemic diseases. The methods of the present invention are excellent as safe and effective therapeutic methods for ischemia with less adverse effects. Currently, surgical revascularization methods such as percutaneous transluminal coronary angioplasty (PTCA) and coronary artery bypass graft (CABG) are mainly used to treat acute myocardial infarction. In the methods of the present invention, revascularization can be enhanced using genetic engineering techniques. Therefore, active improvement in cardiac function and shortening of the period confined to bed are expected. In addition, the methods of the present invention produce excellent therapeutic effects in the treatment of limb ischemia or such.

## Claims

1. A method for treating ischemic heart diseases, which comprises the step of administering angiopoietin-1 or a vector encoding angiopoietin-1.

2. The method for treating ischemic heart diseases according to claim 1, which comprises the step of administering angiopoietin-1 or a vector encoding angiopoietin-1, and in which a vascular endothelial growth factor is not administered.

3. The method according to claim 1 or 2, wherein angiopoietin-1 or the vector encoding angiopoietin-1 is a viral vector encoding angiopoietin-1.

4. The method according to claim 3, wherein the viral vector is an adenoviral vector.

5. The method according to claim 3, wherein the viral vector is a minus-strand RNA viral vector.

6. The method according to claim 1 or 2, wherein angiopoietin-1 or the vector encoding angiopoietin-1 is a naked DNA.

7. The method according to any one of claims 1 to 6, wherein angiopoietin-1 or the vector encoding angiopoietin-1 is a vector that drives angiopoietin-1 expression using a CA promoter or a promoter having a transcriptional activity equivalent to or higher than that of said CA promoter.

8. The method according to any one of claims 1 to 7, wherein the administration of angiopoietin-1 or the vector encoding angiopoietin-1 is an injection into cardiac muscle.

**9.** A method for treating ischemic diseases, which comprises the step of administering a viral vector encoding angiopoietin-1.

**10.** The method for treating ischemic diseases according to claim 9, which comprises the step of administering a viral vector encoding angiopoietin-1, and wherein a vascular endothelial growth factor is not administered.

**11.** The method according to claim 9 or 10, wherein the viral vector is an adenoviral vector.

**12.** The method according to claim 9 or 10, wherein the viral vector is a minus-strand RNA viral vector.

**13.** The method according to any one of claims 9 to 12, wherein the vector administration is an injection into an ischemic site.

**14.** A genetically modified mesenchymal cell comprising a foreign gene encoding angiopoietin-1.

**15.** The mesenchymal cell according to claim 14, into which an adenoviral vector encoding angiopoietin-1 has been introduced.

**16.** The mesenchymal cell according to claim 14, into which a minus-strand RNA viral vector encoding angiopoietin-1 has been introduced.

**17.** A therapeutic composition for ischemia, which comprises the mesenchymal cell according to any one of claims 14 to 16 and a pharmaceutically acceptable carrier.

**18.** A method for producing a genetically modified mesenchymal cell, wherein the method comprises the step of contacting the mesenchymal cell with a minus-strand RNA viral vector carrying a gene.

**19.** The method according to claim 18, wherein the gene encodes angiopoietin-1.

FIG. 1

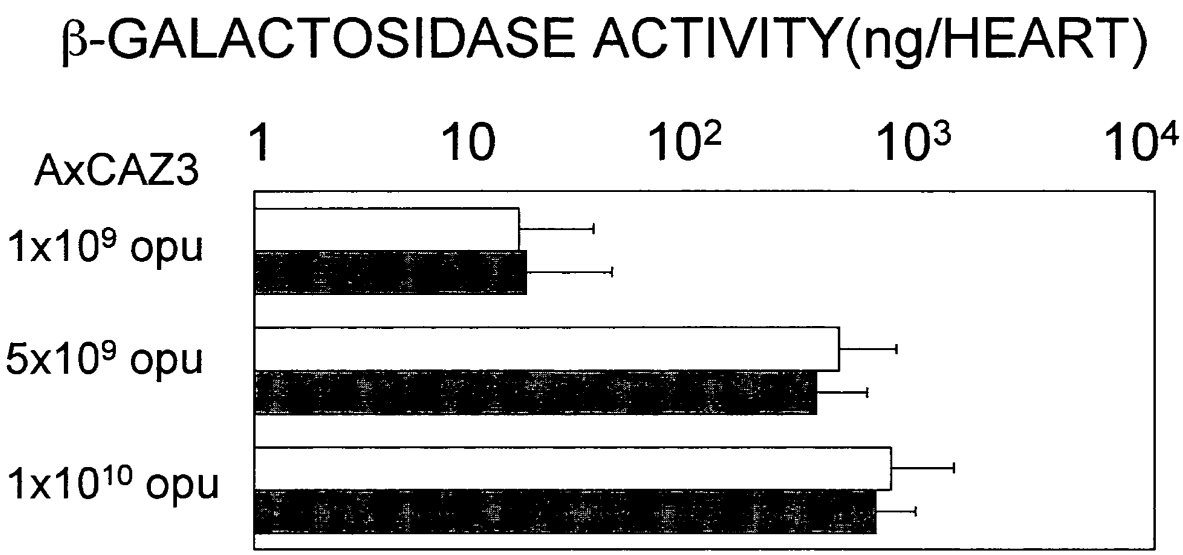
β-GALACTOSIDASE ACTIVITY(ng/HEART)
AxCAZ3
1
10
10²
10³
10⁴
1x10⁹ opu
5x10⁹ opu
1x10¹⁰ opu

FIG. 2

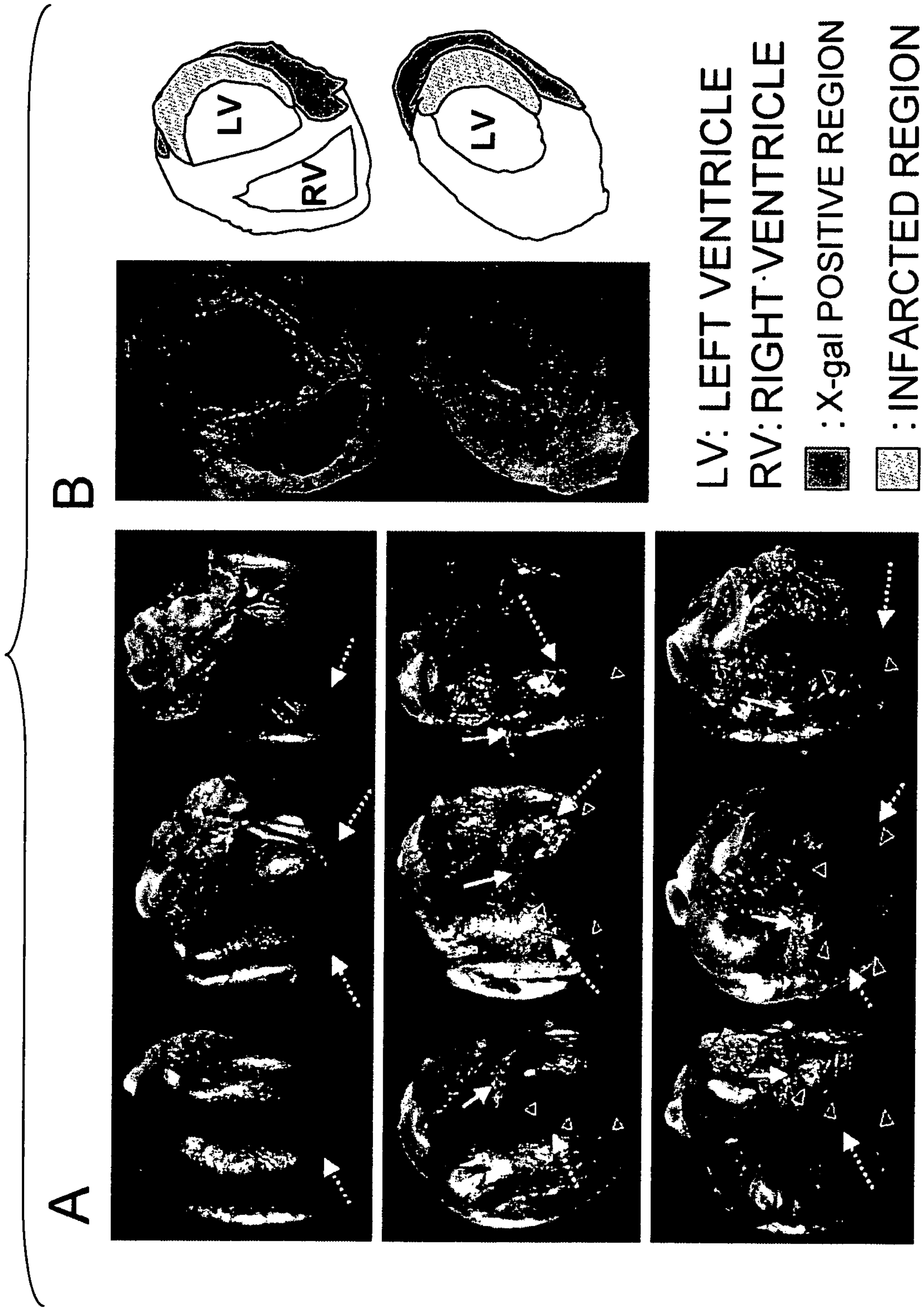
A
B
LV
RV
LV
LV: LEFT VENTRICLE
RV: RIGHT VENTRICLE
: X-gal POSITIVE REGION
: INFARCTED REGION

FIG. 3

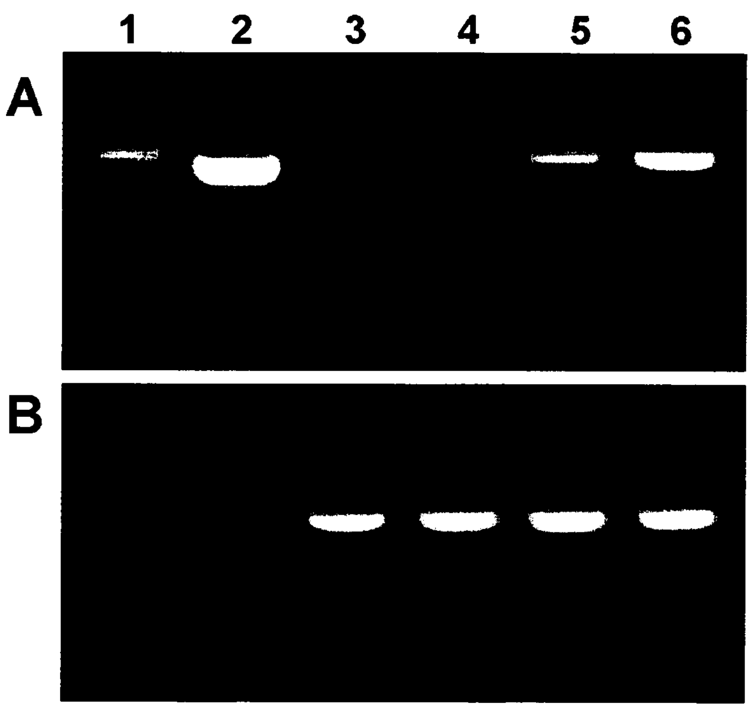

# FIG. 4

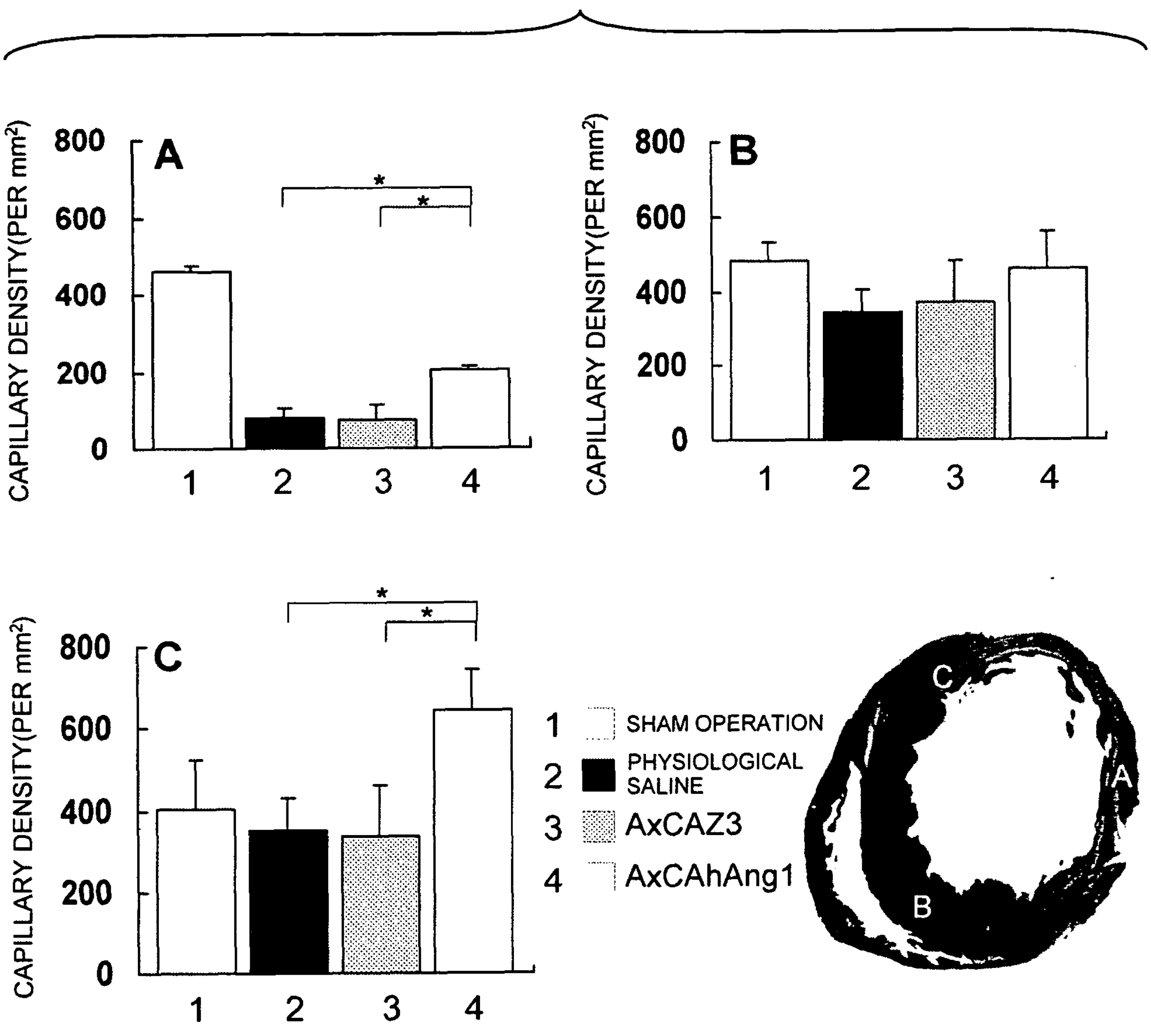
CAPILLARY DENSITY(PER mm²)
800
600
400
200
0
A
*
*
1
2
3
4
B
C
1 SHAM OPERATION
2 PHYSIOLOGICAL SALINE
3 AxCAZ3
4 AxCAhAng1

FIG. 5

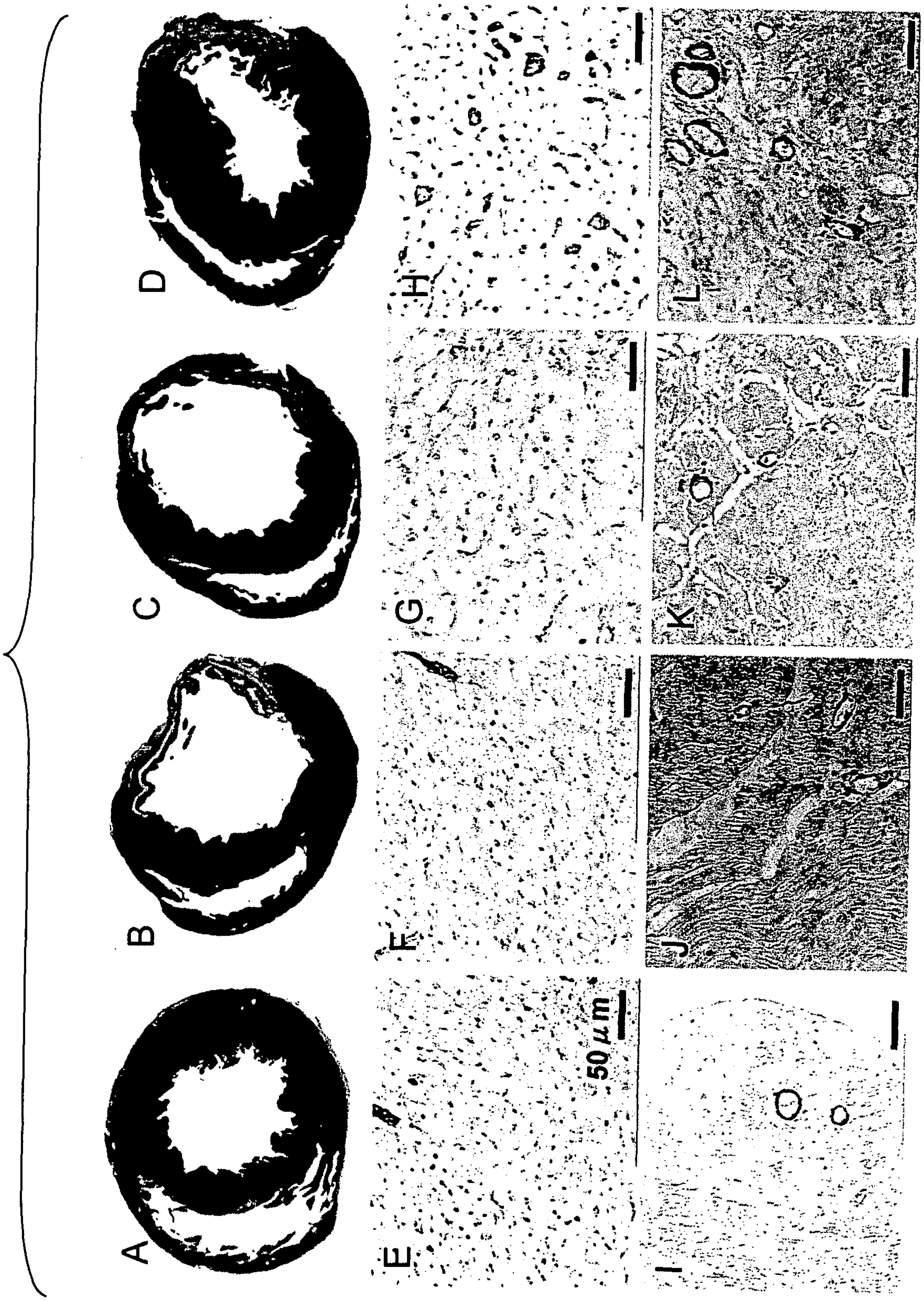

FIG. 6

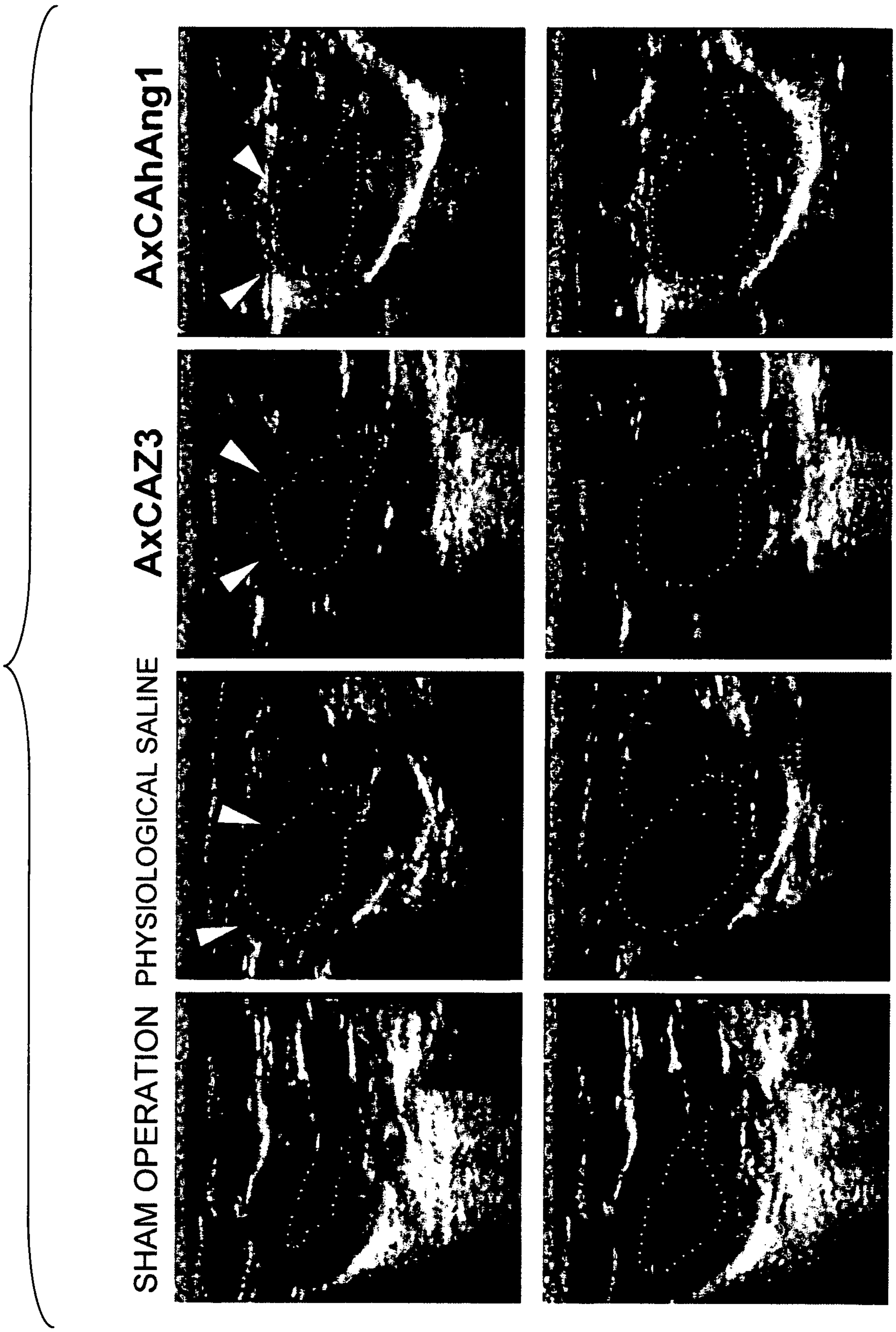

FIG. 7

CONTROL

DAY 3 DAY 9

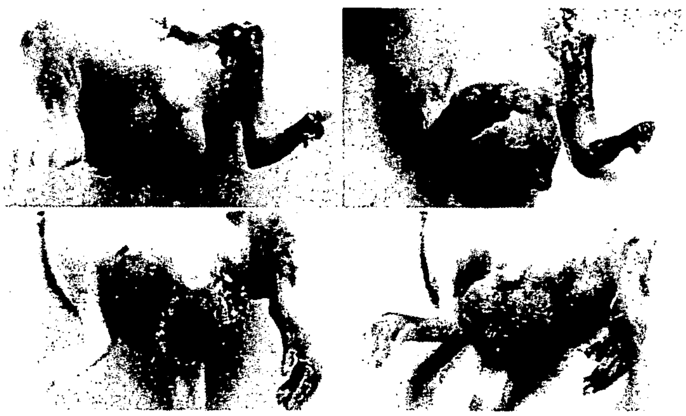

A | B
C | D

AxCAhAng1

DAY 3 DAY 10

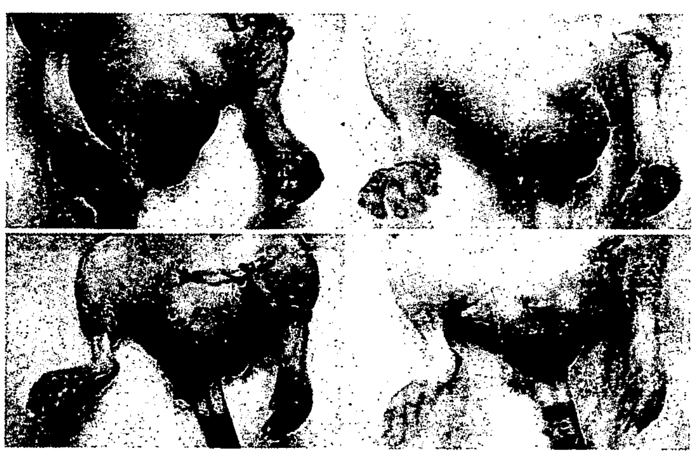

E | F
G | H

FIG. 8

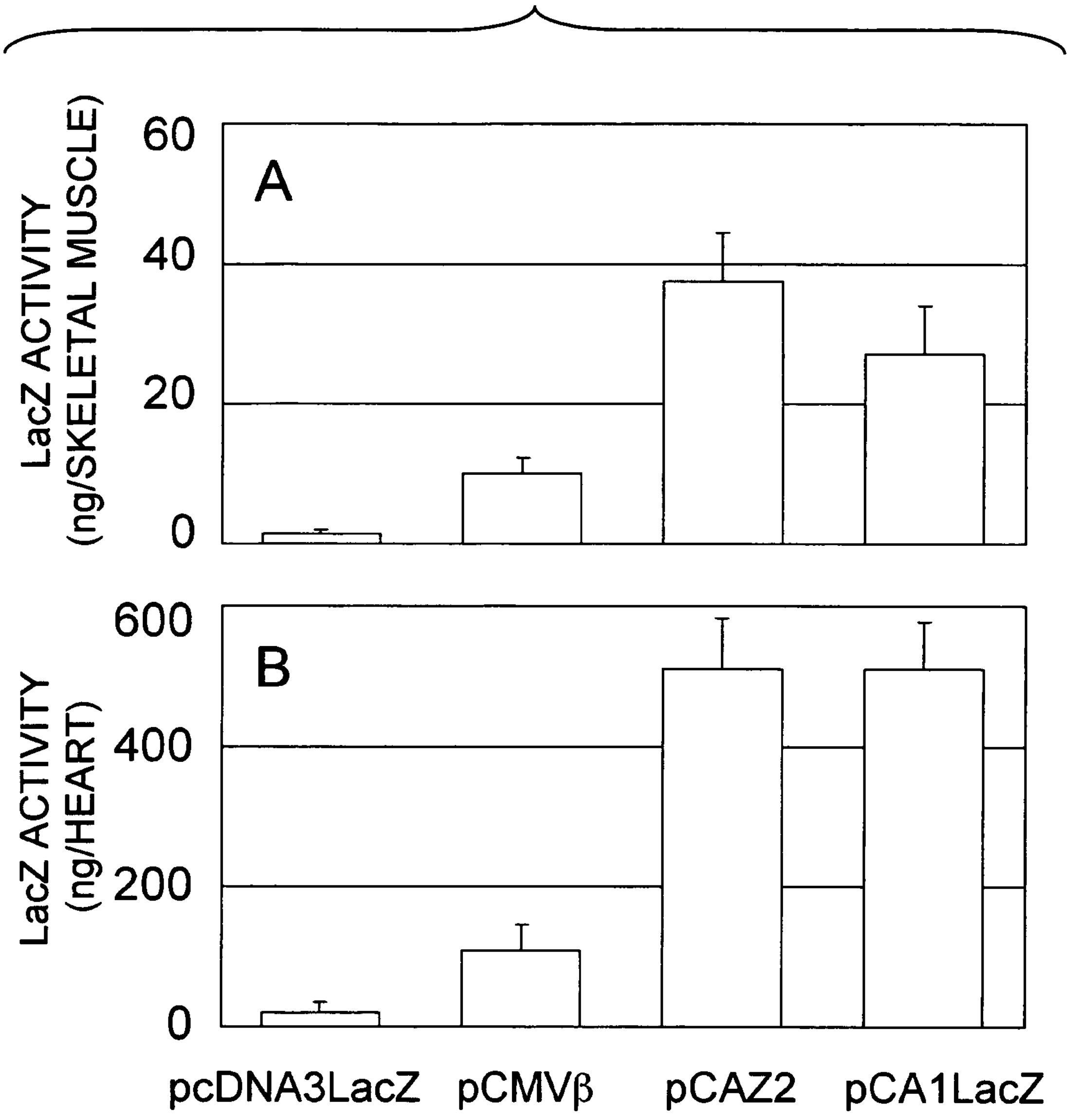
A
B
60
40
20
0
600
400
200
0
LacZ ACTIVITY (ng/SKELETAL MUSCLE)
LacZ ACTIVITY (ng/HEART)
pcDNA3LacZ
pCMVβ
pCAZ2
pCA1LacZ

FIG. 9

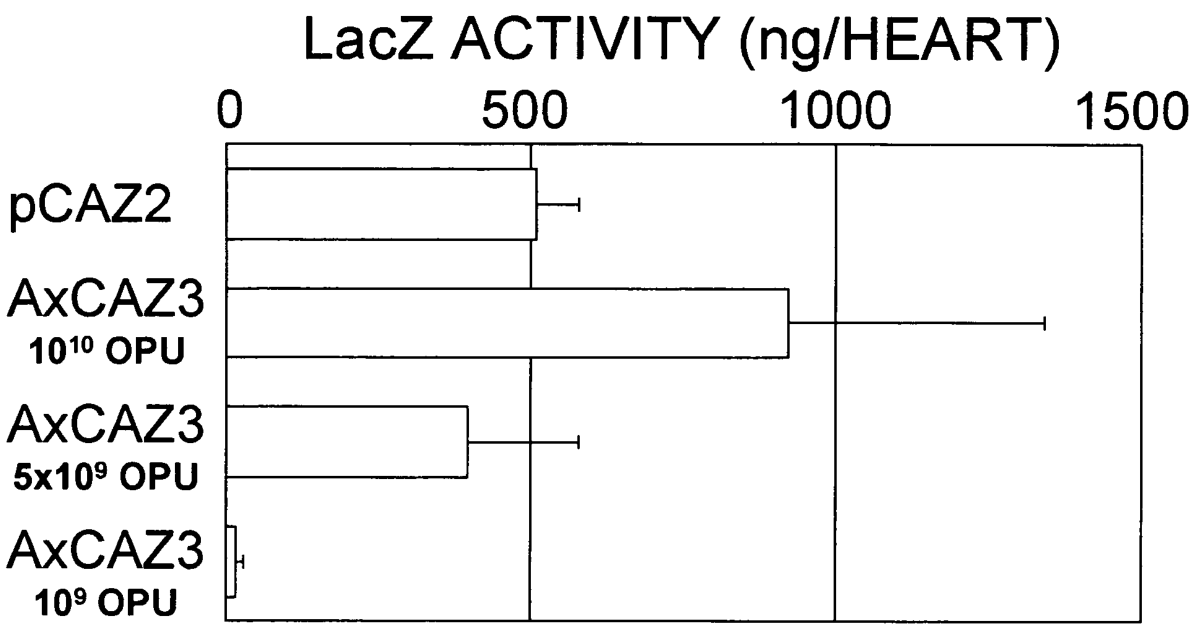
LacZ ACTIVITY (ng/HEART)
0
500
1000
1500
pCAZ2
AxCAZ3
10^10 OPU
AxCAZ3
5x10^9 OPU
AxCAZ3
10^9 OPU

# FIG. 10

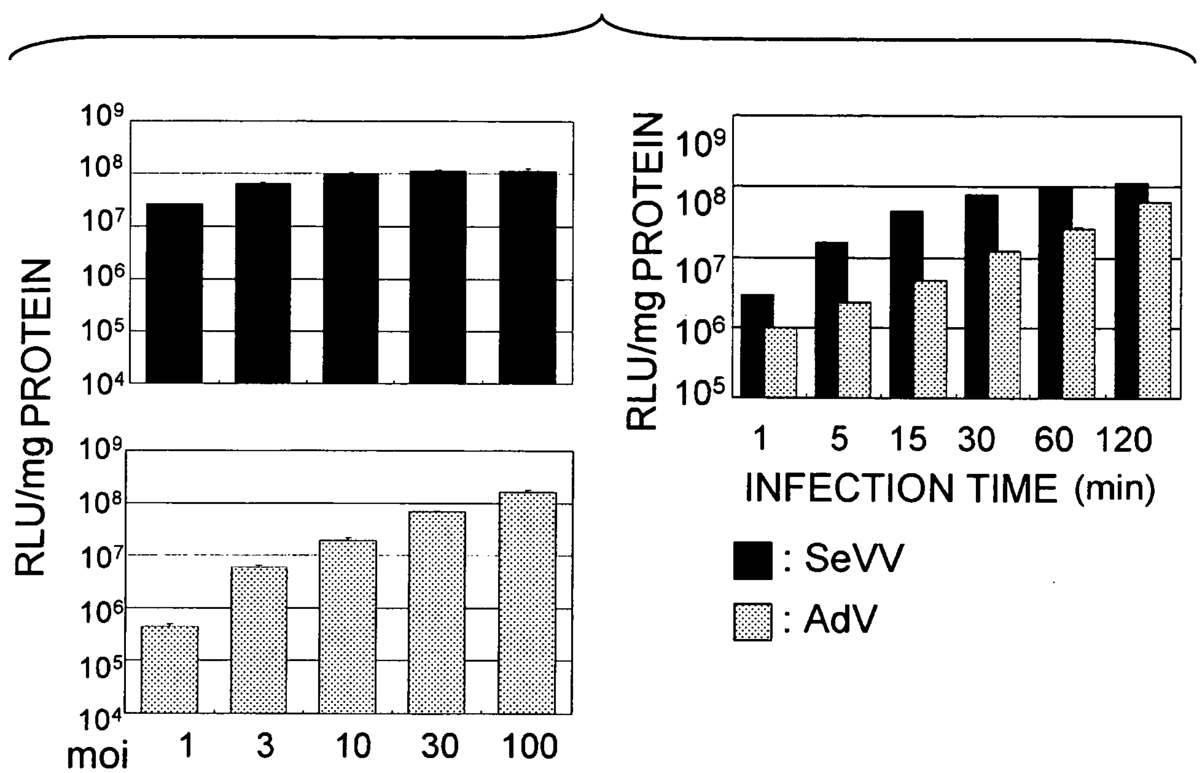

RLU/mg PROTEIN
10⁹
10⁸
10⁷
10⁶
10⁵
10⁴
moi
1
3
10
30
100
1
5
15
30
60
120
INFECTION TIME (min)
: SeVV
: AdV

FIG. 11

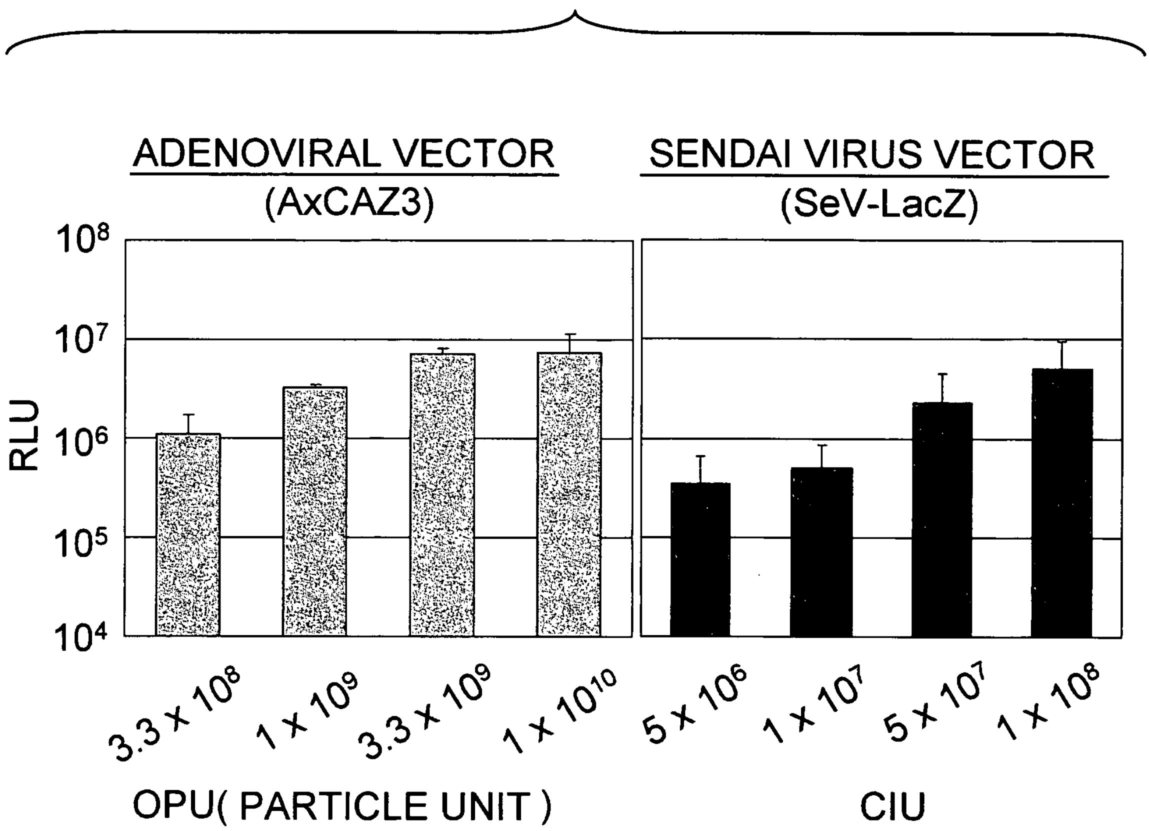
ADENOVIRAL VECTOR
(AxCAZ3)
SENDAI VIRUS VECTOR
(SeV-LacZ)
RLU
10^8
10^7
10^6
10^5
10^4
3.3 x 10^8
1 x 10^9
3.3 x 10^9
1 x 10^10
5 x 10^6
1 x 10^7
5 x 10^7
1 x 10^8
OPU( PARTICLE UNIT )
CIU

FIG. 12

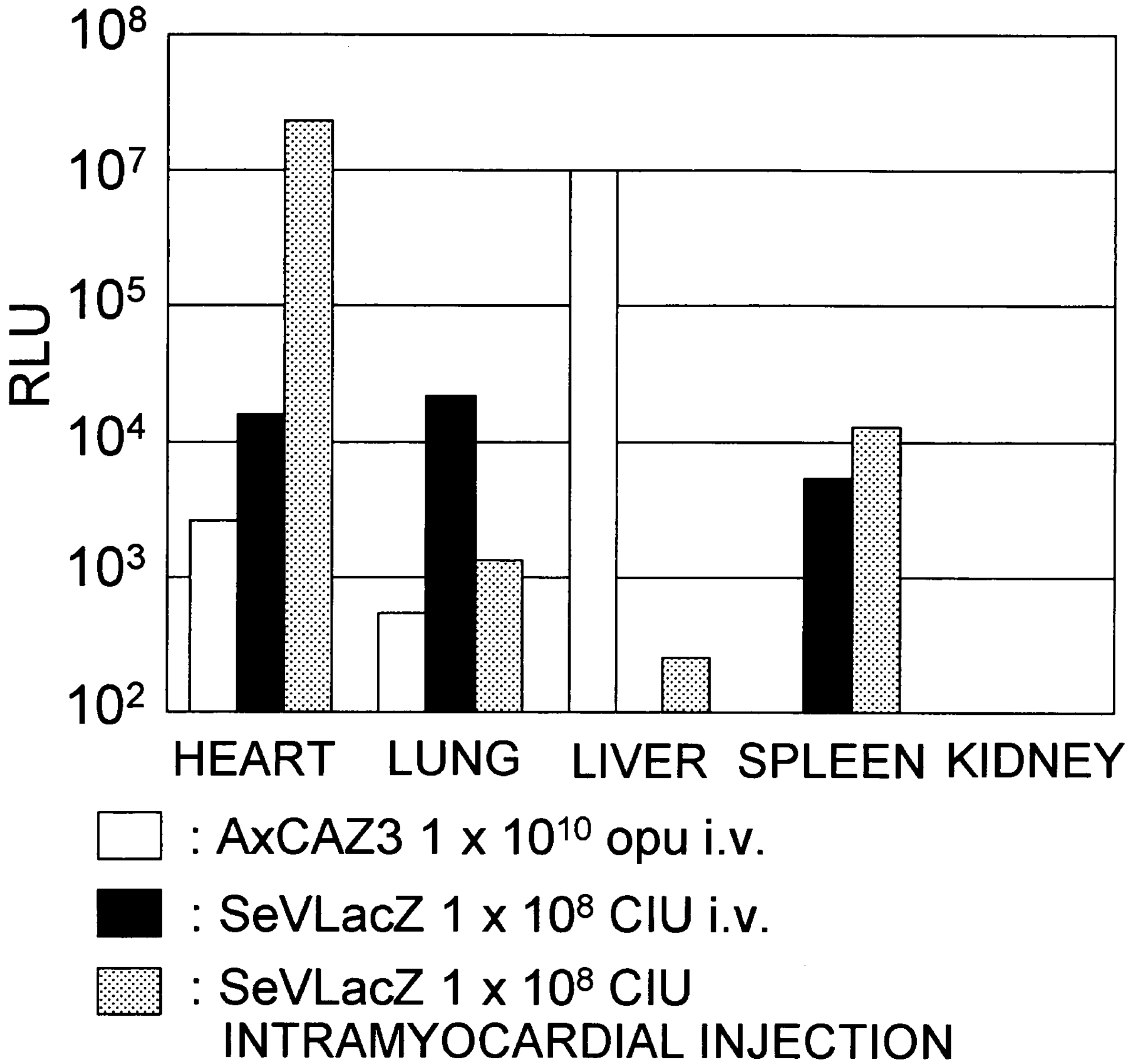
RLU
10^8
10^7
10^5
10^4
10^3
10^2
HEART
LUNG
LIVER
SPLEEN
KIDNEY
: AxCAZ3 1 x 10^10 opu i.v.
: SeVLacZ 1 x 10^8 CIU i.v.
: SeVLacZ 1 x 10^8 CIU INTRAMYOCARDIAL INJECTION

FIG. 13

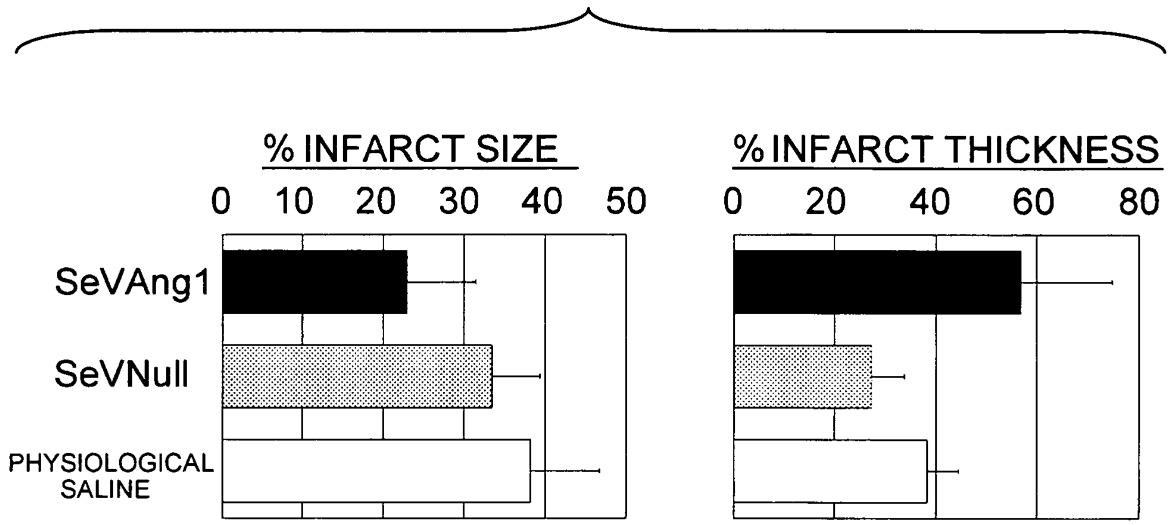
% INFARCT SIZE
0 10 20 30 40 50
SeVAng1
SeVNull
PHYSIOLOGICAL SALINE
% INFARCT THICKNESS
0 20 40 60 80

FIG. 14

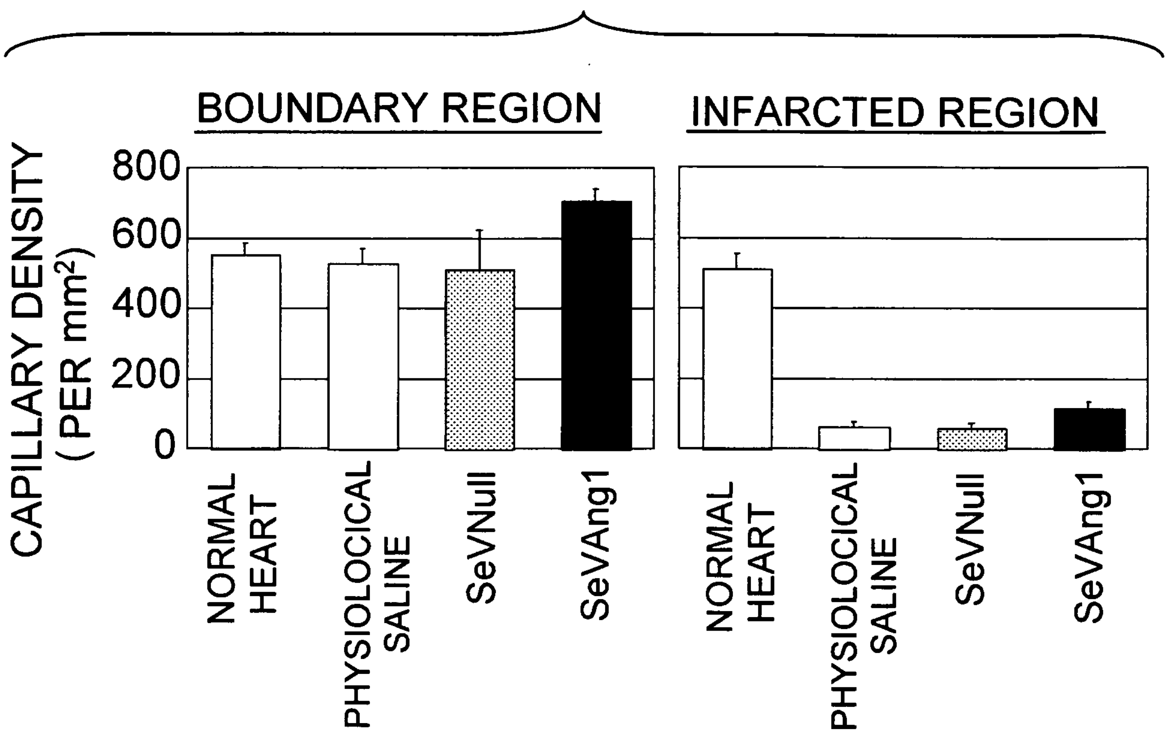
BOUNDARY REGION
INFARCTED REGION
CAPILLARY DENSITY (PER mm2)
800
600
400
200
0
NORMAL HEART
PHYSIOLOCICAL SALINE
SeVNull
SeVAng1
NORMAL HEART
PHYSIOLOCICAL SALINE
SeVNull
SeVAng1

FIG. 15

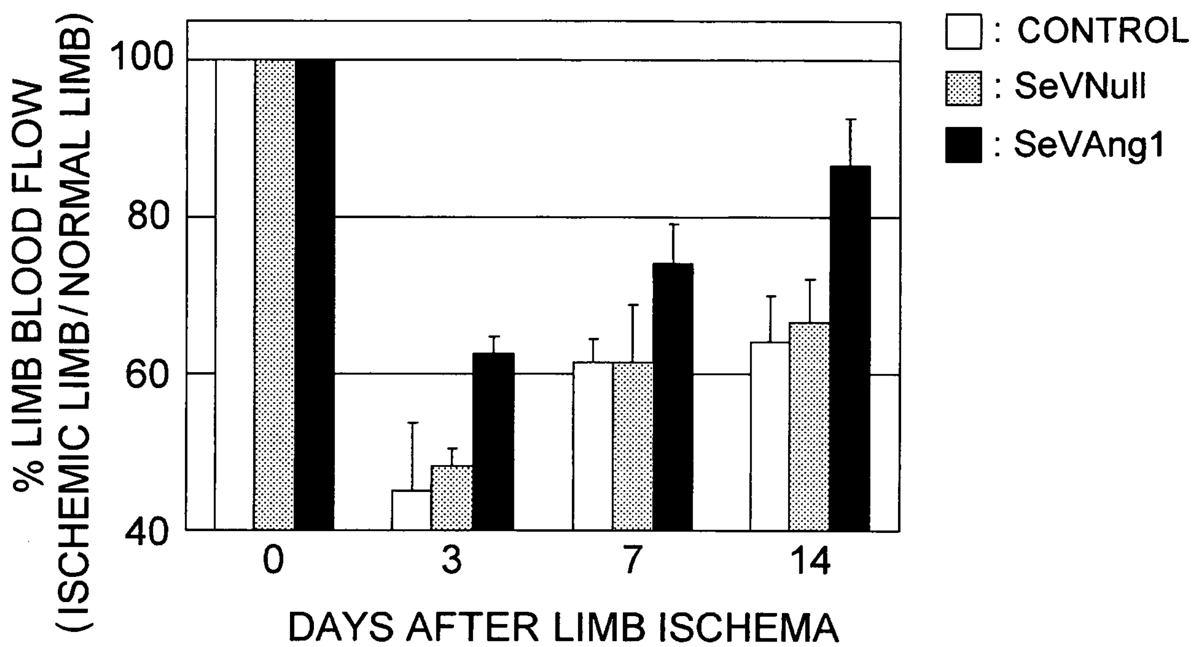
% LIMB BLOOD FLOW
(ISCHEMIC LIMB/NORMAL LIMB)
100
80
60
40
0
3
7
14
DAYS AFTER LIMB ISCHEMA
: CONTROL
: SeVNull
: SeVAng1

FIG. 16

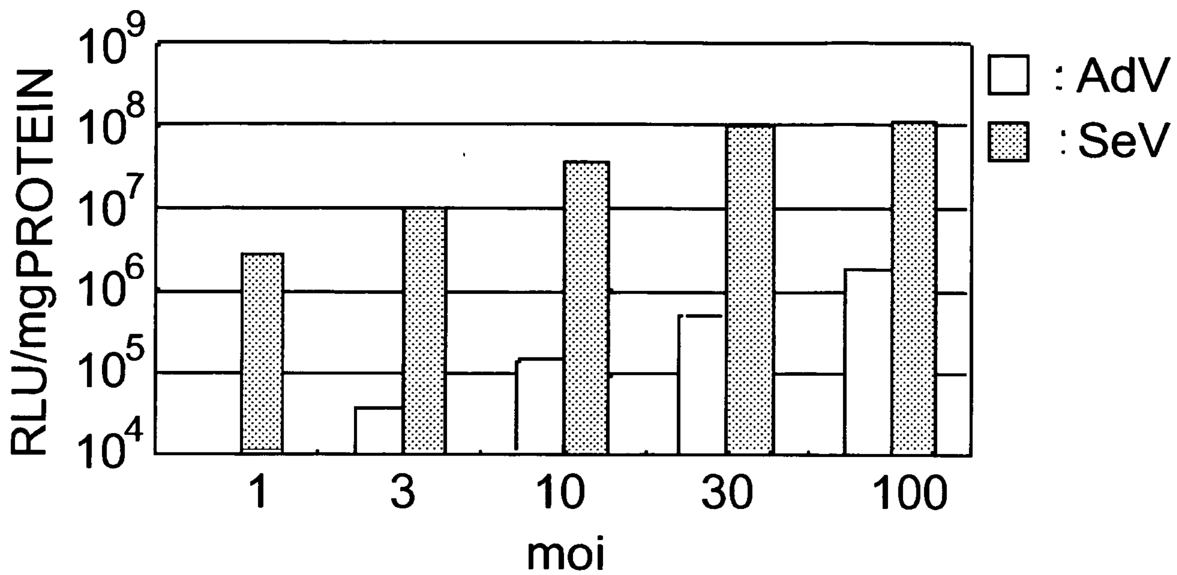

FIG. 17

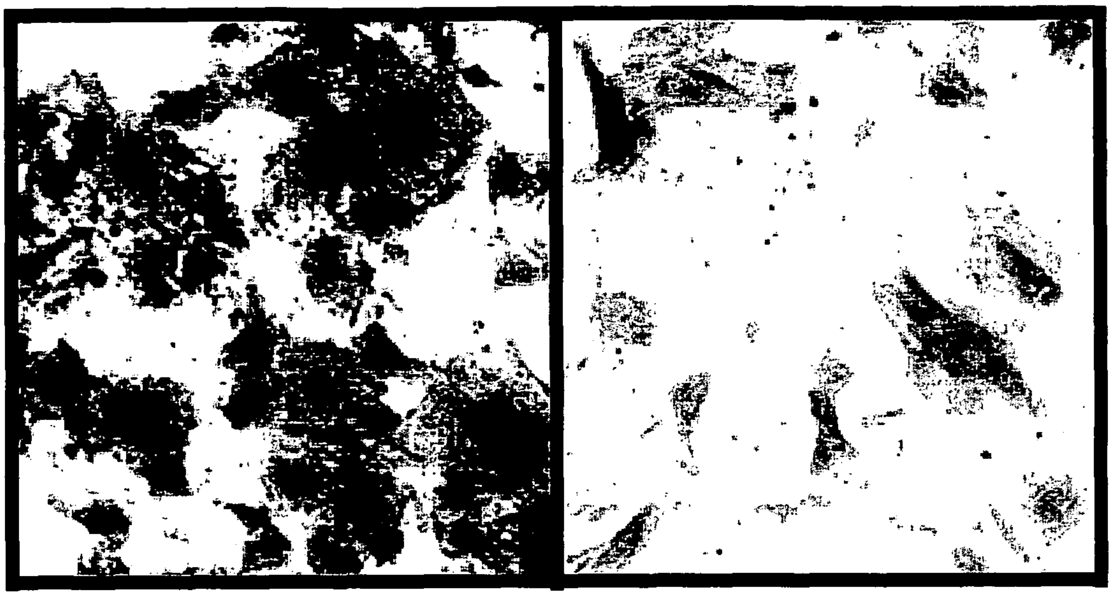

(A) SeVV (B) AdV

FIG. 18

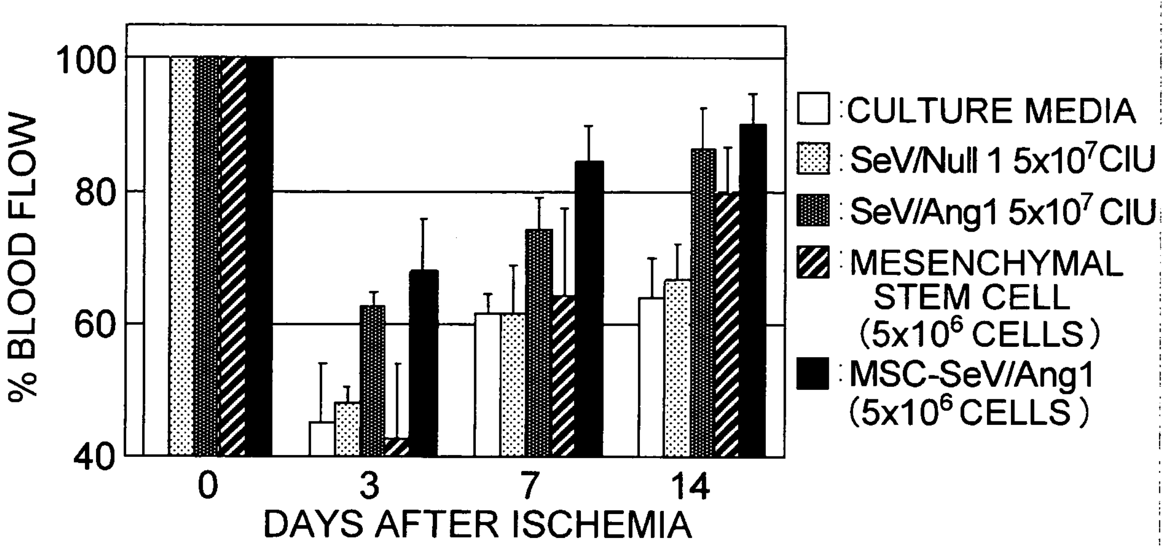
100
80
60
40
% BLOOD FLOW
0
3
7
14
DAYS AFTER ISCHEMIA
:CULTURE MEDIA
:SeV/Null 1 5x10^7 CIU
:SeV/Ang1 5x10^7 CIU
:MESENCHYMAL STEM CELL (5x10^6 CELLS)
:MSC-SeV/Ang1 (5x10^6 CELLS)

# FIG. 19

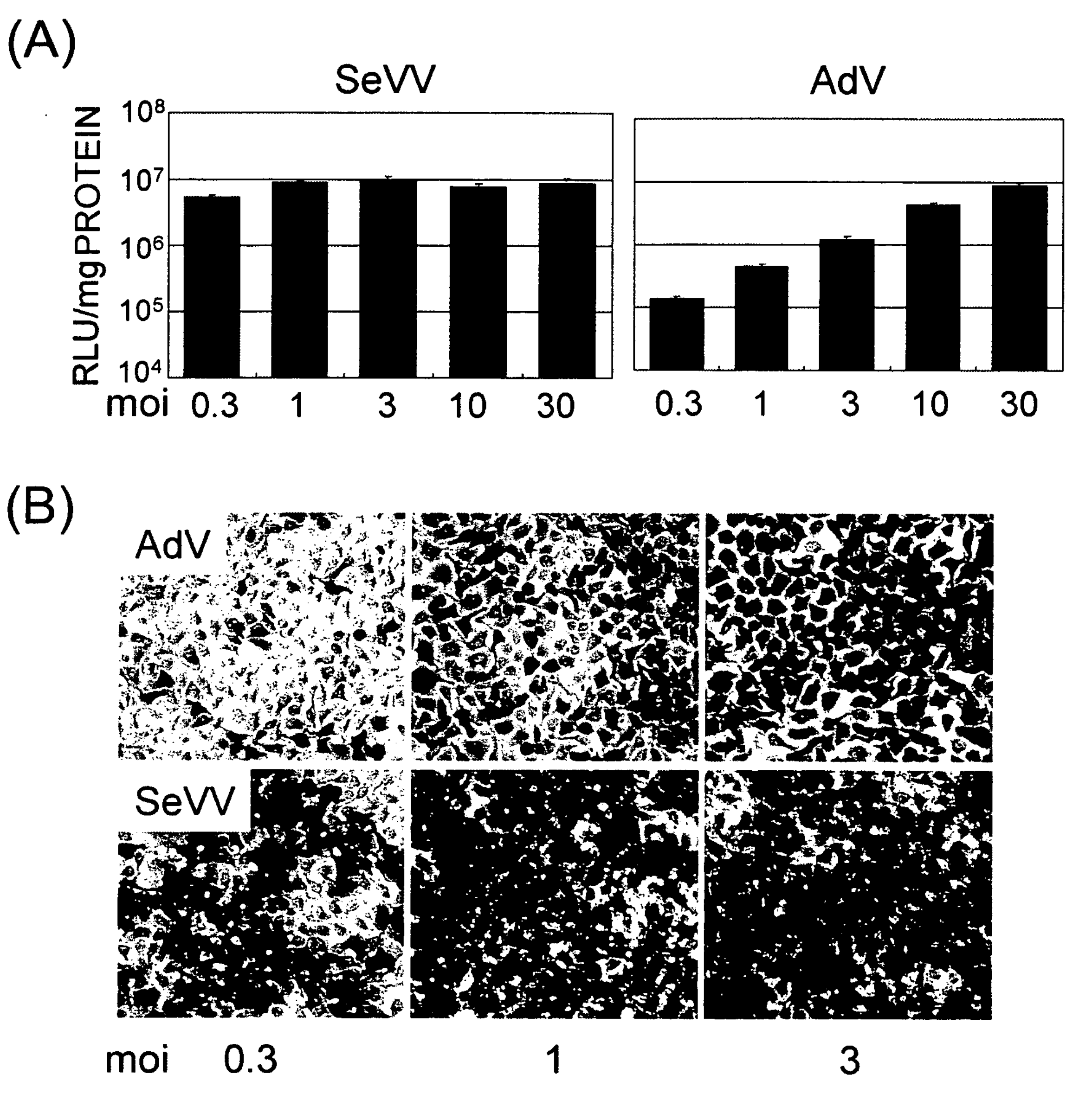
(A)
SeVV
AdV
RLU/mgPROTEIN
10^8
10^7
10^6
10^5
10^4
moi 0.3 1 3 10 30
0.3 1 3 10 30
(B)
AdV
SeVV
moi 0.3 1 3

# FIG. 20

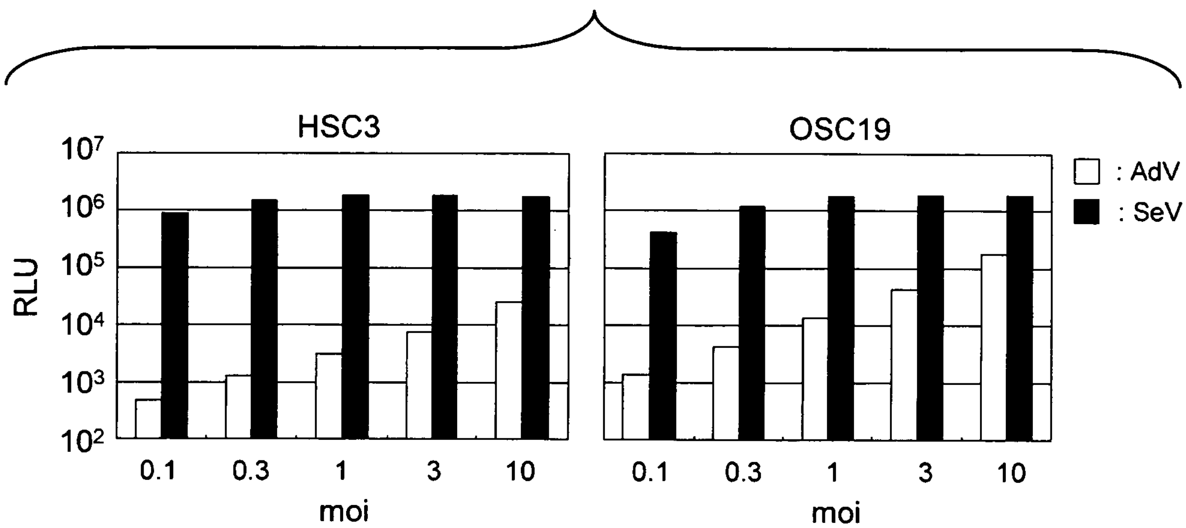

FIG. 21

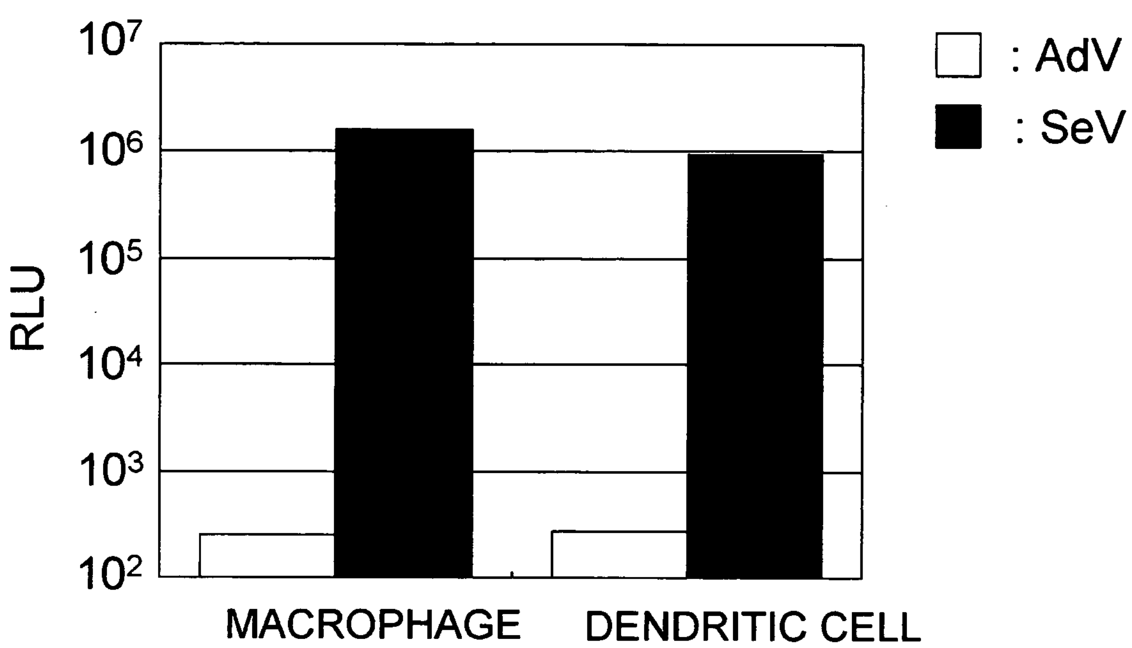

10^7
10^6
10^5
10^4
10^3
10^2
RLU
: AdV
: SeV
MACROPHAGE
DENDRITIC CELL

## INTERNATIONAL SEARCH REPORT

International application No.
PCT/JP2004/000957

A. CLASSIFICATION OF SUBJECT MATTER
Int.Cl[7] C12N15/86, C12N15/861, C12N5/10, A61K48/00, A61P9/10

According to International Patent Classification (IPC) or to both national classification and IPC

B. FIELDS SEARCHED
Minimum documentation searched (classification system followed by classification symbols)
Int.Cl[7] C12N15/86, C12N15/861, C12N5/10, A61K48/00, A61P9/10

Documentation searched other than minimum documentation to the extent that such documents are included in the fields searched

Electronic data base consulted during the international search (name of data base and, where practicable, search terms used)
WPI(DIALOG), BIOSIS(DIALOG), MEDLINE(STN)

C. DOCUMENTS CONSIDERED TO BE RELEVANT

| Category* | Citation of document, with indication, where appropriate, of the relevant passages | Relevant to claim No. |
|---|---|---|
| X<br>Y | HATTORI, K. et al., Vascular endothelial growth factor and angiopoietin-1 stimulate postnatal hematopoiesis by recruitment of vasculogenic and hematopoietic stem cells., J.Exp.Med., Vol.193, No.9, pages 1005 to 1014 (2001) | 14,15<br>16-19 |
| X<br>Y | TAKAKURA, N. et al., A role for hematopoietic stem cells in promoting angiogenesis., Cell, Vol.102, No.2, pages 199 to 209 (2000) | 14,15<br>16-19 |
| Y | WO 02/100441 A2 (Dnavec Res.Inc.), 19 December, 2002 (19.12.02), (Family: none) | 16-19 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 March, 2004 (01.03.04) | 16 March, 2004 (16.03.04) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/000957

C (Continuation). DOCUMENTS CONSIDERED TO BE RELEVANT

| Category* | Citation of document, with indication, where appropriate, of the relevant passages | Relevant to claim No. |
|---|---|---|
| Y | WO 97/27310 A1 (Oxford Biomedica UK Ltd.), 31 July, 1997 (31.07.97), & EP 880594 A1 & JP 2000-500986 A | 17 |
| P,A | TAKAHASHI, K. et al., Adenoviral-delivered angiopoietin-1 reduces the infarction and attenuates the progression of cardiac dysfunction in the Mol Ther, Vol.8, No.4, pages 584 to 592 (2003 October) | 14-19 |

Form PCT/ISA/210 (continuation of second sheet) (January 2004)

**INTERNATIONAL SEARCH REPORT**

International application No.
PCT/JP2004/000957

**Box No. I Nucleotide and/or amino acid sequence(s) (Continuation of item1.b of the first sheet)**

1. With regard to any nucleotide and/or amino acid sequence disclosed in the international application and necessary to the claimed invention, the international search was carried out on the basis of:

   a. type of material
      - [X] a sequence listing
      - [ ] table(s) related to the sequence listing

   b. format of material
      - [ ] in written format
      - [X] in computer readable form

   c. time of filing/furnishing
      - [ ] contained in the international application as filed
      - [X] filed together with the international application in computer readable form
      - [ ] furnished subsequently to this Authority for the purposes of search

2. [X] In addition, in the case that more than one version or copy of a sequence listing and/or table relating thereto has been filed or furnished, the required statements that the information in the subsequent or additional copies is identical to that in the application as filed or does not go beyond the application as filed, as appropriate, were furnished.

3. Additional comments:

Form PCT/ISA/210 (continuation of first sheet (1)) (January 2004)

**INTERNATIONAL SEARCH REPORT**

International application No.
PCT/JP2004/000957

**Box No. II Observations where certain claims were found unsearchable (Continuation of item 2 of first sheet)**

This international search report has not been established in respect of certain claims under Article 17(2)(a) for the following reasons:

1. [X] Claims Nos.: 1 to 13
because they relate to subject matter not required to be searched by this Authority, namely:
The inventions as set forth in these claims are relevant to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest.

[ ] No protest accompanied the payment of additional search fees.

Form PCT/ISA/210 (continuation of first sheet (2)) (January 2004)

SEQUENCE LISTING

<110> DNAVEC RESEARCH INC.

<120> Method of treating ischemic disease

<130> D3-A0208P

<150> JP 2003-040806
<151> 2003-02-19

<160> 9

<170> PatentIn version 3.1

<210> 1
<211> 3372
<212> DNA
<213> Homo sapiens

<220>
<221> CDS
<222> (1)..(3372)
<223>

<400> 1
```
atg gac tct tta gcc agc tta gtt ctc tgt gga gtc agc ttg ctc ctt      48
Met Asp Ser Leu Ala Ser Leu Val Leu Cys Gly Val Ser Leu Leu Leu
1               5                   10                  15

tct gga act gtg gaa ggt gcc atg gac ttg atc ttg atc aat tcc cta      96
Ser Gly Thr Val Glu Gly Ala Met Asp Leu Ile Leu Ile Asn Ser Leu
            20                  25                  30

cct ctt gta tct gat gct gaa aca tct ctc acc tgc att gcc tct ggg     144
Pro Leu Val Ser Asp Ala Glu Thr Ser Leu Thr Cys Ile Ala Ser Gly
        35                  40                  45

tgg cgc ccc cat gag ccc atc acc ata gga agg gac ttt gaa gcc tta     192
Trp Arg Pro His Glu Pro Ile Thr Ile Gly Arg Asp Phe Glu Ala Leu
```

50 55 60

atg aac cag cac cag gat ccg ctg gaa gtt act caa gat gtg acc aga 240
Met Asn Gln His Gln Asp Pro Leu Glu Val Thr Gln Asp Val Thr Arg
65 70 75 80

gaa tgg gct aaa aaa gtt gtt tgg aag aga gaa aag gct agt aag atc 288
Glu Trp Ala Lys Lys Val Val Trp Lys Arg Glu Lys Ala Ser Lys Ile
85 90 95

aat ggt gct tat ttc tgt gaa ggg cga gtt cga gga gag gca atc agg 336
Asn Gly Ala Tyr Phe Cys Glu Gly Arg Val Arg Gly Glu Ala Ile Arg
100 105 110

ata cga acc atg aag atg cgt caa caa gct tcc ttc cta cca gct act 384
Ile Arg Thr Met Lys Met Arg Gln Gln Ala Ser Phe Leu Pro Ala Thr
115 120 125

tta act atg act gtg gac aag gga gat aac gtg aac ata tct ttc aaa 432
Leu Thr Met Thr Val Asp Lys Gly Asp Asn Val Asn Ile Ser Phe Lys
130 135 140

aag gta ttg att aaa gaa gaa gat gca gtg att tac aaa aat ggt tcc 480
Lys Val Leu Ile Lys Glu Glu Asp Ala Val Ile Tyr Lys Asn Gly Ser
145 150 155 160

ttc atc cat tca gtg ccc cgg cat gaa gta cct gat att cta gaa gta 528
Phe Ile His Ser Val Pro Arg His Glu Val Pro Asp Ile Leu Glu Val
165 170 175

cac ctg cct cat gct cag ccc cag gat gct gga gtg tac tcg gcc agg 576
His Leu Pro His Ala Gln Pro Gln Asp Ala Gly Val Tyr Ser Ala Arg
180 185 190

tat ata gga gga aac ctc ttc acc tcg gcc ttc acc agg ctg ata gtc 624
Tyr Ile Gly Gly Asn Leu Phe Thr Ser Ala Phe Thr Arg Leu Ile Val
195 200 205

cgg aga tgt gaa gcc cag aag tgg gga cct gaa tgc aac cat ctc tgt 672
Arg Arg Cys Glu Ala Gln Lys Trp Gly Pro Glu Cys Asn His Leu Cys

210 215 220

act gct tgt atg aac aat ggt gtc tgc cat gaa gat act gga gaa tgc 720
Thr Ala Cys Met Asn Asn Gly Val Cys His Glu Asp Thr Gly Glu Cys
225 230 235 240

att tgc cct cct ggg ttt atg gga agg acg tgt gag aag gct tgt gaa 768
Ile Cys Pro Pro Gly Phe Met Gly Arg Thr Cys Glu Lys Ala Cys Glu
245 250 255

ctg cac acg ttt ggc aga act tgt aaa gaa agg tgc agt gga caa gag 816
Leu His Thr Phe Gly Arg Thr Cys Lys Glu Arg Cys Ser Gly Gln Glu
260 265 270

gga tgc aag tct tat gtg ttc tgt ctc cct gac ccc tat ggg tgt tcc 864
Gly Cys Lys Ser Tyr Val Phe Cys Leu Pro Asp Pro Tyr Gly Cys Ser
275 280 285

tgt gcc aca ggc tgg aag ggt ctg cag tgc aat gaa gca tgc cac cct 912
Cys Ala Thr Gly Trp Lys Gly Leu Gln Cys Asn Glu Ala Cys His Pro
290 295 300

ggt ttt tac ggg cca gat tgt aag ctt agg tgc agc tgc aac aat ggg 960
Gly Phe Tyr Gly Pro Asp Cys Lys Leu Arg Cys Ser Cys Asn Asn Gly
305 310 315 320

gag atg tgt gat cgc ttc caa gga tgt ctc tgc tct cca gga tgg cag 1008
Glu Met Cys Asp Arg Phe Gln Gly Cys Leu Cys Ser Pro Gly Trp Gln
325 330 335

ggg ctc cag tgt gag aga gaa ggc ata ccg agg atg acc cca aag ata 1056
Gly Leu Gln Cys Glu Arg Glu Gly Ile Pro Arg Met Thr Pro Lys Ile
340 345 350

gtg gat ttg cca gat cat ata gaa gta aac agt ggt aaa ttt aat ccc 1104
Val Asp Leu Pro Asp His Ile Glu Val Asn Ser Gly Lys Phe Asn Pro
355 360 365

att tgc aaa gct tct ggc tgg ccg cta cct act aat gaa gaa atg acc 1152
Ile Cys Lys Ala Ser Gly Trp Pro Leu Pro Thr Asn Glu Glu Met Thr

370 375 380

ctg gtg aag ccg gat ggg aca gtg ctc cat cca aaa gac ttt aac cat 1200
Leu Val Lys Pro Asp Gly Thr Val Leu His Pro Lys Asp Phe Asn His
385 390 395 400

acg gat cat ttc tca gta gcc ata ttc acc atc cac cgg atc ctc ccc 1248
Thr Asp His Phe Ser Val Ala Ile Phe Thr Ile His Arg Ile Leu Pro
405 410 415

cct gac tca gga gtt tgg gtc tgc agt gtg aac aca gtg gct ggg atg 1296
Pro Asp Ser Gly Val Trp Val Cys Ser Val Asn Thr Val Ala Gly Met
420 425 430

gtg gaa aag ccc ttc aac att tct gtt aaa gtt ctt cca aag ccc ctg 1344
Val Glu Lys Pro Phe Asn Ile Ser Val Lys Val Leu Pro Lys Pro Leu
435 440 445

aat gcc cca aac gtg att gac act gga cat aac ttt gct gtc atc aac 1392
Asn Ala Pro Asn Val Ile Asp Thr Gly His Asn Phe Ala Val Ile Asn
450 455 460

atc agc tct gag cct tac ttt ggg gat gga cca atc aaa tcc aag aag 1440
Ile Ser Ser Glu Pro Tyr Phe Gly Asp Gly Pro Ile Lys Ser Lys Lys
465 470 475 480

ctt cta. tac aaa ccc gtt aat cac tat gag gct tgg caa cat att caa 1488
Leu Leu Tyr Lys Pro Val Asn His Tyr Glu Ala Trp Gln His Ile Gln
485 490 495

gtg aca aat gag att gtt aca ctc aac tat ttg gaa cct cgg aca gaa 1536
Val Thr Asn Glu Ile Val Thr Leu Asn Tyr Leu Glu Pro Arg Thr Glu
500 505 510

tat gaa ctc tgt gtg caa ctg gtc cgt cgt gga gag ggt ggg gaa ggg 1584
Tyr Glu Leu Cys Val Gln Leu Val Arg Arg Gly Glu Gly Gly Glu Gly
515 520 525

cat cct gga cct gtg aga cgc ttc aca aca gct tct atc gga ctc cct 1632
His Pro Gly Pro Val Arg Arg Phe Thr Thr Ala Ser Ile Gly Leu Pro

530 535 540

cct cca aga ggt cta aat ctc ctg cct aaa agt cag acc act cta aat 1680
Pro Pro Arg Gly Leu Asn Leu Leu Pro Lys Ser Gln Thr Thr Leu Asn
545 550 555 560

ttg acc tgg caa cca ata ttt cca agc tcg gaa gat gac ttt tat gtt 1728
Leu Thr Trp Gln Pro Ile Phe Pro Ser Ser Glu Asp Asp Phe Tyr Val
565 570 575

gaa gtg gag aga agg tct gtg caa aaa agt gat cag cag aat att aaa 1776
Glu Val Glu Arg Arg Ser Val Gln Lys Ser Asp Gln Gln Asn Ile Lys
580 585 590

gtt cca ggc aac ttg act tcg gtg cta ctt aac aac tta cat ccc agg 1824
Val Pro Gly Asn Leu Thr Ser Val Leu Leu Asn Asn Leu His Pro Arg
595 600 605

gag cag tac gtg gtc cga gct aga gtc aac acc aag gcc cag ggg gaa 1872
Glu Gln Tyr Val Val Arg Ala Arg Val Asn Thr Lys Ala Gln Gly Glu
610 615 620

tgg agt gaa gat ctc act gct tgg acc ctt agt gac att ctt cct cct 1920
Trp Ser Glu Asp Leu Thr Ala Trp Thr Leu Ser Asp Ile Leu Pro Pro
625 630 635 640

caa cca gaa aac atc aag att tcc aac att aca cac tcc tcg gct gtg 1968
Gln Pro Glu Asn Ile Lys Ile Ser Asn Ile Thr His Ser Ser Ala Val
645 650 655

att tct tgg aca ata ttg gat ggc tat tct att tct tct att act atc 2016
Ile Ser Trp Thr Ile Leu Asp Gly Tyr Ser Ile Ser Ser Ile Thr Ile
660 665 670

cgt tac aag gtt caa ggc aag aat gaa gac cag cac gtt gat gtg aag 2064
Arg Tyr Lys Val Gln Gly Lys Asn Glu Asp Gln His Val Asp Val Lys
675 680 685

ata aag aat gcc acc atc att cag tat cag ctc aag ggc cta gag cct 2112
Ile Lys Asn Ala Thr Ile Ile Gln Tyr Gln Leu Lys Gly Leu Glu Pro

690 695 700

gaa aca gca tac cag gtg gac att ttt gca gag aac aac ata ggg tca 2160
Glu Thr Ala Tyr Gln Val Asp Ile Phe Ala Glu Asn Asn Ile Gly Ser
705 710 715 720

agc aac cca gcc ttt tct cat gaa ctg gtg acc ctc cca gaa tct caa 2208
Ser Asn Pro Ala Phe Ser His Glu Leu Val Thr Leu Pro Glu Ser Gln
725 730 735

gca cca gcg gac ctc gga ggg ggg aag atg ctg ctt ata gcc atc ctt 2256
Ala Pro Ala Asp Leu Gly Gly Gly Lys Met Leu Leu Ile Ala Ile Leu
740 745 750

ggc tct gct gga atg acc tgc ctg act gtg ctg ttg gcc ttt ctg atc 2304
Gly Ser Ala Gly Met Thr Cys Leu Thr Val Leu Leu Ala Phe Leu Ile
755 760 765

ata ttg caa ttg aag agg gca aat gtg caa agg aga atg gcc caa gcc 2352
Ile Leu Gln Leu Lys Arg Ala Asn Val Gln Arg Arg Met Ala Gln Ala
770 775 780

ttc caa aac gtg agg gaa gaa cca gct gtg cag ttc aac tca ggg act 2400
Phe Gln Asn Val Arg Glu Glu Pro Ala Val Gln Phe Asn Ser Gly Thr
785 790 795 800

ctg gcc cta aac agg aag gtc aaa aac aac cca gat cct aca att tat 2448
Leu Ala Leu Asn Arg Lys Val Lys Asn Asn Pro Asp Pro Thr Ile Tyr
805 810 815

cca gtg ctt gac tgg aat gac atc aaa ttt caa gat gtg att ggg gag 2496
Pro Val Leu Asp Trp Asn Asp Ile Lys Phe Gln Asp Val Ile Gly Glu
820 825 830

ggc aat ttt ggc caa gtt ctt aag gcg cgc atc aag aag gat ggg tta 2544
Gly Asn Phe Gly Gln Val Leu Lys Ala Arg Ile Lys Lys Asp Gly Leu
835 840 845

cgg atg gat gct gcc atc aaa aga atg aaa gaa tat gcc tcc aaa gat 2592
Arg Met Asp Ala Ala Ile Lys Arg Met Lys Glu Tyr Ala Ser Lys Asp

850 855 860

gat cac agg gac ttt gca gga gaa ctg gaa gtt ctt tgt aaa ctt gga 2640
Asp His Arg Asp Phe Ala Gly Glu Leu Glu Val Leu Cys Lys Leu Gly
865 870 875 880

cac cat cca aac atc atc aat ctc tta gga gca tgt gaa cat cga ggc 2688
His His Pro Asn Ile Ile Asn Leu Leu Gly Ala Cys Glu His Arg Gly
885 890 895

tac ttg tac ctg gcc att gag tac gcg ccc cat gga aac ctt ctg gac 2736
Tyr Leu Tyr Leu Ala Ile Glu Tyr Ala Pro His Gly Asn Leu Leu Asp
900 905 910

ttc ctt cgc aag agc cgt gtg ctg gag acg gac cca gca ttt gcc att 2784
Phe Leu Arg Lys Ser Arg Val Leu Glu Thr Asp Pro Ala Phe Ala Ile
915 920 925

gcc aat agc acc gcg tcc aca ctg tcc tcc cag cag ctc ctt cac ttc 2832
Ala Asn Ser Thr Ala Ser Thr Leu Ser Ser Gln Gln Leu Leu His Phe
930 935 940

gct gcc gac gtg gcc cgg ggc atg gac tac ttg agc caa aaa cag ttt 2880
Ala Ala Asp Val Ala Arg Gly Met Asp Tyr Leu Ser Gln Lys Gln Phe
945 950 955 960

atc cac agg gat ctg gct gcc aga aac att tta gtt ggt gaa aac tat 2928
Ile His Arg Asp Leu Ala Ala Arg Asn Ile Leu Val Gly Glu Asn Tyr
965 970 975

gtg gca aaa ata gca gat ttt gga ttg tcc cga ggt caa gag gtg tac 2976
Val Ala Lys Ile Ala Asp Phe Gly Leu Ser Arg Gly Gln Glu Val Tyr
980 985 990

gtg aaa aag aca atg gga agg ctc cca gtg cgc tgg atg gcc atc gag 3024
Val Lys Lys Thr Met Gly Arg Leu Pro Val Arg Trp Met Ala Ile Glu
995 1000 1005

tca ctg aat tac agt gtg tac aca acc aac agt gat gta tgg tcc 3069
Ser Leu Asn Tyr Ser Val Tyr Thr Thr Asn Ser Asp Val Trp Ser

1010 1015 1020

tat ggt gtg tta cta tgg gag att gtt agc tta gga ggc aca ccc 3114
Tyr Gly Val Leu Leu Trp Glu Ile Val Ser Leu Gly Gly Thr Pro
1025 1030 1035

tac tgc ggg atg act tgt gca gaa ctc tac gag aag ctg ccc cag 3159
Tyr Cys Gly Met Thr Cys Ala Glu Leu Tyr Glu Lys Leu Pro Gln
1040 1045 1050

ggc tac aga ctg gag aag ccc ctg aac tgt gat gat gag gtg tat 3204
Gly Tyr Arg Leu Glu Lys Pro Leu Asn Cys Asp Asp Glu Val Tyr
1055 1060 1065

gat cta atg aga caa tgc tgg cgg gag aag cct tat gag agg cca 3249
Asp Leu Met Arg Gln Cys Trp Arg Glu Lys Pro Tyr Glu Arg Pro
1070 1075 1080

tca ttt gcc cag ata ttg gtg tcc tta aac aga atg tta gag gag 3294
Ser Phe Ala Gln Ile Leu Val Ser Leu Asn Arg Met Leu Glu Glu
1085 1090 1095

cga aag acc tac gtg aat acc acg ctt tat gag aag ttt act tat 3339
Arg Lys Thr Tyr Val Asn Thr Thr Leu Tyr Glu Lys Phe Thr Tyr
1100 1105 1110

gca gga att gac tgt tct gct gaa gaa gcg gcc 3372
Ala Gly Ile Asp Cys Ser Ala Glu Glu Ala Ala
1115 1120

<210> 2
<211> 1124
<212> PRT
<213> Homo sapiens

<400> 2
Met Asp Ser Leu Ala Ser Leu Val Leu Cys Gly Val Ser Leu Leu Leu
1 5 10 15

Ser Gly Thr Val Glu Gly Ala Met Asp Leu Ile Leu Ile Asn Ser Leu
20 25 30

Pro Leu Val Ser Asp Ala Glu Thr Ser Leu Thr Cys Ile Ala Ser Gly
35 40 45

Trp Arg Pro His Glu Pro Ile Thr Ile Gly Arg Asp Phe Glu Ala Leu
50 55 60

Met Asn Gln His Gln Asp Pro Leu Glu Val Thr Gln Asp Val Thr Arg
65 70 75 80

Glu Trp Ala Lys Lys Val Val Trp Lys Arg Glu Lys Ala Ser Lys Ile
85 90 95

Asn Gly Ala Tyr Phe Cys Glu Gly Arg Val Arg Gly Glu Ala Ile Arg
100 105 110

Ile Arg Thr Met Lys Met Arg Gln Gln Ala Ser Phe Leu Pro Ala Thr
115 120 125

Leu Thr Met Thr Val Asp Lys Gly Asp Asn Val Asn Ile Ser Phe Lys
130 135 140

Lys Val Leu Ile Lys Glu Glu Asp Ala Val Ile Tyr Lys Asn Gly Ser
145 150 155 160

Phe Ile His Ser Val Pro Arg His Glu Val Pro Asp Ile Leu Glu Val
165 170 175

His Leu Pro His Ala Gln Pro Gln Asp Ala Gly Val Tyr Ser Ala Arg
180 185 190

Tyr Ile Gly Gly Asn Leu Phe Thr Ser Ala Phe Thr Arg Leu Ile Val
195 200 205

Arg Arg Cys Glu Ala Gln Lys Trp Gly Pro Glu Cys Asn His Leu Cys
210 215 220

Thr Ala Cys Met Asn Asn Gly Val Cys His Glu Asp Thr Gly Glu Cys

225 230 235 240

Ile Cys Pro Pro Gly Phe Met Gly Arg Thr Cys Glu Lys Ala Cys Glu
245 250 255

Leu His Thr Phe Gly Arg Thr Cys Lys Glu Arg Cys Ser Gly Gln Glu
260 265 270

Gly Cys Lys Ser Tyr Val Phe Cys Leu Pro Asp Pro Tyr Gly Cys Ser
275 280 285

Cys Ala Thr Gly Trp Lys Gly Leu Gln Cys Asn Glu Ala Cys His Pro
290 295 300

Gly Phe Tyr Gly Pro Asp Cys Lys Leu Arg Cys Ser Cys Asn Asn Gly
305 310 315 320

Glu Met Cys Asp Arg Phe Gln Gly Cys Leu Cys Ser Pro Gly Trp Gln
325 330 335

Gly Leu Gln Cys Glu Arg Glu Gly Ile Pro Arg Met Thr Pro Lys Ile
340 345 350

Val Asp Leu Pro Asp His Ile Glu Val Asn Ser Gly Lys Phe Asn Pro
355 360 365

Ile Cys Lys Ala Ser Gly Trp Pro Leu Pro Thr Asn Glu Glu Met Thr
370 375 380

Leu Val Lys Pro Asp Gly Thr Val Leu His Pro Lys Asp Phe Asn His
385 390 395 400

Thr Asp His Phe Ser Val Ala Ile Phe Thr Ile His Arg Ile Leu Pro
405 410 415

Pro Asp Ser Gly Val Trp Val Cys Ser Val Asn Thr Val Ala Gly Met
420 425 430

Val Glu Lys Pro Phe Asn Ile Ser Val Lys Val Leu Pro Lys Pro Leu
435 440 445

Asn Ala Pro Asn Val Ile Asp Thr Gly His Asn Phe Ala Val Ile Asn
450 455 460

Ile Ser Ser Glu Pro Tyr Phe Gly Asp Gly Pro Ile Lys Ser Lys Lys
465 470 475 480

Leu Leu Tyr Lys Pro Val Asn His Tyr Glu Ala Trp Gln His Ile Gln
485 490 495

Val Thr Asn Glu Ile Val Thr Leu Asn Tyr Leu Glu Pro Arg Thr Glu
500 505 510

Tyr Glu Leu Cys Val Gln Leu Val Arg Arg Gly Glu Gly Gly Glu Gly
515 520 525

His Pro Gly Pro Val Arg Arg Phe Thr Thr Ala Ser Ile Gly Leu Pro
530 535 540

Pro Pro Arg Gly Leu Asn Leu Leu Pro Lys Ser Gln Thr Thr Leu Asn
545 550 555 560

Leu Thr Trp Gln Pro Ile Phe Pro Ser Ser Glu Asp Asp Phe Tyr Val
565 570 575

Glu Val Glu Arg Arg Ser Val Gln Lys Ser Asp Gln Gln Asn Ile Lys
580 585 590

Val Pro Gly Asn Leu Thr Ser Val Leu Leu Asn Asn Leu His Pro Arg
595 600 605

Glu Gln Tyr Val Val Arg Ala Arg Val Asn Thr Lys Ala Gln Gly Glu
610 615 620

Trp Ser Glu Asp Leu Thr Ala Trp Thr Leu Ser Asp Ile Leu Pro Pro
625 630 635 640

Gln Pro Glu Asn Ile Lys Ile Ser Asn Ile Thr His Ser Ser Ala Val
645 650 655

Ile Ser Trp Thr Ile Leu Asp Gly Tyr Ser Ile Ser Ser Ile Thr Ile
660 665 670

Arg Tyr Lys Val Gln Gly Lys Asn Glu Asp Gln His Val Asp Val Lys
675 680 685

Ile Lys Asn Ala Thr Ile Ile Gln Tyr Gln Leu Lys Gly Leu Glu Pro
690 695 700

Glu Thr Ala Tyr Gln Val Asp Ile Phe Ala Glu Asn Asn Ile Gly Ser
705 710 715 720

Ser Asn Pro Ala Phe Ser His Glu Leu Val Thr Leu Pro Glu Ser Gln
725 730 735

Ala Pro Ala Asp Leu Gly Gly Gly Lys Met Leu Leu Ile Ala Ile Leu
740 745 750

Gly Ser Ala Gly Met Thr Cys Leu Thr Val Leu Leu Ala Phe Leu Ile
755 760 765

Ile Leu Gln Leu Lys Arg Ala Asn Val Gln Arg Arg Met Ala Gln Ala
770 775 780

Phe Gln Asn Val Arg Glu Glu Pro Ala Val Gln Phe Asn Ser Gly Thr
785 790 795 800

Leu Ala Leu Asn Arg Lys Val Lys Asn Asn Pro Asp Pro Thr Ile Tyr
805 810 815

Pro Val Leu Asp Trp Asn Asp Ile Lys Phe Gln Asp Val Ile Gly Glu
820 825 830

Gly Asn Phe Gly Gln Val Leu Lys Ala Arg Ile Lys Lys Asp Gly Leu
835 840 845

Arg Met Asp Ala Ala Ile Lys Arg Met Lys Glu Tyr Ala Ser Lys Asp
850 855 860

Asp His Arg Asp Phe Ala Gly Glu Leu Glu Val Leu Cys Lys Leu Gly

865 870 875 880

His His Pro Asn Ile Ile Asn Leu Leu Gly Ala Cys Glu His Arg Gly
885 890 895

Tyr Leu Tyr Leu Ala Ile Glu Tyr Ala Pro His Gly Asn Leu Leu Asp
900 905 910

Phe Leu Arg Lys Ser Arg Val Leu Glu Thr Asp Pro Ala Phe Ala Ile
915 920 925

Ala Asn Ser Thr Ala Ser Thr Leu Ser Ser Gln Gln Leu Leu His Phe
930 935 940

Ala Ala Asp Val Ala Arg Gly Met Asp Tyr Leu Ser Gln Lys Gln Phe
945 950 955 960

Ile His Arg Asp Leu Ala Ala Arg Asn Ile Leu Val Gly Glu Asn Tyr
965 970 975

Val Ala Lys Ile Ala Asp Phe Gly Leu Ser Arg Gly Gln Glu Val Tyr
980 985 990

Val Lys Lys Thr Met Gly Arg Leu Pro Val Arg Trp Met Ala Ile Glu
995 1000 1005

Ser Leu Asn Tyr Ser Val Tyr Thr Thr Asn Ser Asp Val Trp Ser
1010 1015 1020

Tyr Gly Val Leu Leu Trp Glu Ile Val Ser Leu Gly Gly Thr Pro
1025 1030 1035

Tyr Cys Gly Met Thr Cys Ala Glu Leu Tyr Glu Lys Leu Pro Gln
1040 1045 1050

Gly Tyr Arg Leu Glu Lys Pro Leu Asn Cys Asp Asp Glu Val Tyr
1055 1060 1065

Asp Leu Met Arg Gln Cys Trp Arg Glu Lys Pro Tyr Glu Arg Pro
1070 1075 1080

Ser Phe Ala Gln Ile Leu Val Ser Leu Asn Arg Met Leu Glu Glu
    1085                1090                1095

Arg Lys Thr Tyr Val Asn Thr Thr Leu Tyr Glu Lys Phe Thr Tyr
    1100                1105                1110

Ala Gly Ile Asp Cys Ser Ala Glu Glu Ala Ala
    1115                1120

<210> 3
<211> 1494
<212> DNA
<213> Homo sapiens

<220>
<221> CDS
<222> (1)..(1494)
<223>

<400> 3
atg aca gtt ttc ctt tcc ttt gct ttc ctc gct gcc att ctg act cac 48
Met Thr Val Phe Leu Ser Phe Ala Phe Leu Ala Ala Ile Leu Thr His
1               5                   10                  15

ata ggg tgc agc aat cag cgc cga agt cca gaa aac agt ggg aga aga 96
Ile Gly Cys Ser Asn Gln Arg Arg Ser Pro Glu Asn Ser Gly Arg Arg
            20                  25                  30

tat aac cgg att caa cat ggg caa tgt gcc tac act ttc att ctt cca 144
Tyr Asn Arg Ile Gln His Gly Gln Cys Ala Tyr Thr Phe Ile Leu Pro
        35                  40                  45

gaa cac gat ggc aac tgt cgt gag agt acg aca gac cag tac aac aca 192
Glu His Asp Gly Asn Cys Arg Glu Ser Thr Thr Asp Gln Tyr Asn Thr
    50                  55                  60

aac gct ctg cag aga gat gct cca cac gtg gaa ccg gat ttc tct tcc 240
Asn Ala Leu Gln Arg Asp Ala Pro His Val Glu Pro Asp Phe Ser Ser

```
65                  70                  75                  80

cag aaa ctt caa cat ctg gaa cat gtg atg gaa aat tat act cag tgg        288
Gln Lys Leu Gln His Leu Glu His Val Met Glu Asn Tyr Thr Gln Trp
                85                  90                  95

ctg caa aaa ctt gag aat tac att gtg gaa aac atg aag tcg gag atg        336
Leu Gln Lys Leu Glu Asn Tyr Ile Val Glu Asn Met Lys Ser Glu Met
            100                 105                 110

gcc cag ata cag cag aat gca gtt cag aac cac acg gct acc atg ctg        384
Ala Gln Ile Gln Gln Asn Ala Val Gln Asn His Thr Ala Thr Met Leu
        115                 120                 125

gag ata gga acc agc ctc ctc tct cag act gca gag cag acc aga aag        432
Glu Ile Gly Thr Ser Leu Leu Ser Gln Thr Ala Glu Gln Thr Arg Lys
    130                 135                 140

ctg aca gat gtt gag acc cag gta cta aat caa act tct cga ctt gag        480
Leu Thr Asp Val Glu Thr Gln Val Leu Asn Gln Thr Ser Arg Leu Glu
145                 150                 155                 160

ata cag ctg ctg gag aat tca tta tcc acc tac aag cta gag aag caa        528
Ile Gln Leu Leu Glu Asn Ser Leu Ser Thr Tyr Lys Leu Glu Lys Gln
                165                 170                 175

ctt ctt caa cag aca aat gaa atc ttg aag atc cat gaa aaa aac agt        576
Leu Leu Gln Gln Thr Asn Glu Ile Leu Lys Ile His Glu Lys Asn Ser
            180                 185                 190

tta tta gaa cat aaa atc tta gaa atg gaa gga aaa cac aag gaa gag        624
Leu Leu Glu His Lys Ile Leu Glu Met Glu Gly Lys His Lys Glu Glu
        195                 200                 205

ttg gac acc tta aag gaa gag aaa gag aac ctt caa ggc ttg gtt act        672
Leu Asp Thr Leu Lys Glu Glu Lys Glu Asn Leu Gln Gly Leu Val Thr
    210                 215                 220

cgt caa aca tat ata atc cag gag ctg gaa aag caa tta aac aga gct        720
Arg Gln Thr Tyr Ile Ile Gln Glu Leu Glu Lys Gln Leu Asn Arg Ala
```

225 230 235 240

acc acc aac aac agt gtc ctt cag aag cag caa ctg gag ctg atg gac 768
Thr Thr Asn Asn Ser Val Leu Gln Lys Gln Gln Leu Glu Leu Met Asp
245 250 255

aca gtc cac aac ctt gtc aat ctt tgc act aaa gaa ggt gtt tta cta 816
Thr Val His Asn Leu Val Asn Leu Cys Thr Lys Glu Gly Val Leu Leu
260 265 270

aag gga gga aaa aga gag gaa gag aaa cca ttt aga gac tgt gca gat 864
Lys Gly Gly Lys Arg Glu Glu Glu Lys Pro Phe Arg Asp Cys Ala Asp
275 280 285

gta tat caa gct ggt ttt aat aaa agt gga atc tac act att tat att 912
Val Tyr Gln Ala Gly Phe Asn Lys Ser Gly Ile Tyr Thr Ile Tyr Ile
290 295 300

aat aat atg cca gaa ccc aaa aag gtg ttt tgc aat atg gat gtc aat 960
Asn Asn Met Pro Glu Pro Lys Lys Val Phe Cys Asn Met Asp Val Asn
305 310 315 320

ggg gga ggt tgg act gta ata caa cat cgt gaa gat gga agt cta gat 1008
Gly Gly Gly Trp Thr Val Ile Gln His Arg Glu Asp Gly Ser Leu Asp
325 330 335

ttc caa aga ggc tgg aag gaa tat aaa atg ggt ttt gga aat ccc tcc 1056
Phe Gln Arg Gly Trp Lys Glu Tyr Lys Met Gly Phe Gly Asn Pro Ser
340 345 350

ggt gaa tat tgg ctg ggg aat gag ttt att ttt gcc att acc agt cag 1104
Gly Glu Tyr Trp Leu Gly Asn Glu Phe Ile Phe Ala Ile Thr Ser Gln
355 360 365

agg cag tac atg cta aga att gag tta atg gac tgg gaa ggg aac cga 1152
Arg Gln Tyr Met Leu Arg Ile Glu Leu Met Asp Trp Glu Gly Asn Arg
370 375 380

gcc tat tca cag tat gac aga ttc cac ata gga aat gaa aag caa aac 1200
Ala Tyr Ser Gln Tyr Asp Arg Phe His Ile Gly Asn Glu Lys Gln Asn

```
385                 390                 395                 400

tat agg ttg tat tta aaa ggt cac act ggg aca gca gga aaa cag agc     1248
Tyr Arg Leu Tyr Leu Lys Gly His Thr Gly Thr Ala Gly Lys Gln Ser
                405                 410                 415

agc ctg atc tta cac ggt gct gat ttc agc act aaa gat gct gat aat     1296
Ser Leu Ile Leu His Gly Ala Asp Phe Ser Thr Lys Asp Ala Asp Asn
            420                 425                 430

gac aac tgt atg tgc aaa tgt gcc ctc atg tta aca gga gga tgg tgg     1344
Asp Asn Cys Met Cys Lys Cys Ala Leu Met Leu Thr Gly Gly Trp Trp
        435                 440                 445

ttt gat gct tgt ggc ccc tcc aat cta aat gga atg ttc tat act gcg     1392
Phe Asp Ala Cys Gly Pro Ser Asn Leu Asn Gly Met Phe Tyr Thr Ala
    450                 455                 460

gga caa aac cat gga aaa ctg aat ggg ata aag tgg cac tac ttc aaa     1440
Gly Gln Asn His Gly Lys Leu Asn Gly Ile Lys Trp His Tyr Phe Lys
465                 470                 475                 480

ggg ccc agt tac tcc tta cgt tcc aca act atg atg att cga cct tta     1488
Gly Pro Ser Tyr Ser Leu Arg Ser Thr Thr Met Met Ile Arg Pro Leu
                485                 490                 495

gat ttt                                                             1494
Asp Phe



<210>  4
<211>  498
<212>  PRT
<213>  Homo sapiens

<400>  4
Met Thr Val Phe Leu Ser Phe Ala Phe Leu Ala Ala Ile Leu Thr His
1               5                   10                  15
```

Ile Gly Cys Ser Asn Gln Arg Arg Ser Pro Glu Asn Ser Gly Arg Arg
20 25 30

Tyr Asn Arg Ile Gln His Gly Gln Cys Ala Tyr Thr Phe Ile Leu Pro
35 40 45

Glu His Asp Gly Asn Cys Arg Glu Ser Thr Thr Asp Gln Tyr Asn Thr
50 55 60

Asn Ala Leu Gln Arg Asp Ala Pro His Val Glu Pro Asp Phe Ser Ser
65 70 75 80

Gln Lys Leu Gln His Leu Glu His Val Met Glu Asn Tyr Thr Gln Trp
85 90 95

Leu Gln Lys Leu Glu Asn Tyr Ile Val Glu Asn Met Lys Ser Glu Met
100 105 110

Ala Gln Ile Gln Gln Asn Ala Val Gln Asn His Thr Ala Thr Met Leu
115 120 125

Glu Ile Gly Thr Ser Leu Leu Ser Gln Thr Ala Glu Gln Thr Arg Lys
130 135 140

Leu Thr Asp Val Glu Thr Gln Val Leu Asn Gln Thr Ser Arg Leu Glu
145 150 155 160

Ile Gln Leu Leu Glu Asn Ser Leu Ser Thr Tyr Lys Leu Glu Lys Gln
165 170 175

Leu Leu Gln Gln Thr Asn Glu Ile Leu Lys Ile His Glu Lys Asn Ser
180 185 190

Leu Leu Glu His Lys Ile Leu Glu Met Glu Gly Lys His Lys Glu Glu
195 200 205

Leu Asp Thr Leu Lys Glu Glu Lys Glu Asn Leu Gln Gly Leu Val Thr
210 215 220

Arg Gln Thr Tyr Ile Ile Gln Glu Leu Glu Lys Gln Leu Asn Arg Ala

225 230 235 240

Thr Thr Asn Asn Ser Val Leu Gln Lys Gln Gln Leu Glu Leu Met Asp
245 250 255

Thr Val His Asn Leu Val Asn Leu Cys Thr Lys Glu Gly Val Leu Leu
260 265 270

Lys Gly Gly Lys Arg Glu Glu Glu Lys Pro Phe Arg Asp Cys Ala Asp
275 280 285

Val Tyr Gln Ala Gly Phe Asn Lys Ser Gly Ile Tyr Thr Ile Tyr Ile
290 295 300

Asn Asn Met Pro Glu Pro Lys Lys Val Phe Cys Asn Met Asp Val Asn
305 310 315 320

Gly Gly Gly Trp Thr Val Ile Gln His Arg Glu Asp Gly Ser Leu Asp
325 330 335

Phe Gln Arg Gly Trp Lys Glu Tyr Lys Met Gly Phe Gly Asn Pro Ser
340 345 350

Gly Glu Tyr Trp Leu Gly Asn Glu Phe Ile Phe Ala Ile Thr Ser Gln
355 360 365

Arg Gln Tyr Met Leu Arg Ile Glu Leu Met Asp Trp Glu Gly Asn Arg
370 375 380

Ala Tyr Ser Gln Tyr Asp Arg Phe His Ile Gly Asn Glu Lys Gln Asn
385 390 395 400

Tyr Arg Leu Tyr Leu Lys Gly His Thr Gly Thr Ala Gly Lys Gln Ser
405 410 415

Ser Leu Ile Leu His Gly Ala Asp Phe Ser Thr Lys Asp Ala Asp Asn
420 425 430

Asp Asn Cys Met Cys Lys Cys Ala Leu Met Leu Thr Gly Gly Trp Trp
435 440 445

Phe Asp Ala Cys Gly Pro Ser Asn Leu Asn Gly Met Phe Tyr Thr Ala
450 455 460

Gly Gln Asn His Gly Lys Leu Asn Gly Ile Lys Trp His Tyr Phe Lys
465 470 475 480

Gly Pro Ser Tyr Ser Leu Arg Ser Thr Thr Met Met Ile Arg Pro Leu
485 490 495

Asp Phe

<210> 5
<211> 1744
<212> DNA
<213> Artificial

<220>
<223> artificially synthesized sequence

<400> 5
actagttatt aatagtaatc aattacgggg tcattagttc atagcccata tatggagttc 60
cgcgttacat aacttacggt aaatggcccg cctggctgac cgcccaacga cccccgccca 120
ttgacgtcaa taatgacgta tgttcccata gtaacgccaa tagggacttt ccattgacgt 180
caatgggtgg agtatttacg gtaaactgcc cacttggcag tacatcaagt gtatcatatg 240
ccaagtacgc cccctattga cgtcaatgac ggtaaatggc ccgcctggca ttatgcccag 300
tacatgacct tatgggactt tcctacttgg cagtacatct acgtattagt catcgctatt 360
accatggtcg aggtgagccc cacgttctgc ttcactctcc ccatctcccc cccctcccca 420
cccccaattt tgtatttatt tattttttaa ttattttgtg cagcgatggg ggcggggggg 480
gggggggggc gcgcgccagg cggggcgggg cggggcgagg ggcggggcgg ggcgaggcgg 540
agaggtgcgg cggcagccaa tcagagcggc gcgctccgaa agtttccttt tatggcgagg 600
cggcggcggc ggcggcccta taaaaagcga agcgcgcggc gggcggggag tcgctgcgac 660
gctgccttcg ccccgtgccc cgctccgccg ccgcctcgcg ccgcccgccc cggctctgac 720
tgaccgcgtt actcccacag gtgagcgggc gggacggccc ttctcctccg ggctgtaatt 780
agcgcttggt ttaatgacgg cttgtttctt ttctgtggct gcgtgaaagc cttgaggggc 840
tccgggaggg ccctttgtgc ggggggagcg gctcgggggg tgcgtgcgtg tgtgtgtgcg 900
tggggagcgc cgcgtgcggc tccgcgctgc ccggcggctg tgagcgctgc gggcgcggcg 960
cggggctttg tgcgctccgc agtgtgcgcg aggggagcgc ggccgggggc ggtgccccgc 1020

ggtgcggggg gggctgcgag gggaacaaag gctgcgtgcg gggtgtgtgc gtgggggggt 1080
gagcaggggg tgtgggcgcg tcggtcgggc tgcaaccccc cctgcacccc cctccccgag 1140
ttgctgagca cggcccggct tcgggtgcgg ggctccgtac ggggcgtggc gcggggctcg 1200
ccgtgccggg cggggggtgg cggcaggtgg gggtgccggg cggggcgggg ccgcctcggg 1260
ccggggaggg ctcgggggag gggcgcggcg gcccccggag cgccggcggc tgtcgaggcg 1320
cggcgagccg cagccattgc cttttatggt aatcgtgcga gagggcgcag ggacttcctt 1380
tgtcccaaat ctgtgcggag ccgaaatctg ggaggcgccg ccgcaccccc tctagcgggc 1440
gcggggcgaa gcggtgcggc gccggcagga aggaaatggg cggggagggc cttcgtgcgt 1500
cgccgcgccg ccgtcccctt ctccctctcc agcctcgggg ctgtccgcgg ggggacggct 1560
gccttcgggg gggacggggc agggcggggt tcggcttctg gcgtgtgacc ggcggctcta 1620
gagcctctgc taaccatgtt catgccttct tctttttcct acagctcctg ggcaacgtgc 1680
tggttattgt gctgtctcat cattttggca aagaattcgg cttgatcgaa gcttgcccac 1740
catg 1744

<210> 6
<211> 30
<212> DNA
<213> Artificial

<220>
<223> an artificially synthesized primer

<400> 6
cagaggcagt acatgctaag aattgagtta 30

<210> 7
<211> 24
<212> DNA
<213> Artificial

<220>
<223> an artificially synthesized primer

<400> 7
agatgctcaa ggggcttcat gatg 24

<210> 8

<211> 20
<212> DNA
<213> Artificial

<220>
<223> an artificially synthesized primer

<400> 8
tattgggcgc ctggtcacca 20

<210> 9
<211> 20
<212> DNA
<213> Artificial

<220>
<223> an artificially synthesized primer

<400> 9
ccaccttctt gatgtcatca 20